# EUROPEAN PATENT SPECIFICATION

(11) **EP 0 707 654 B1**
(45) Date of publication and mention of the grant of the patent: **15.09.1999**
(21) Application number: 94922547.8
(22) Date of filing: 08.07.1994
(51) Int. Cl.: C12N 15/75, C12N 15/32, C12N 15/52, C07K 14/325, A01N 63/00, C12N 9/00, A01H 5/00, C12N 1/21, C12R 1/07

(54) **BACILLUS THURINGIENSIS TRANSPOSON TN5401 AND ITS USE IN A SITE-SPECIFIC RECOMBINATION SYSTEM FOR BACILLUS THURINGIENSIS STRAIN DEVELOPMENT**
BACILLUS THURINGIENSIS TRANSPOSON TN5401 UND SEINE VERWENDUNG IN EINEM ORTS-SPEZIFISCHEN REKOMBINATIONSSYSTEM FÜR DIE ENTWICKLUNG VON B. THURINGIENSIS-STÄMMEN
TRANSPOSON TN5401 DU BACILLUS THURINGIENSIS ET SON UTILISATION DANS UN SYSTEME DE RECOMBINAISON SITOSPECIFIQUE POUR DEVELOPPER DES SOUCHES DE BACILLUS THURINGIENSIS

(30) Priority: 08.07.1993 US 89986; 24.06.1994 US 266408
(43) Date of publication of application: 24.04.1996
(73) Proprietor: ECOGEN, INC., Langhorne, PA 19047-3023 (US)
(72) Inventor: BAUM, James, A., Doylestown, PA 18901 (US)
(74) Representative: VOSSIUS & PARTNER
(86) International application number: PCT/US94/07886
(87) International publication number: WO 95/02058

(56) References cited:
- WO-A-91/18102
- EMBO (EUR MOL BIOL ORGAN) J 7 (5). 1988. 1515-1526, MAHILLON, J. ET AL. 'STRUCTURAL AND FUNCTIONAL ANALYSIS OF TN4430 IDENTIFICATION OF AN INTEGRASE-LIKE PROTEIN INVOLVED IN THE CO-INTEGRATE-RESOLUTION PROCESS.' cited in the application
- PLASMID 19 (2). 1988. 169-173, MAHILLON, J. ET AL. 'CLONING AND PARTIAL CHARACTERIZATION OF THREE SMALL CRYPTIC PLASMIDS FROM BACILLUS-THURINGIENSIS.' cited in the application
- FEMS MICROBIOL. LETT. (1988), 49(3), 417-22 , LERECLUS, D. ET AL 'Characterization of two Bacillus thuringiensis plasmids whose replication is thermosensitive in B. subtilis' cited in the application
- JOURNAL OF BACTERIOLOGY 176 (10). 2835-2845, May 1994 BAUM, J. A. 'Tn5401, a new class II transposable element from Bacillus thuringiensis.'
- MOLECULAR MICROBIOLOGY 14 (2). 1994. 381-389, ADAMS, L. ET AL. 'Elucidation of the mechanism of CryIIIA overproduction in a mutagenized strain of Bacillus thuringiensis var. tenebrionis.'

## Description

### Field of the Invention

The present invention relates to a novel transposon isolated from *Bacillus thuringiensis* and its use in a site-specific recombination system for the construction of recombinant *Bacillus thuringiensis* strains that contain one or more insecticidal toxin genes introduced from other *Bacillus thuringiensis* strains and that are useful as insecticides.

### Background of the Invention

*Bacillus thuringiensis ("B.t.")* is a gram-positive soil bacterium that produces proteinaceous crystalline inclusions during sporulation. These *B.t.* crystal proteins are often highly toxic to specific insects. Insecticidal activities have been identified for crystal proteins from various *B.t.* strains against insect larvae from the insect orders Lepidoptera (caterpillars), Diptera (mosquitos, flies) and Coleoptera (beetles).

Recently certain *B.t.* strains and *B.t.* crystal proteins have been reported as having activity against non-insect species such as nematodes. The term "insecticidal," as used herein with reference to *B.t.* strains and their crystal proteins, is intended to include such pathogenic activities against non-insect species.

Individual *B.t.* crystal proteins, also called delta-endotoxins or parasporal crystals or toxin proteins, can differ extensively in their structure and insecticidal activity. These insecticidal proteins are encoded by genes typically located on large plasmids, greater than 30 megadaltons (mDa) in size, that are found in *B.t.* strains. A number cf these *B.t.* toxin genes have been cloned and the insecticidal crystal protein products characterized for their specific insecticidal properties. A good review of cloned *B.t.* toxin genes and crystal proteins is given by Höfte et al., *Microbiol. Rev. 53*:242-255 (1989) (hereinafter Höfte and Whiteley, 1989), who also propose a useful nomenclature and classification scheme that has been adopted in this disclosure.

The insecticidal properties of *B.t.* have been long recognized, and *B.t.* strains were first commercially introduced in biological insecticide products in the 1960's. Commercialized *B.t.* insecticide formulations typically contain dried *B.t.* fermentation cultures whose crystal protein is toxic to various insect species and, in the past, were derived from "wild-type" *B.t.* strains, i.e., purified cultures of *B.t.* strains isolated from natural sources.

Several newly commercialized *B.t.* strains are genetically altered strains that have increased insecticidal potency as well as insecticidal activity against a broader spectrum of target insects, as compared with the parent *B.t.* strains. Such strains are exemplified in International Patent Publication No. WO 88/08877, published November 17, 1988 by applicant Ecogen Inc., and its counterpart U.S. Patent No. 5,080,857 issued to González *et al.* on January 14, 1992.

Development of these genetically altered *B.t.* strains did not involve recombinant DNA technology but was instead based on the techniques of plasmid conjugal transfer, which is a natural form of genetic exchange between bacteria, and of plasmid curing, in which certain nonessential plasmids are deleted from a bacterium.

Plasmid conjugal transfer, or conjugation, is limited by the fact that many plasmids carrying useful toxin genes are not amenable to transfer from their native host *B.t.* strain to another "recipient" *B.t.* strain. Furthermore, some plasmids which can be transferred by conjugation are inherently incompatible with other plasmids, so a stable "transconjugant" *B.t.* strain, containing the two desired, incompatible plasmids, cannot be constructed.

Another drawback to conjugation is that some mobilizable, or transferable, plasmids carry undesirable toxin genes in addition to the desired gene, so the quantity of the desired crystal protein produced is limited by concurrent production of an unwanted crystal protein.

Despite the demonstrated efficacy of commercialized transconjugant *B.t.* strains against certain target insects, there is a clear need for improved *B.t.* strains against other insect pests. Development of such *B.t.* strains will be facilitated by use of recombinant DNA technology in *B.t.* strain construction.

Recombinant DNA procedures provide great flexibility in the construction of novel plasmids containing one or more toxin genes, by permitting selection, manipulation and control of crystal protein type and production and of gene regulation and expression. Some techniques for utilizing the recombinant DNA approach in the production of transformed *B.t.* strains are described in European Patent Application Publication No. EP 0 342 633, published November 23, 1989 by applicant Ciba-Geigy AG, and in European Patent Application Publication No. 0 537 105, published April 14, 1993 by applicant Sandoz Ltd.

The recombinant *B.t.* strains disclosed in EP 0 342 633, EP 0 537 105 and other publications are generally characterized by the presence of one or more antibiotic resistance marker genes on the recombinant plasmid harboring the desired *B.t.* toxin gene(s). Such antibiotic resistance marker genes provide a means for the identification and selection of transformed *B.t.* strains containing the recombinant toxin-encoding plasmid but are undesirable in viable *B.t.* strains developed for use in commercial insecticide formulations. Since antibiotic resistance genes are not ordinarily present in native *B.t.* strains, pesticide and environmental regulatory agencies may be reluctant to approve antibiotic-resistant recombinant *B.t.* strains for unrestricted environmental release and for use in biological insecticide formulations.

A major reason for the presence of antibiotic resistance genes in recombinant *B.t.* strains described in the literature is the use of bifunctional cloning vectors containing such resistance marker genes. Portions of these cloning vectors are typically derived from plasmids not native to *B.t.*, e.g., *Escherichia coli, Bacillus cereus, Bacillus subtilis* or *Staphylococcus aureus* plasmids, and contain, in addition to the antibiotic resistance marker gene, an origin of replication from a non-*B.t.* source that is also functional in B.t. and therefore permits the cloning vector to be replicated and maintained in *B.t.*

International Patent Publication No. WO 91/18102, published November 28, 1990 by applicant Ecogen Inc., describes a plasmid shuttle vector for recombinant *B.t.* strain development that facilitates incorporation of recombinant plasmids into *B.t.* strain constructs that contain no DNA derived from *E. coli* or other non-*B.t.* biological sources. Using this shuttle vector, a cloned *B.t.* toxin gene and *B.t.* plasmid replication origin region are isolated as a single restriction fragment that, upon self-ligation, is introduced into *B.t.* by cotransformation. This plasmid shuttle vector utilizes a *B.t.* replication origin derived from large resident plasmids of *B.t.*, a multiple cloning site and strategically placed restriction endonuclease cleavage sites to enable construction of *B.t.* strains that are free of antibiotic resistance marker genes and free of non-*B.t.* replication origins.

A second approach for constructing such *B.t.* strains is a multistep technique described by Lereclus et al., *Bio/Technology 10*:418-421 (1992) that relies on the presence of IS*232* in a resident *B.t.* toxin plasmid to effect homologous recombination. A cloned *B.t.* toxin gene is inserted within a cloned fragment of IS*232* (which is found on some naturally-occurring toxin-encoding *B.t.* plasmids) that is inserted into a shuttle plasmid thermosensitive for replication in *B.t.* The shuttle plasmid is then used to transform a *B.t.* strain containing the IS*232* fragment on a resident *B.t.* plasmid, and transformants are selected at non-permissive temperature for clones in which the shuttle vector has integrated via homologous recombination into a copy of IS*232* present on the resident plasmid. Subsequently, individual clones are screened for a second homologous recombination event that eliminates the shuttle vector and conserves the newly introduced toxin gene. This technique is limited by the laborious nature of its steps and its reliance on homologous recombination using IS*232*-containing resident *B.t.* plasmids, whose copy number cannot readily be altered to increase gene expression.

Removal of unwanted selectable marker genes or other unwanted DNA has been described for transgenic plants and eukaryotic cells via the so-called Cre/lox recombination system of bacteriophage P1, where the *cre* gene encoding the Cre recombinase enzyme is activated to delete the unwanted DNA, which is bracketed by *lox* recombination site sequences. International Patent Publication No. WO 93/01283, published January 21, 1993 by applicant U.S. Department of Agriculture, and Dale et al., "Gene transfer with subsequent removal of the selection gene from the host genome," *Proc. Natl. Acad. Sci. USA 88*:10558-10562 (1991), describe such a system for removal of a antibiotic resistance marker gene from transgenic tobacco plants.

U.S. Patent No. 4,959,317 issued September 25, 1990 to Sauer describes the application of the Cre/*lox* recombination system to yeast cells and to a mouse cell line to delete or invert selected DNA sequences.

Höfte and Whiteley, 1989, in discussing factors such as conjugative plasmid transfer that account for the observed mobility of crystal protein genes among *B.t.* strains, note past reports of some *cryIA-type* genes and the *cryIVB* gene being associated with insertion sequence (IS) elements on transposon-like structures (see paragraph bridging pages 245-246). Nevertheless, the role of repeat sequence and/or insertion sequence elements and transposon-like structures in the mobility of *B.t.* crystal protein genes still remains speculative.

Among known *B.t.* strains, only one transposon (transposable element) has been reported in the literature as having been isolated from *B.t.* Mahillon et al., *EMBO J. 7*: 1515-1526 (1988) provide a detailed description of this transposon, originally reported in a 1983 publication and now named Tn*4430*. Murphy, "Transposable Elements in Gram-Positive Bacteria," Chapt. 9 in *Mobile DNA,* Berg et al., eds., Am. Soc. Microbiol., Washington, D.C. (1989) pp. 269-288, likewise discusses Tn*4430*, in the context of other transposable elements found in gram-positive bacteria.

Mahillon et al., *Plasmid 19*:169-173 (1988), describe the cloning in *E. coli* and restriction mapping of three small cryptic plasmids from *B.t.* var. *thuringiensis,* one of the plasmids being pGI2 which was reported to contain the *B.t.* transposon Tn*4430*. The authors speculate (at page 173) that the cloned plasmids could serve as the starting point for the development of new shuttle vectors for *E. coli* and *B.t.* but offer no details concerning the construction and use of such hypothetical plasmid shuttle vectors. The complete nucleotide sequence of the small cryptic plasmid pGI2, including Tn*4430*, is reported by Mahillon et al. in *Nucl. Acids Res. 16*:11827-11828 (1988).

Earlier references cited by Mahillon et al. in *EMBO J. 2*:1515-1526 (1988) disclose that, although Tn*4430* is widely distributed among *B.t.* species, the functional role of Tn*4430* in *B.t.,* if any, remains unclear. Despite occasional mention in investigative research publications concerning *B.t.*, of Tn*4430* and of homology of its elements with other known insertion sequence elements, this transposon has not been utilized to facilitate construction of insecticidal *B.t.* strains; see, e.g., Lereclus et al., *FEMS Microbiol. Lett. 49*:417-422 (1988).

The novel transposon of the present invention, designated Tn*5401,* is only the second transposon to be isolated from *B.t.* since the discovery of Tn*4430* over ten years ago. Unlike Tn*4430* which is widely distributed among *B.t.* species, transposon Tn*5401* appears to be found in only a few relatively rare *B.t.* species.

The present invention also encompasses a site-specific recombination system for recombinant *B.t.* strain construction that preferably utilizes certain elements of transposon Tn*5401*, e.g., its internal resolution site and recombinase gene. The site-specific recombination system of this invention represents a significant advance over the approach described in International Patent Publication No. WO 91/18102 because it facilitates the rapid development and construction of recombinant *B.t.* strains whose recombinant plasmids possess highly desirable characteristics. They are completely free of foreign DNA from non-*B.t.* sources and can carry *B.t.* toxin genes that provide insecticidal properties superior to *B.t.* strains presently used in commercial bioinsecticides.

### Summary of the Invention

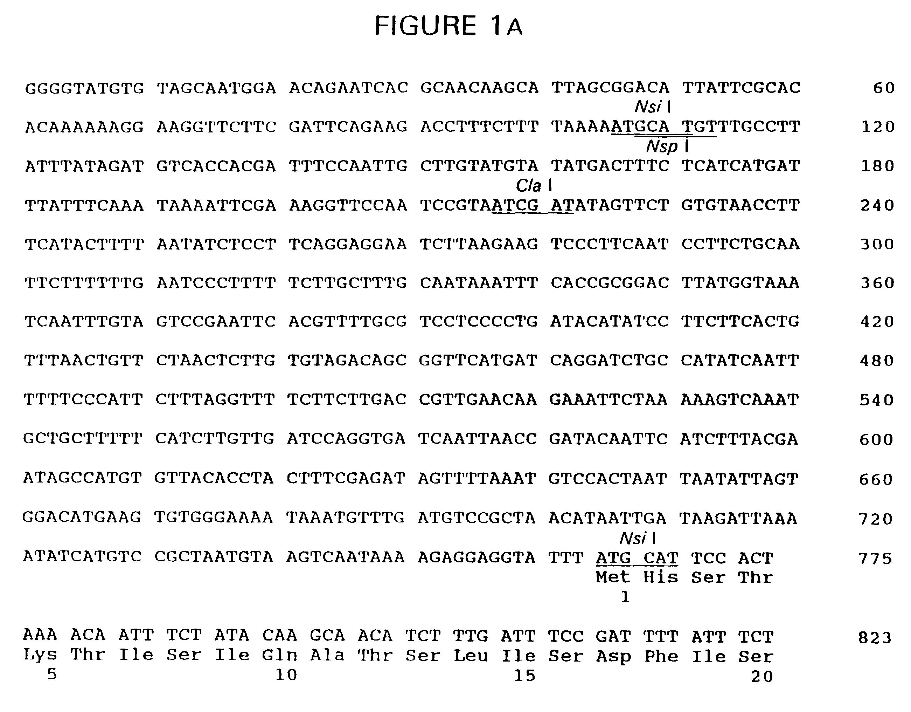

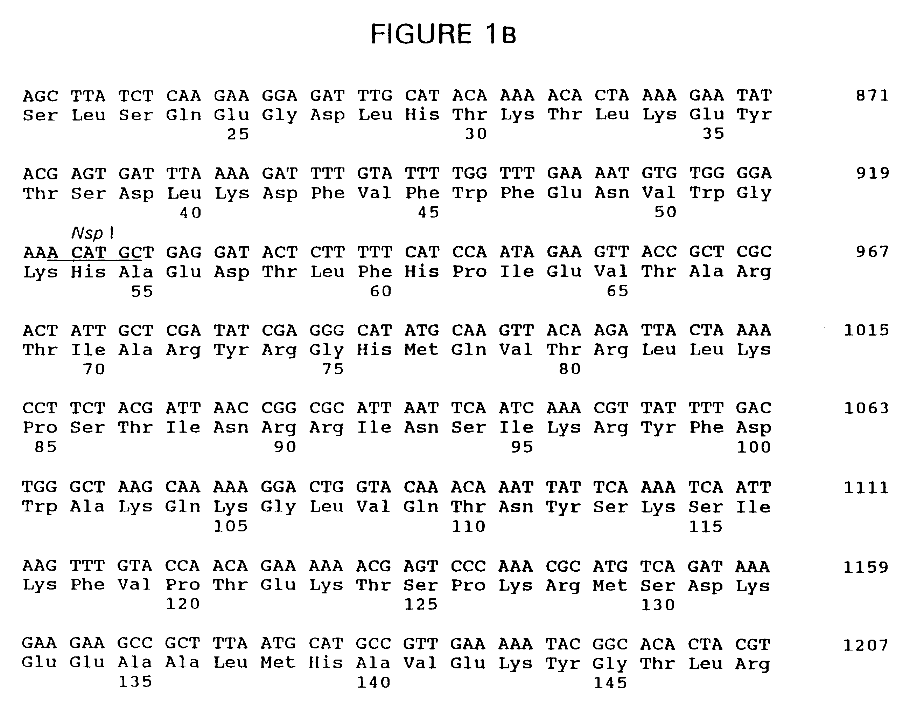

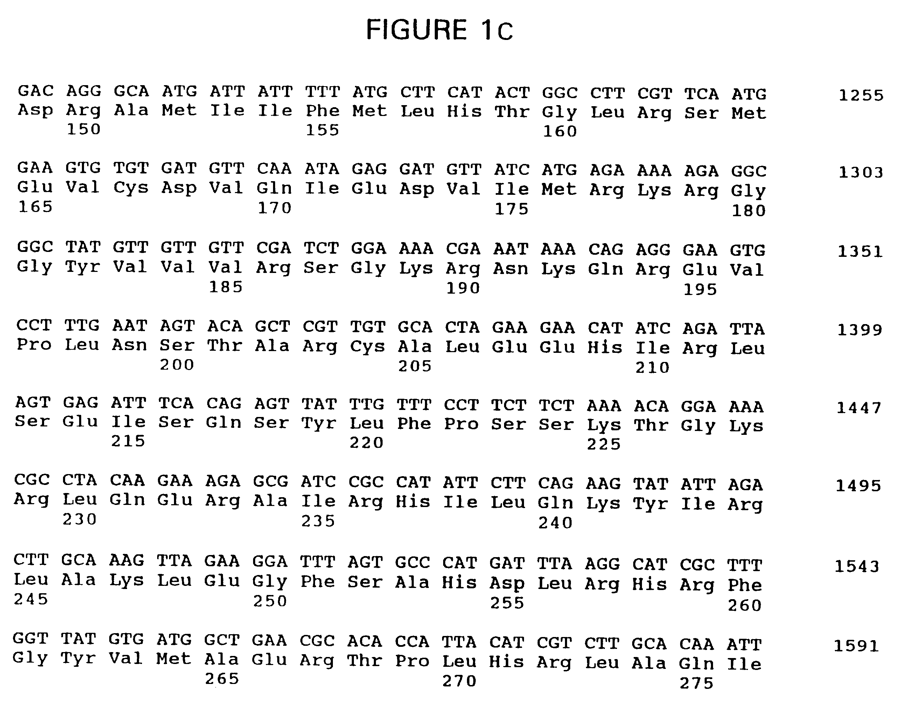

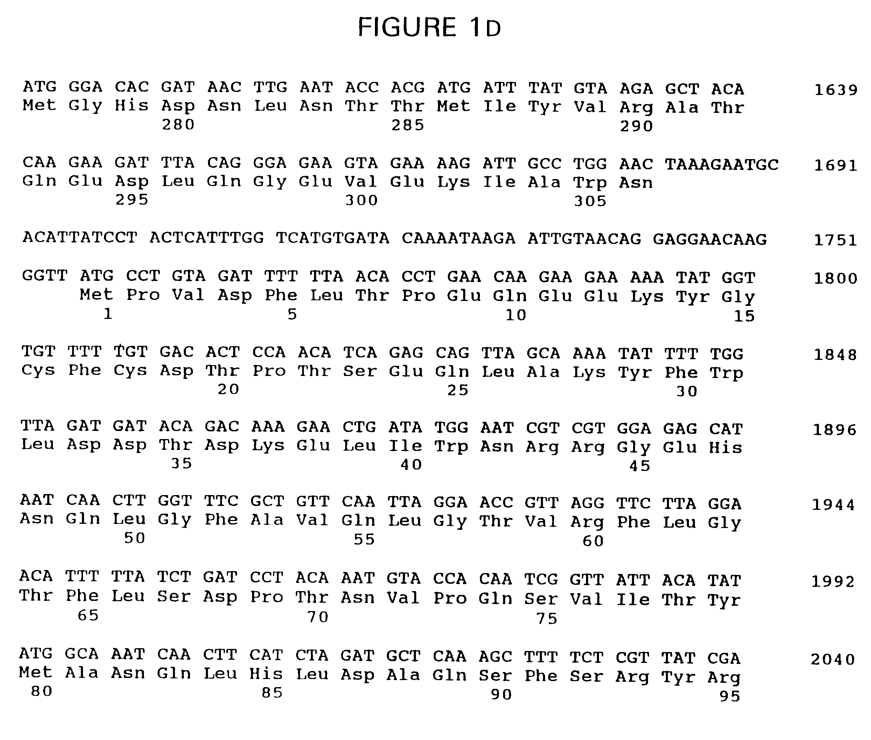

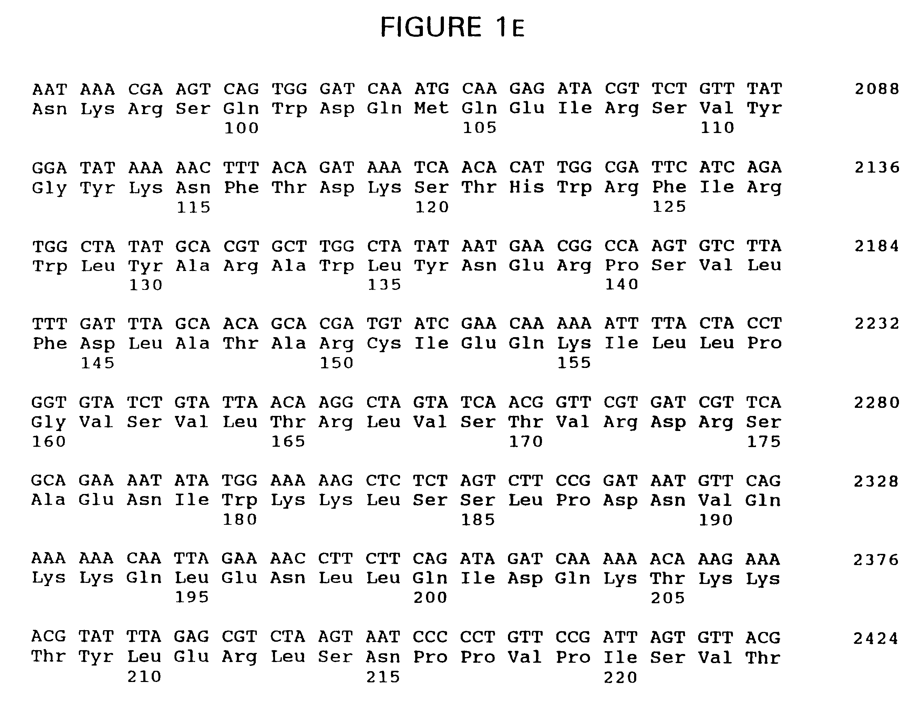

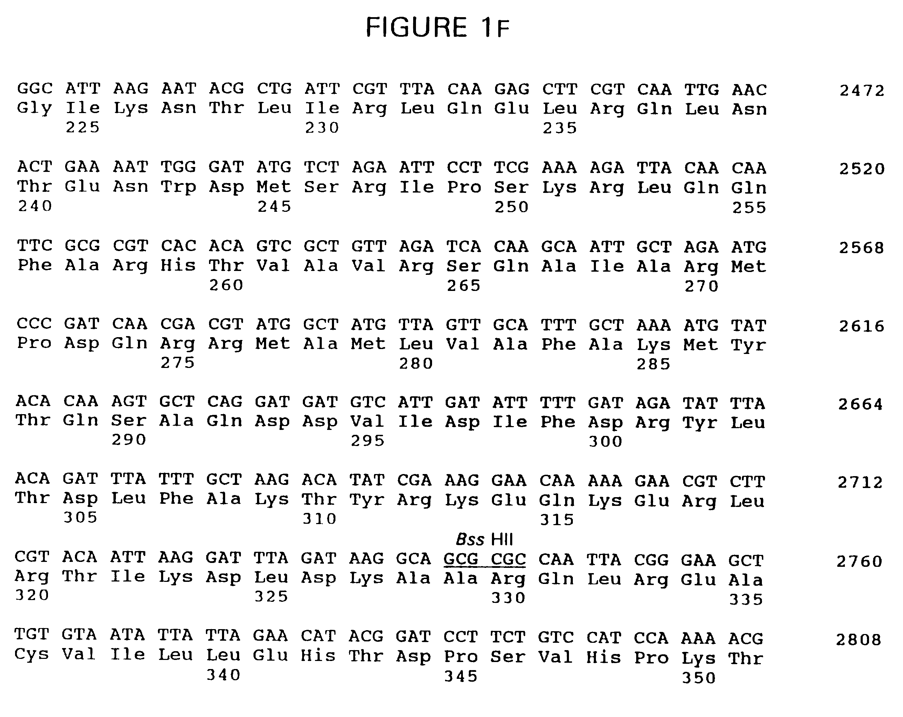

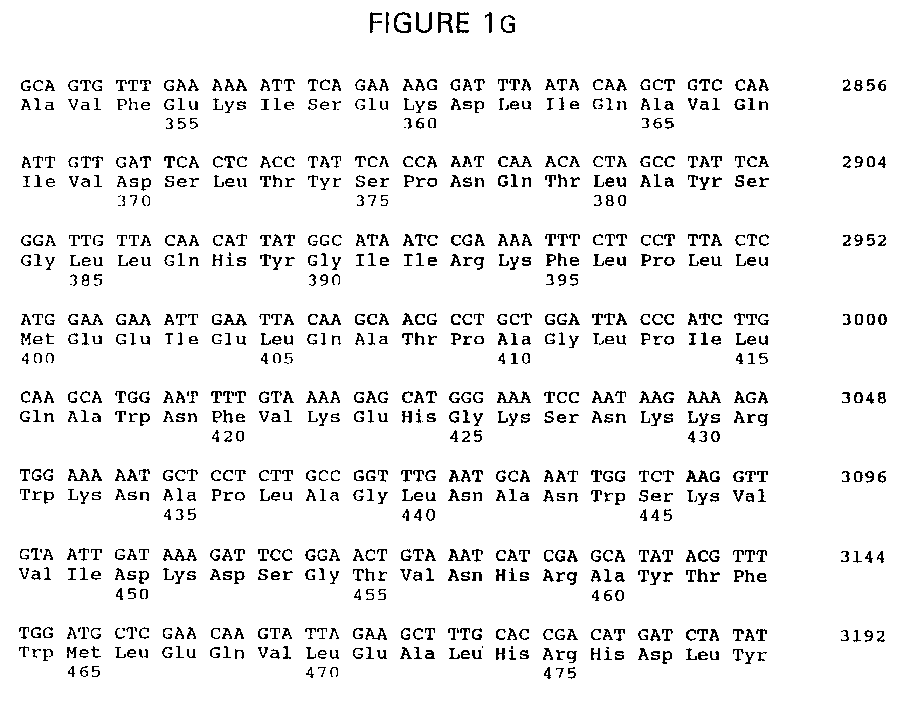

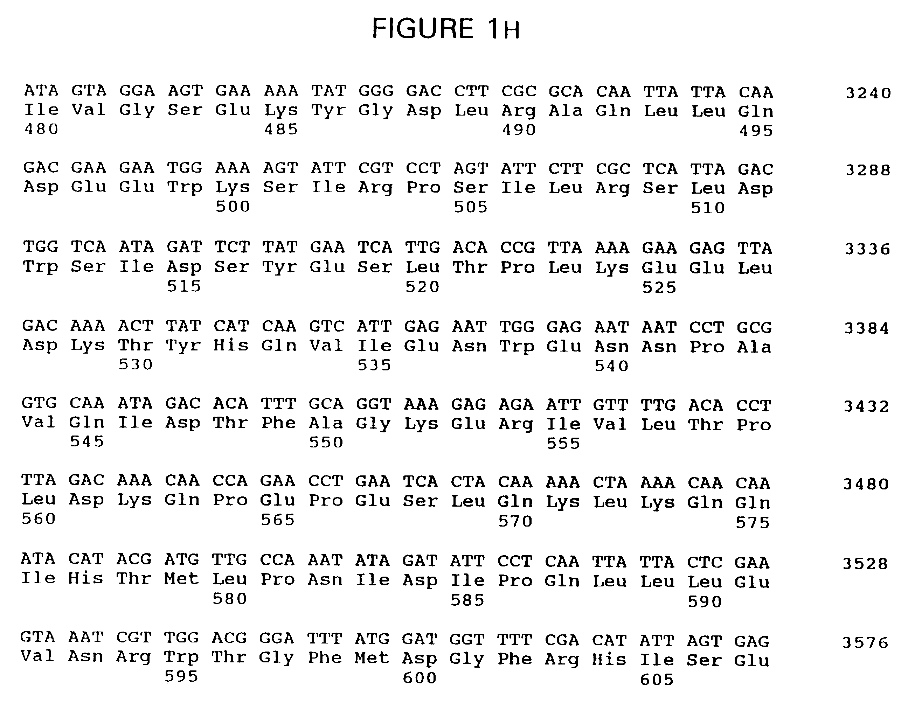

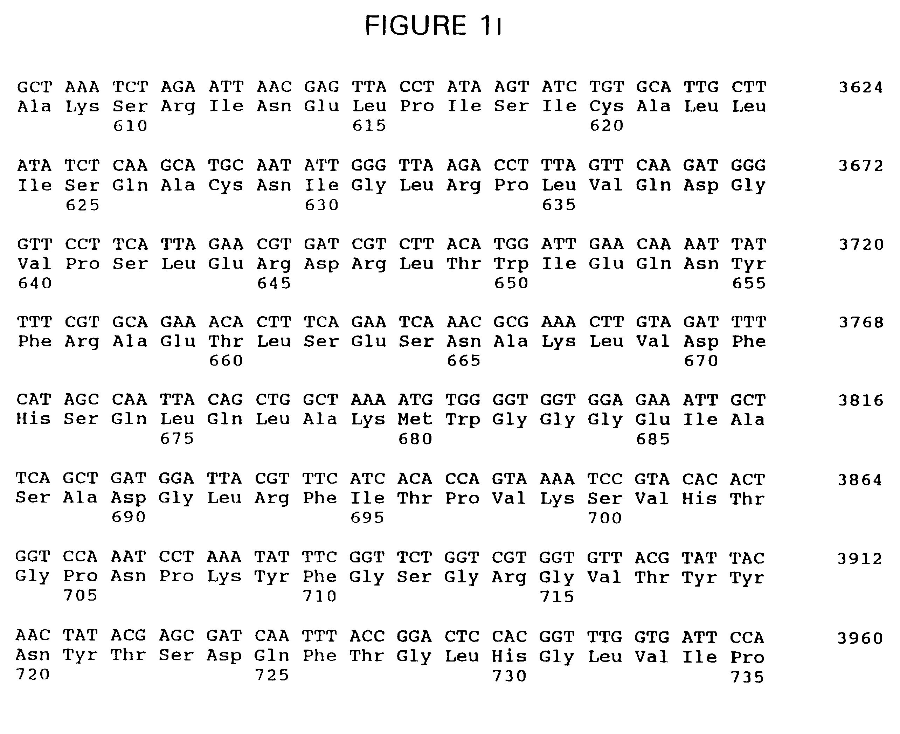

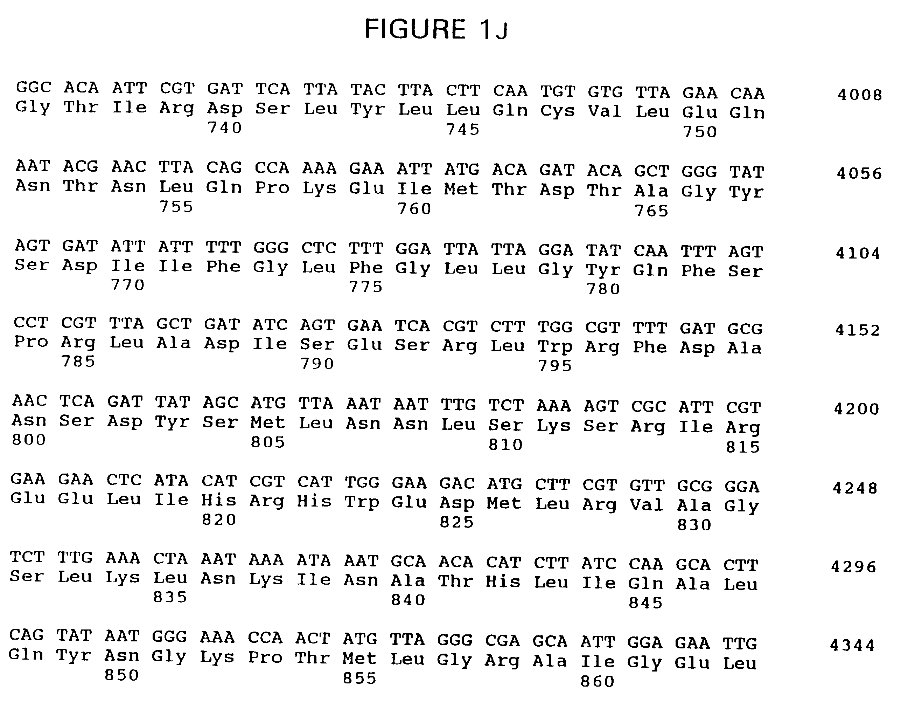

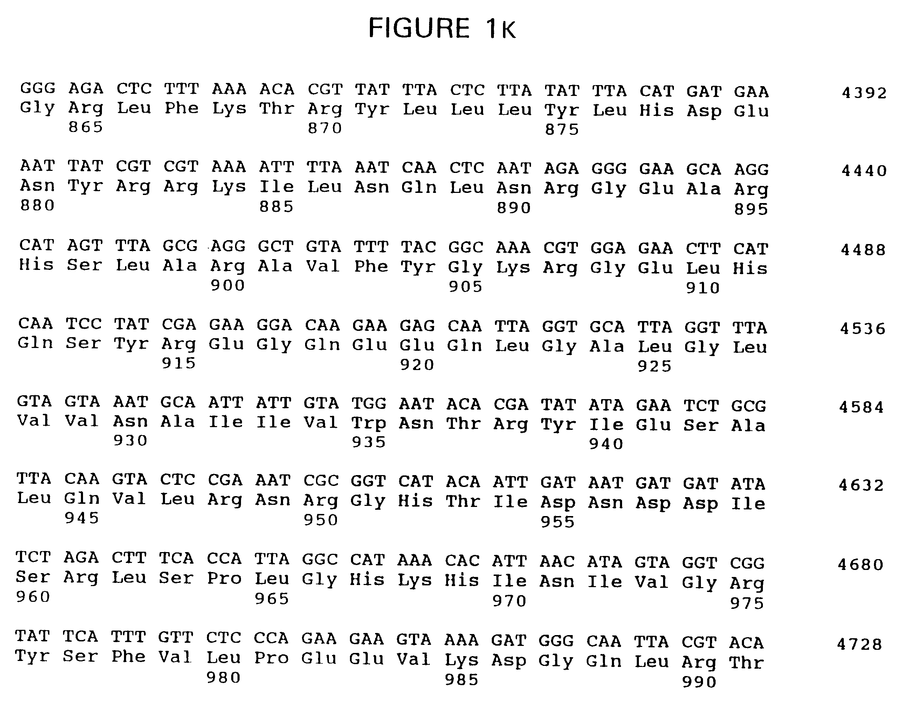

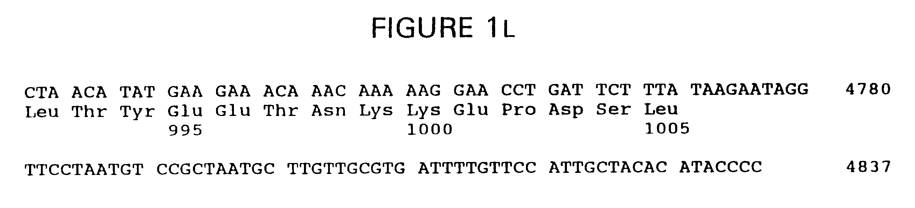

The transposable element of this invention is the isolated, purified transposon designated as Tn*5401* and whose nucleotide base sequence (SEQ ID NO:1) is shown in Figure 1, or a mutant thereof capable of functioning as a transposable element.

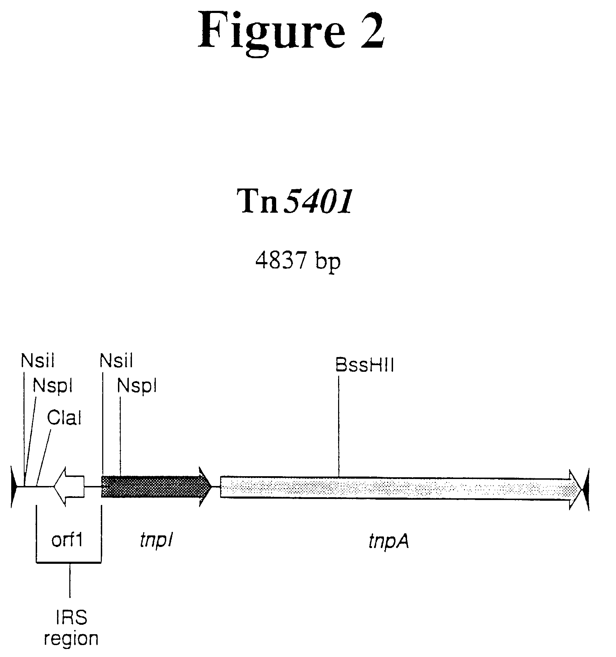

Several unique elements of Tn*5401* are also within the scope of this invention. The locations of these elements are shown in the linear structural map of Tn*5401* in Figure 2. These elements include the isolated, purified DNA sequence containing the internal resolution site, "IRS", of Tn*5401;* the isolated, purified gene designated as the Tn*5401* resolvase/recombinase gene, *tpnI;* and the isolated, purified gene designated as the Tn*5401* transposase gene, *tnpA.*

The resolvase/recombinase gene product, the resolvase protein (SEQ ID NO:2), and the transposase gene product, the transposase protein (SEQ ID NO:3), are also within the scope of this invention. Recombinant plasmids containing either transposon Tn*5401* or its internal resolution site, its resolvase/recombinase gene, or its transposase gene are also embodiments of the present invention, as are bacteria transformed with such recombinant plasmids and capable of expressing the applicable genes on such plasmids.

This invention also includes a plasmid shuttle vector useful for recombinant *Bacillus thuringiensis (B.t.)* strain development, which has (i) an origin of replication functional in *B.t.*, preferably one native to a *B.t.* plasmid, such as *B.t.* origin of replication *ori43, ori43.9, ori44* or *ori60;* (ii) DNA not native to *B.t.,* preferably selected from selectable marker genes and origins of replication functional in *E. coli* or in a Bacillus host species other than *B.t.;* (iii) optionally and preferably, one or more insecticidal protein toxin genes; (iv) two identical internal resolution sites oriented in the same direction and flanking the DNA not native to *B.t.,* thus enabling such *non-B.t.* DNA to be excised via a site-specific recombination event involving the two internal resolution sites. The internal resolution sites are preferably derived from a Tn*3*-type transposon and more preferably are identical to the internal resolution site of transposon *Tn5401.* Host *B.t.* strains or other bacterial strains transformed with this plasmid shuttle vector are also embodiments of this invention.

The method of constructing a recombinant *B.t.* strain containing no DNA elements foreign to *B.t.* is also within the scope of this invention, having the steps of (a) transforming a host *B.t.* strain with a plasmid shuttle vector containing (i) an origin of replication native to *B.t.,* (ii) DNA not native to *B.t.* and useful in the construction of recombinant *B.t.* strains, selected from the group consisting of selectable marker genes, origins of replication functional in *E. coli,* and origins of replication functional in a Bacillus host species other than *B.t.,* (iii) one or more insecticidal *B.t.* protein toxin genes, and (iv) two identical internal resolution sites oriented in the same direction and flanking the DNA not native to *B.t.,* the sites being the same as an internal resolution site from a Tn*3*-type transposon native to *B.t.;* (b) introducing into the transformed *B. t.* strain resolvase protein to effect a site-specific recombination event involving the internal resolution sites, thereby excising from the plasmid shuttle vector the DNA not native to *B.t.;* and (c) recovering a recombinant *B. t.* strain containing a recombinant plasmid capable of replicating in the *B.t.* strain and containing (i) an origin of replication native to *B.t.,* (ii) one or more insecticidal *B.t.* protein toxin genes, and (iii) a single internal resolution site, derived from the site-specific recombination event. Preferred Tn*3*-type transposon sources for the internal resolution site in the plasmid shuttle vector of this method are transposons Tn*5401* and Tn*4430*.

The present invention also encompasses a recombinant plasmid capable of replicating in a *Bacillus thuringiensis* bacterium and having (i) at least one insecticidal protein toxin gene, (ii) an origin of replication functional in *B.t.,* and (iii) a single internal resolution site, preferably derived from a Tn*3*-type transposon and more preferably identical to the internal resolution site of transposon Tn*5401*. Host *B.t.* strains or other bacterial strains containing such recombinant plasmids are also embodiments of this invention, as are insecticidal compositions with such transformed host *B.t.* strains, and as is the method of controlling insect pests utilizing such insecticidal compositions.

### Brief Description of the Drawings

Figure 1 consists of Figures 1A through 1L and depicts the nucleotide sequence for Tn*5401,* the transposon of this invention. The deduced amino acid sequence for an open reading frame extending from nucleotide base positions 764 to 1681 (excluding the terminal nonsense codon) is also shown. The gene of this open reading frame, designated the resolvase gene, encodes a protein with 306 amino acids. The deduced amino acid sequence for another open reading frame, extending from nucleotide positions 1756 to 4770 (excluding the terminal nonsense codon), is also shown. The gene of this second open reading frame, designated the transposase gene, encodes a protein with 1005 amino acids. Certain restriction endonuclease cleavage sites (*NsiI*(2), *NspI*(2), *Cla*I, *Bss*HII) are also shown.

Figure 2 is a linear structural map of transposon Tn*5401* whose 4837 basepair nucleotide sequence is shown in Figure 1. Three open reading frames are shown: "orf1" (open arrow) which encodes a cryptic protein of 85 amino acids in the 3'-5' direction; *"tnpI"* (dark shaded arrow), the resolvase gene; and *"tnpA"* (light shaded arrow), the transposase gene. An internal resolution site is located within the bracketed DNA fragment, "IRS region", shown in Figure 2. Inverted repeats of 53 basepairs at either end of the structural map are shown as black arrowheads. Certain restriction endonuclease cleavage sites (*Nsi*I(2), *Nsp*I (2), *Cla*I, *Bss*HII) are also shown.

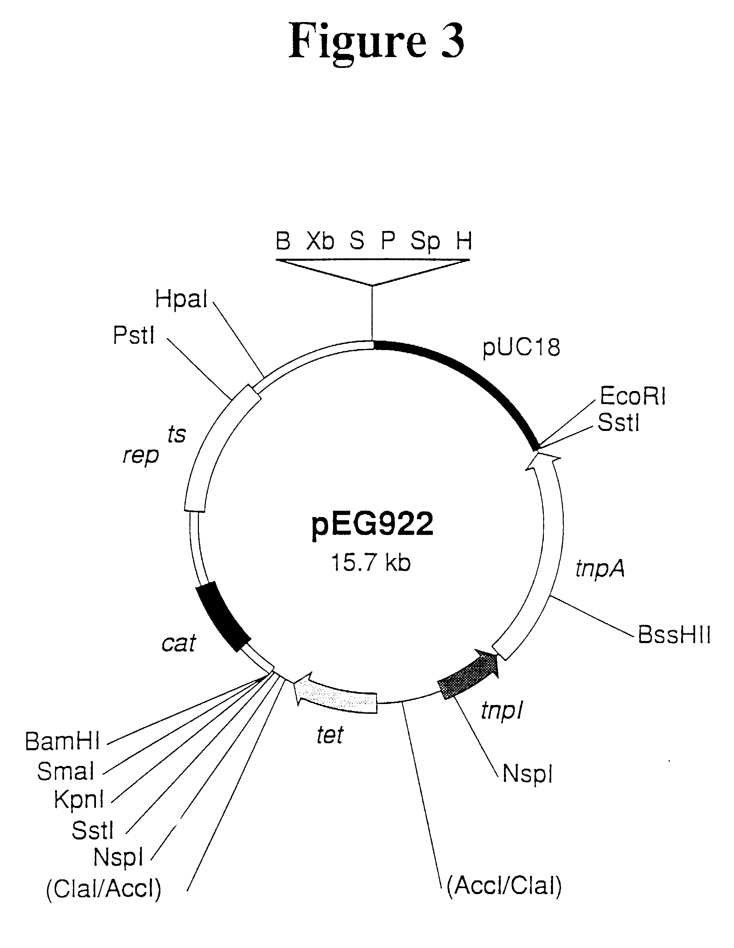

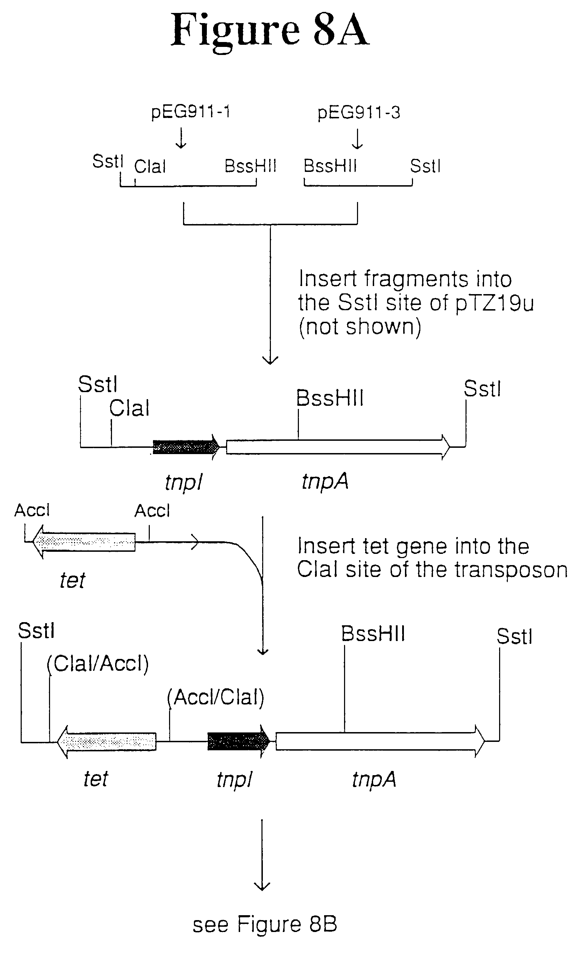

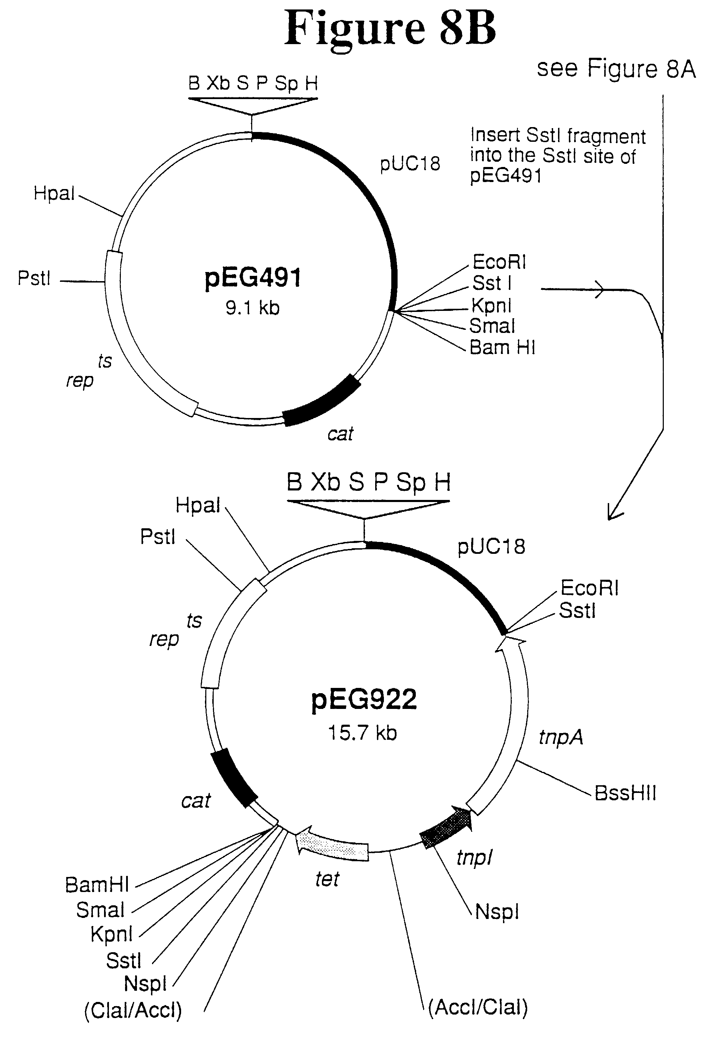

Figure 3 is a circular structural map of the recombinant plasmid pEG922, a 15.7 kilobase (kb) plasmid which contains the transposon Tn*5401* of this invention. Transposon Tn*5401* is contained on a ∼7 kb SstI-SstI fragment which comprises the following elements shown in Figure 3: Tn*5401* resolvase gene, *tnpI* (dark shaded arrow), and Tn*5401* transposase gene, tnpA (open arrow). Tn*5401* also contains an introduced tetracycline resistance gene, *tet* (light shaded arrow), a "tag" that serves as a selectable marker for the transposon. Plasmid pEG922 was constructed by inserting the ∼7 kb *Sst*I fragment containing Tn*5401* into the unique *Sst*I site of plasmid shuttle vector pEG491 (see Figure 8B). The components of shuttle vector pEG491 include: the thin solid black segment indicating the *E. coli* replicon pUC18, the thick open segment indicating the temperature-sensitive replicon, *rep*^{*ts*}*,* from plasmid pE194ts which is functional in gram-positive bacteria but which cannot operate at temperatures at or above 37°C, and the thick black segment indicating the chloramphenicol acetyl transferase gene, *cat.* At the top of the circular structural map of pEG922 is a multiple cloning site. Abbreviations for the restriction endonuclease cleavage sites in the multiple cloning site of Figure 3 are as follows: B=*Bam*HI, Xb=*Xba*I, S=*Sal*I, P=*Pst*I, Sp=*Sph*I, H=*Hind*III.

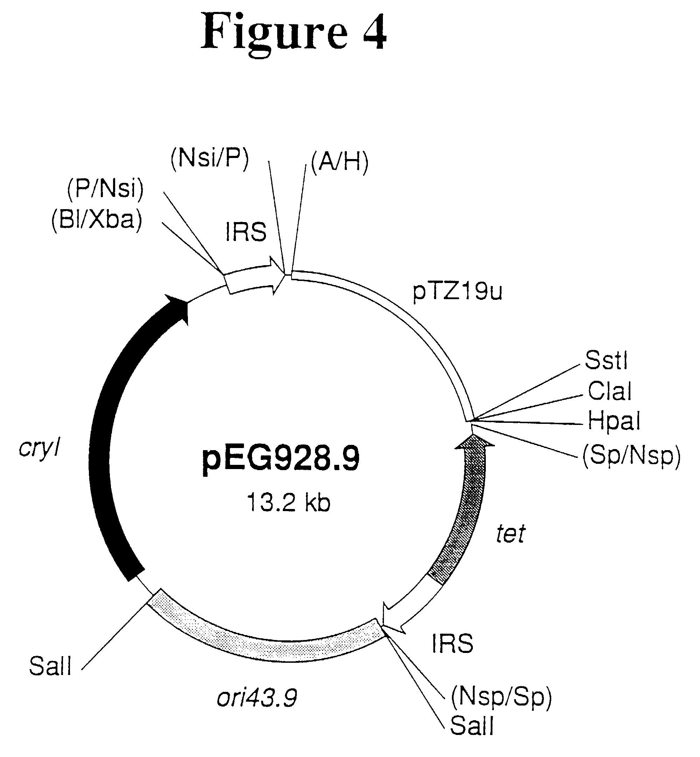

Figure 4 is a circular structural map of the recombinant plasmid shuttle vector pEG928.9 of this invention, which is 13.2 kb in size. The shuttle vector contains identical internal resolution sites, IRS, in the same orientation (open arrows), and these two sites flank the *E. coli* replicon pTZ19u (open segment in Figure 4) and a tetracycline resistance gene, *tet,* from plasmid pBC16 (dark shaded arrow). The plasmid shuttle vector also contains, outside of the IRS sites flanking the DNA not native to *B.t.,* the *ori43.9 B.t.* plasmid origin of replication (light shaded segment) and a cryI-type *B.t.* protein toxin gene (solid arrow). Letter abbreviations for the restriction endonuclease cleavage sites shown in Figure 4 are as follows: A=*Asp*718, Bl=*Bln*I, ClaI=*Cla*I, H=*Hind*III, HpaI=*Hpa*I, Nsi=*Nsi*I, Nsp=*Nsp*I, P=*Pst*I, SalI=*Sal*I, SstI=*Sst*I, Xba=*Xba*I.

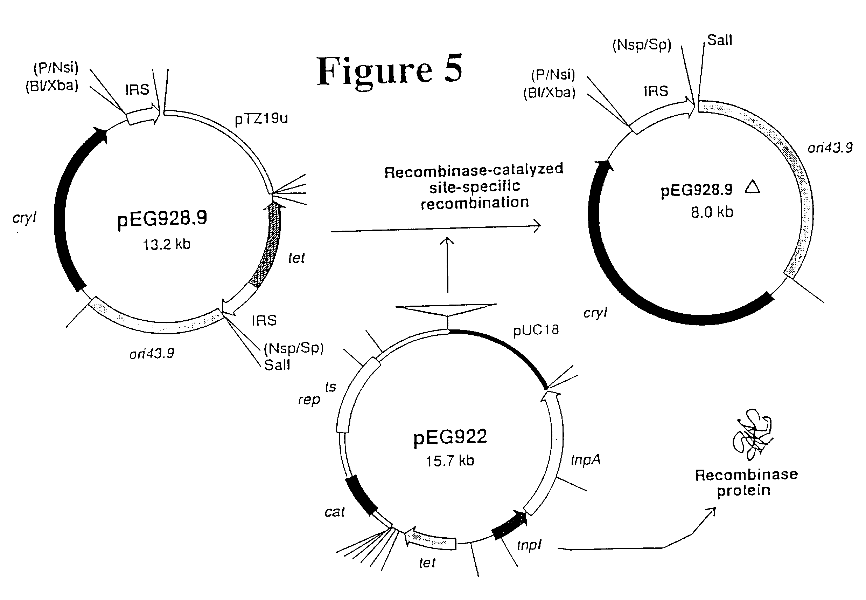

Figure 5 is a schematic illustration of a method in which the plasmid shuttle vector of this invention, pEG928.9, is manipulated to excise its DNA elements which are not native to *B.t.* Removal of the foreign DNA elements, which are bracketed by duplicate IRS sites of transposon Tn*5401,* is accomplished by catalysis with a Tn*5401* transposon-encoded resolvase/recombinase protein. Plasmid pEG928.9, shown and described in more detail in Figure 4, contains DNA not native to *B.t.*, i.e., the *E. coli* replicon pTZ19u and a tetracycline antibiotic resistance gene, *tet.* This foreign DNA is flanked on either side by copies of an internal resolution site, IRS, from transposon Tn*5401*, oriented in the same direction. Plasmid pEG922, shown and described in more detail in Figure 3, contains transposon Tn*5401*, whose resolvase gene, *tnp*I, is capable of expressing the resolvase/recombinase protein at temperatures below 37°C in this temperature-sensitive plasmid. Sequential transformation of a host *B.t.* strain (not shown in the Figure) with both plasmid pEG928.9 and plasmid pEG922 and incubation of the transformed host *B.t.* strain at a temperature of 31°C cause expression of the *tnpI* gene and production of resolvase/recombinase protein, which catalyzes a site-specific recombination event as shown in Figure 5. The resultant plasmid pEG928.9Δ, an 8.0 kb derivative of pEG928.9 from which non-*B.t.* DNA elements have been excised via the site-specific recombination event, contains a *B.t.*-derived origin of replication, *ori43.9,* a *cryI B.t.* protein toxin gene, and a single copy of the internal resolution site, IRS, of transposon Tn*5401*. Abbreviations for the restriction endonuclease cleavage sites shown in this Figure are summarized in the descriptions of Figures 3 and 4.

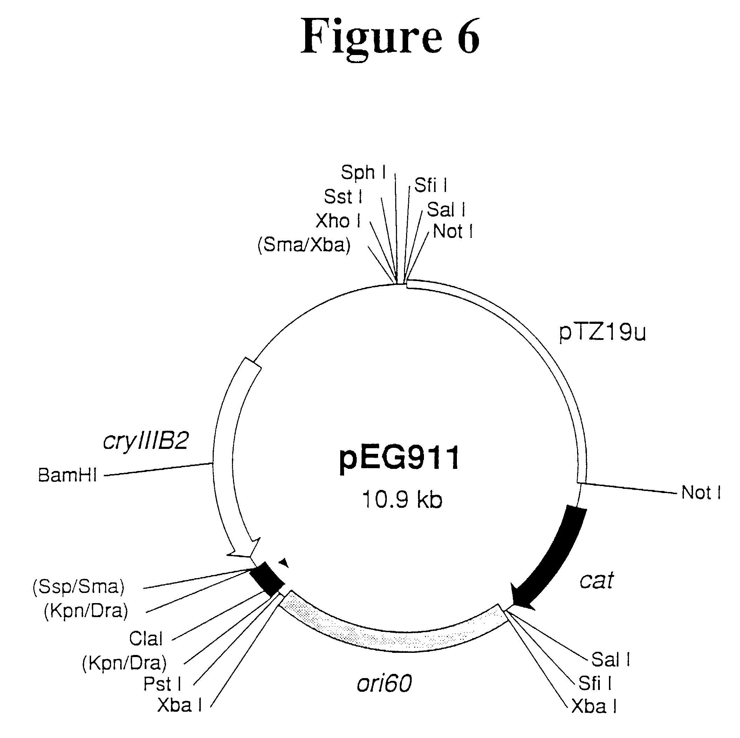

Figure 6 is a circular structural map of plasmid pEG911, from which transposon Tn*5401* was isolated after recovery of the Tn*5401*-containing pEG911 derivative from *B.t.* var. *morrisoni* strain EG2158. Plasmid pEG911, approximately 10.9 kb in size, contains the following elements. The open arrow indicates the *cryIIIB2 B.t.* protein toxin gene; the open segment indicates the *E. coli* replicon pTZ19u; the solid arrow indicates the chloramphenicol acetyl transferase gene, *cat,* from plasmid pC194; the shaded segment indicates the *ori60 B.t.* plasmid origin of replication region; and the solid box segment with accompanying arrowhead indicates a *B.t.* gene transcription terminator. Abbreviations are used for some restriction endonuclease cleavage sites shown in the Figure and these are as follows: Dra=*Dra*I, Kpn=*Kpn*I, Sma=*Sma*I, Ssp=*Ssp*I, Xba=*Xba*I.

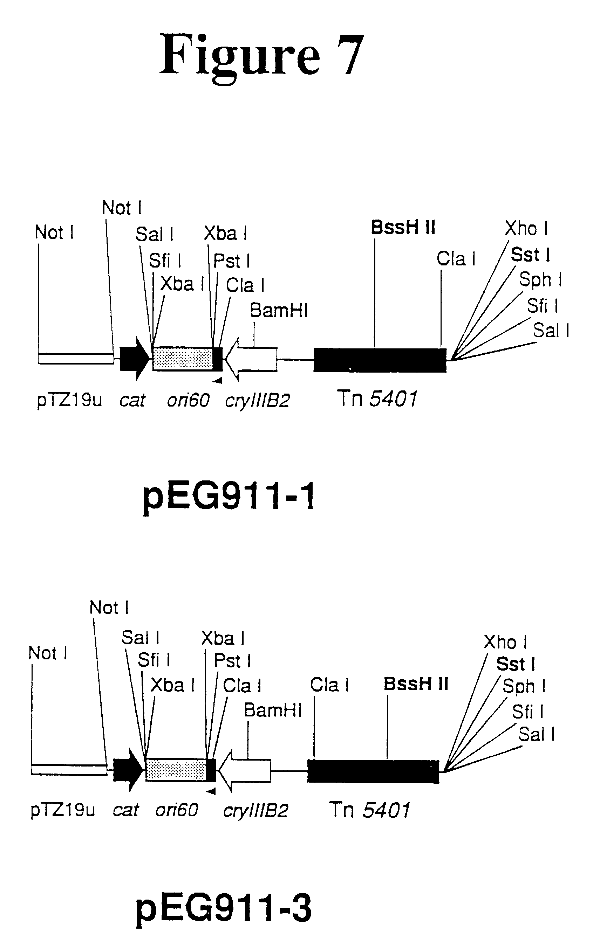

Figure 7 shows linear structural maps for plasmids pEG911-1 and pEG911-3, both of which are derivatives of plasmid pEG911 (see Figure 6) and both of which contain an insertion of transposon Tn*5401* from *B.t.* var. *morrisoni* strain EG2158. The long solid black segment in both of these structural maps indicates the transposon Tn*5401.* As indicated by the location of the *Cla*I and *Bss*HII sites within Tn*5401*, pEG911-1 and pEG911-3 contain Tn*5401* in opposite orientations. Identification of the various elements within plasmids pEG911-1 and pEG911-3 and the abbreviations for restriction sites are as described for Figure 6.

Figure 8 consists of Figures 8A and 8B and is a schematic diagram showing the recombinant DNA procedures used to derive plasmid pEG922, which is also shown in Figure 3 and is utilized in the method of Figure 5. Plasmid pEG922 contains the isolated transposon Tn*5401* of this invention. Details of the steps shown in this Figure for the derivation of plasmid pEG922 are explained in Example 2. Abbreviations are used for some of the restriction endonuclease cleavage sites shown in Figure 8 and these are as described for Figure 3, which also provides a description of the circular structural map of plasmid pEG922.

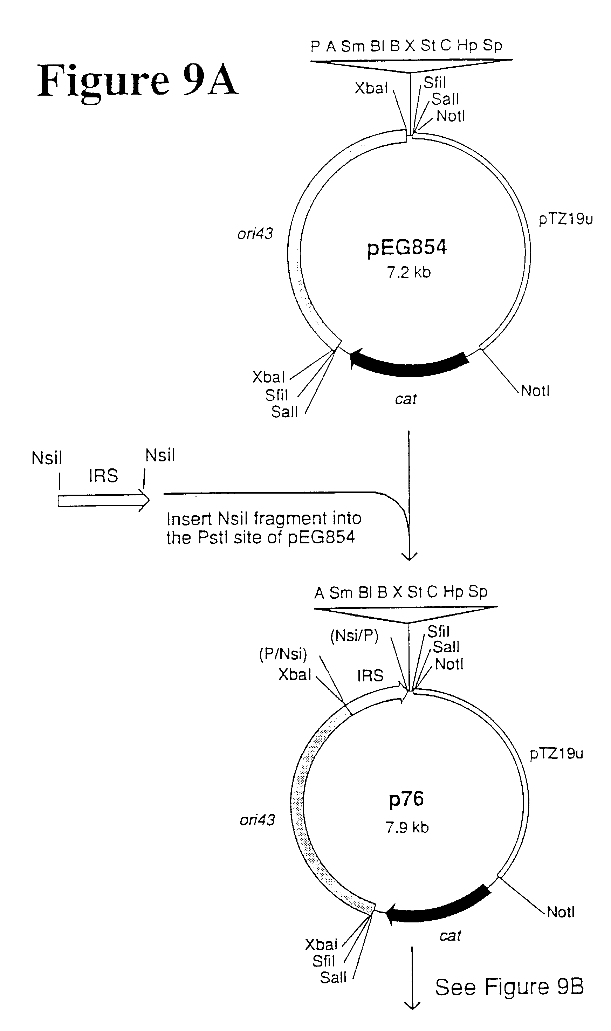

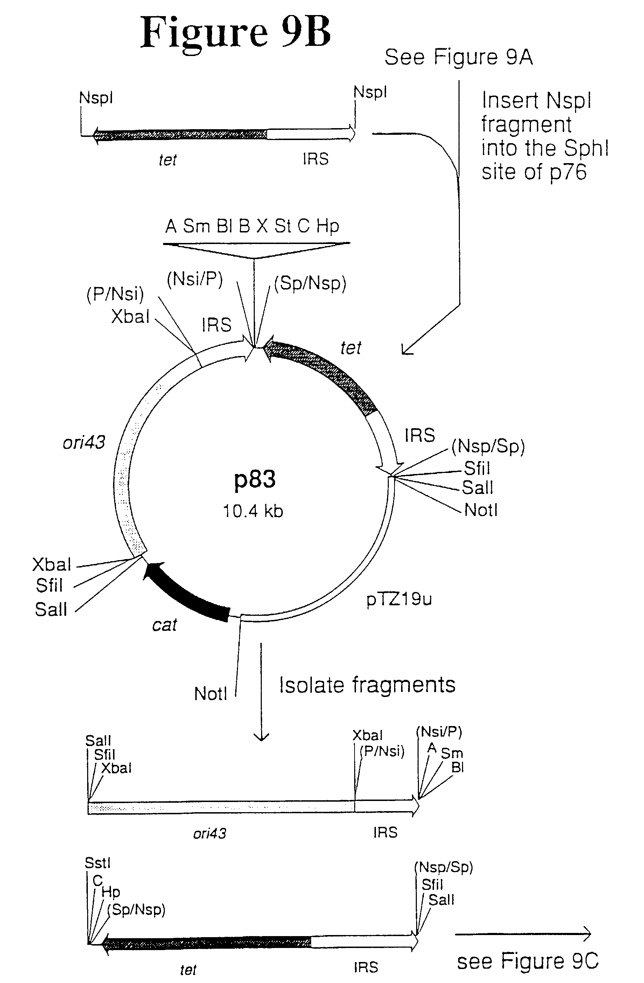

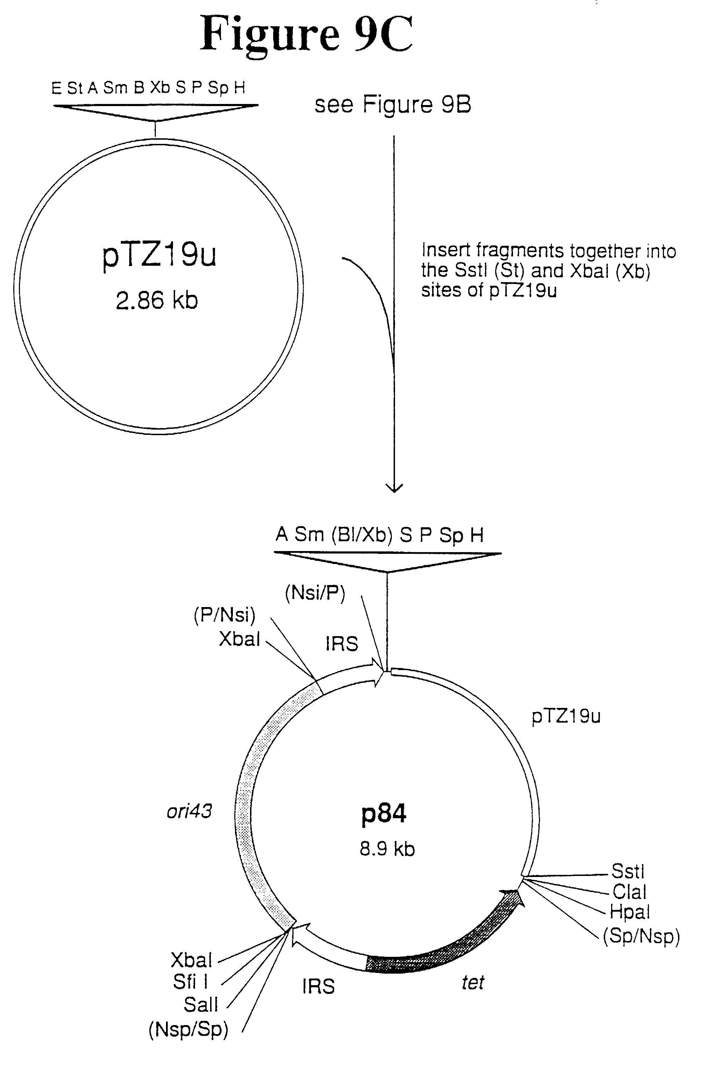

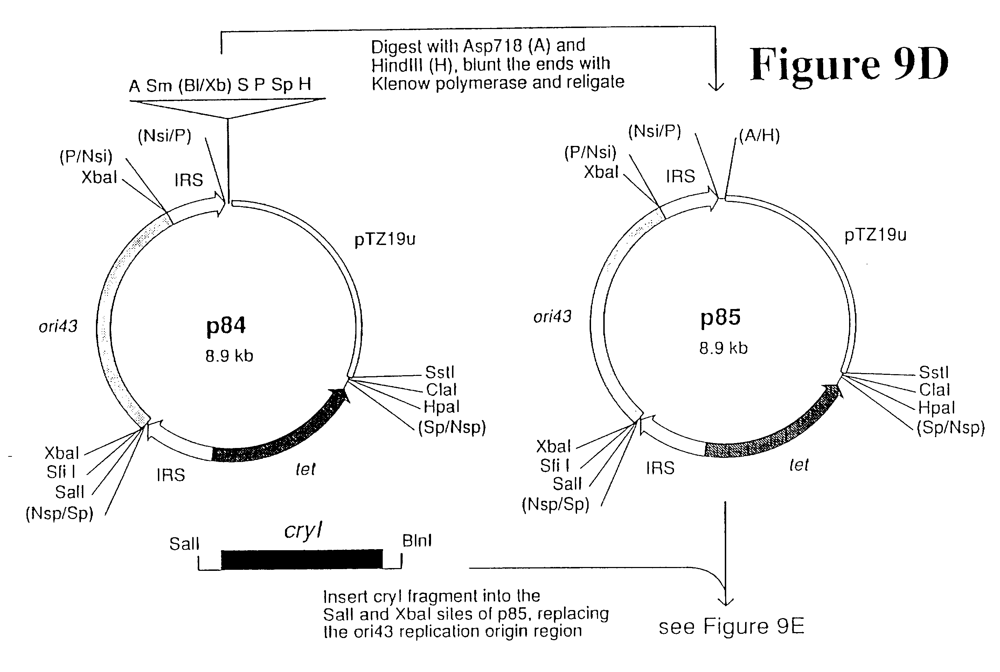

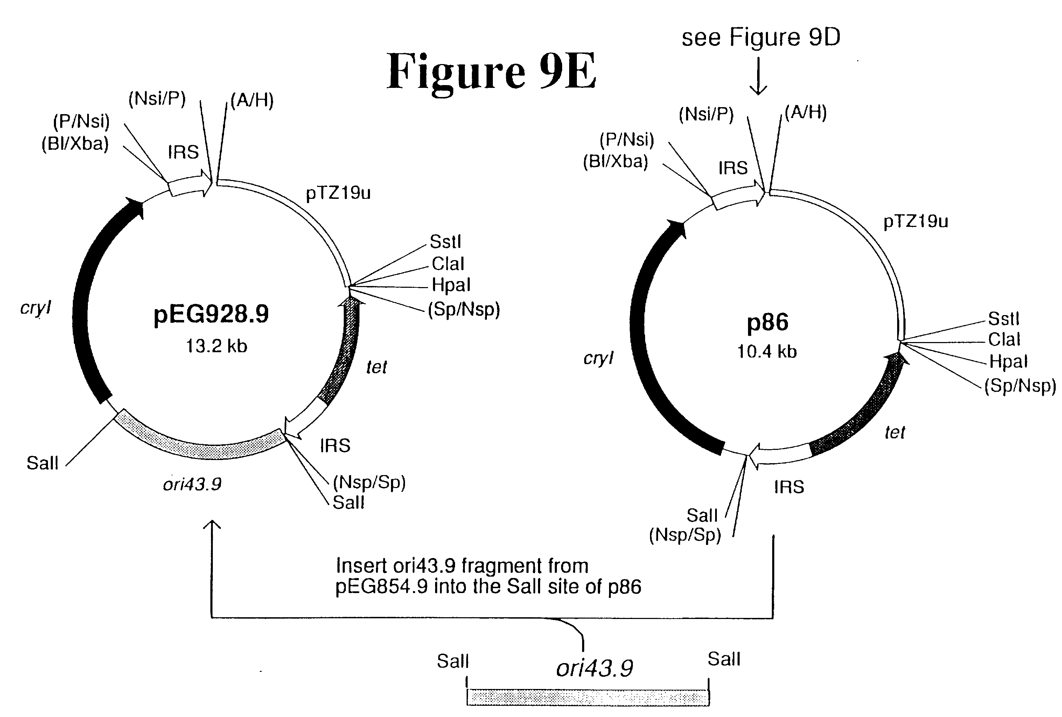

Figure 9 consists of Figures 9A, 9B, 9C, 9D and 9E and is a schematic diagram showing the recombinant DNA procedures used to derive the plasmid shuttle vector pEG928.9 of this invention, which is also shown in Figure 4 and is utilized in the method of Figure 5. Details of the steps shown in this Figure for the derivation of plasmid shuttle vector pEG928.9 are explained in Example 3. Abbreviations are used for some of the restriction endonuclease cleavage sites shown in Figure 9 and these are as follows. For the multiple cloning site in plasmid shuttle vector pEG854 and its derivatives, plasmid clones p76 and p83: P=*Pst*I, A=*Asp*718, Sm=*Sma*I, Bl=*Bln*I, B=*Bam*HI, X=*Xba*I, St=*Sst*I, C=*Cla*I, Hp=*Hpa*I, Sp=*Sph*I. For the multiple cloning site in pTZ19u and its derivative, plasmid clone p84: E=*Eco*RI, St=*Sst*I, A=*Asp*718, Sm=*Sma*I, B=*Bam*HI, Xb=*Xba*I, S=*Sal*I, P=*Pst*I, Sp=*Sph*I, H=*Hind*III. Other abbreviations for restriction sites shown on plasmid shuttle vector pEG928.9 and its precursor plasmid clones are as described for Figure 4, which also provides a description of the circular structural map of pEG928.9.

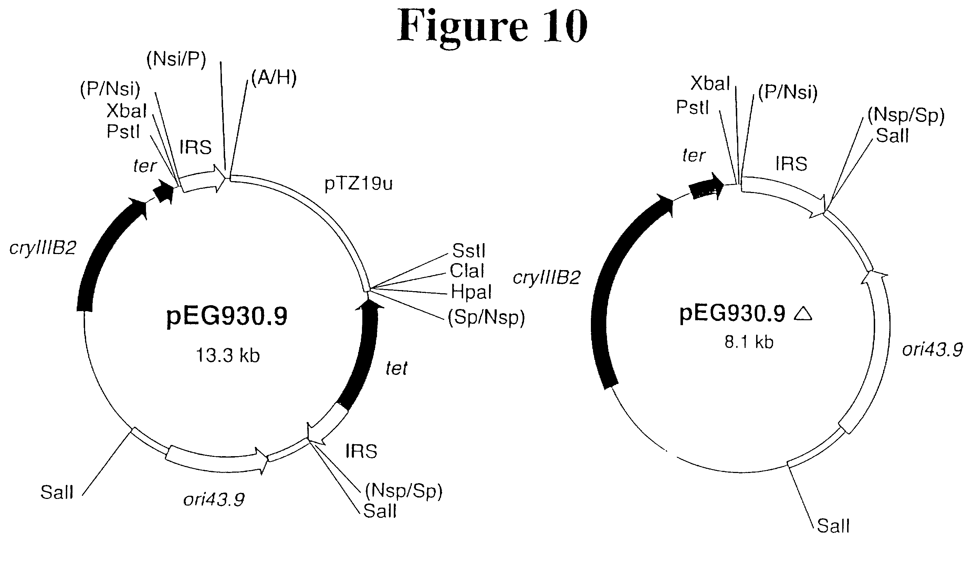

Figure 10 shows circular structural maps of the recombinant plasmid shuttle vector pEG930.9 of this invention, which is 13.3 kb in size and of its derivative plasmid from a site-specific recombination event, plasmid pEG930.9Δ which is 8.1 kb in size. The plasmid shuttle vector pEG930.9 is similar to plasmid shuttle vector pEG928.9 shown in Figure 4 except that the *cry*I-type gene of plasmid pEG928.9 has been replaced with a coleopteran toxin *cryIIIB2* gene (solid long arrow) and a *cryI* transcription terminator, *ter* (short dark shaded arrow). Other symbols and abbreviations for both plasmid pEG930.9 and plasmid pEG930.9Δ are as described for Figure 4.

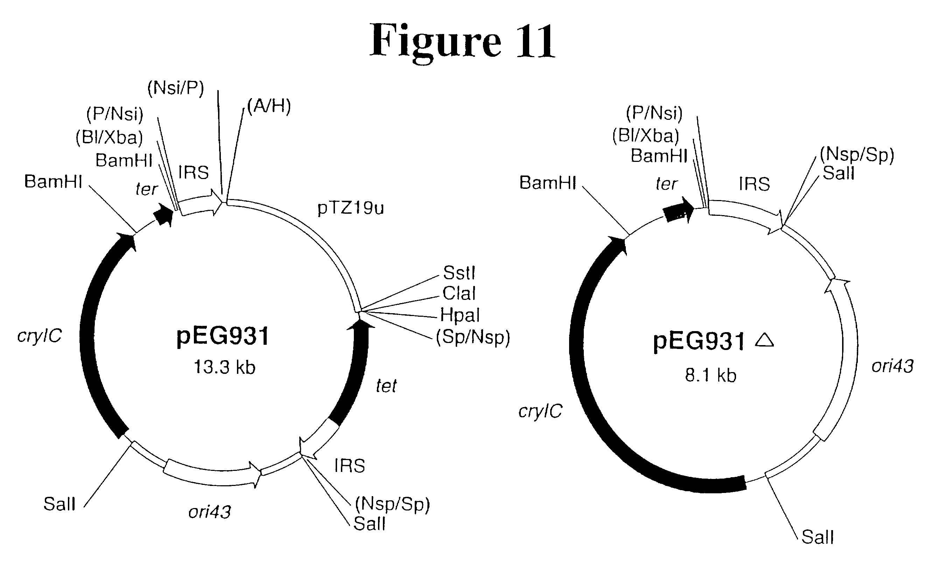

Figure 11 shows circular structural maps of the recombinant plasmid shuttle vector pEG931 of this invention, which is 13.3 kb in size and of its derivative plasmid from a site-specific recombination event, plasmid pEG931Δ which is 8.1 kb in size. The plasmid shuttle vector pEG931 is similar to plasmid shuttle vector pEG928.9 shown in Figure 4 except that the *cryI*-type gene of plasmid pEG928.9 has been replaced with a lepidopteran toxin *cryIC* gene (solid long arrow) and a *cryI* transcription terminator, *ter* (short dark shaded arrow). Other symbols and abbreviations for both plasmid pEG931 and plasmid pEG931Δ are as described for Figure 4.

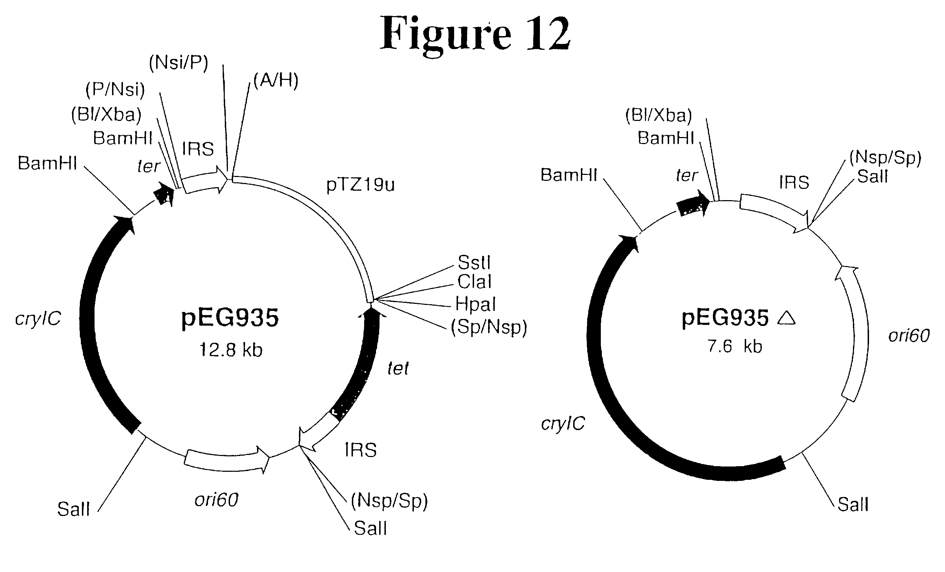

Figure 12 shows circular structural maps of the recombinant plasmid shuttle vector pEG935 of this invention, which is 12.8 kb in size and of its derivative plasmid from a site-specific recombination event, plasmid pEG935Δ which is 7.6 kb in size. The plasmid shuttle vector pEG935 is similar to plasmid shuttle vector pEG931 shown in Figure 11 except that the *B.t.* plasmid origin of replication *ori43* has been replaced with *ori60* (light shaded arrow). Other symbols and abbreviations for both plasmid pEG935 and plasmid pEG935Δ are as described for Figures 4 and 11.

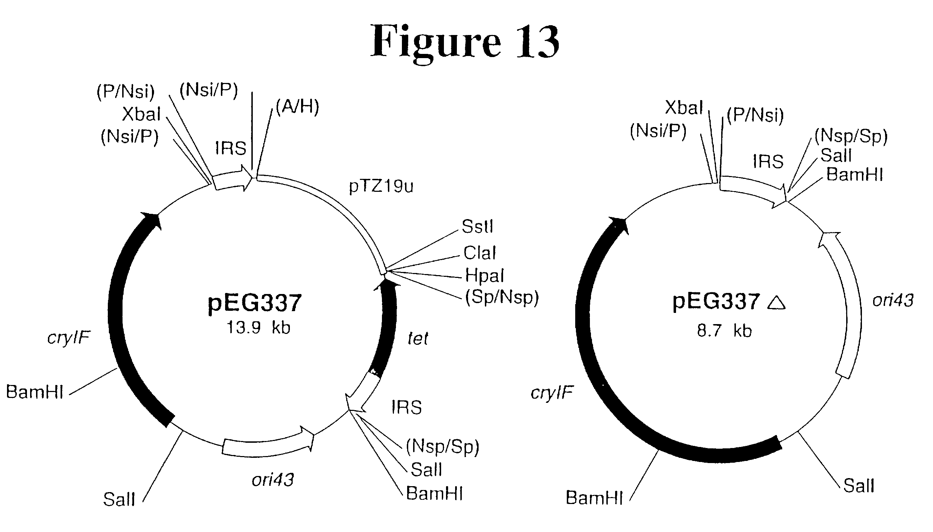

Figure 13 shows circular structural maps of the recombinant plasmid shuttle vector pEG337 of this invention, which is 13.9 kb in size and of its derivative plasmid from a site-specific recombination event, plasmid pEG337Δ which is 8.7 kb in size. The plasmid shuttle vector pEG337 is similar to plasmid shuttle vector pEG931 shown in Figure 11 except that the *cry*IC gene and its accompanying transcription terminator (*ter*) of plasmid pEG931 have been replaced with a lepidopteran toxin *cry*IF gene (solid long arrow). Other symbols and abbreviations for both plasmid pEG337 and plasmid pEG337Δ are as described for Figures 4 and 11.

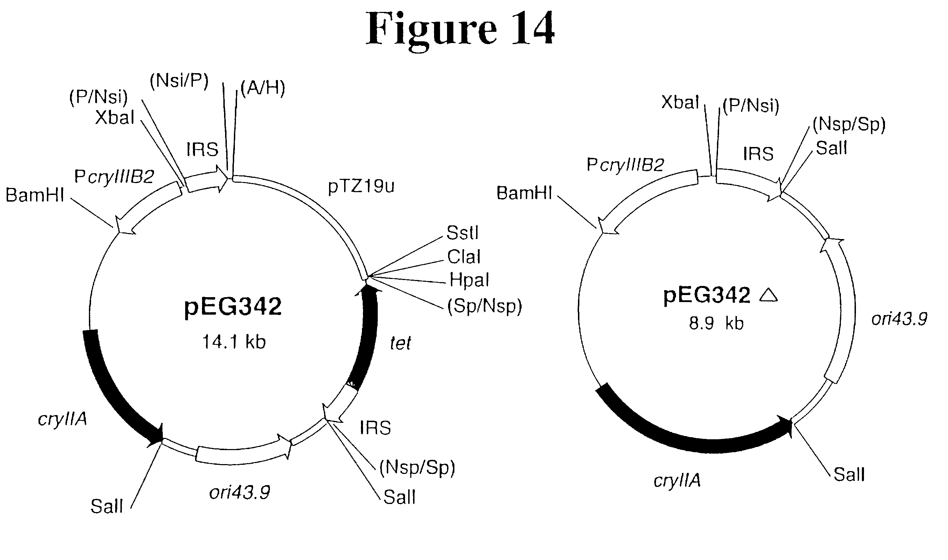

Figure 14 shows circular structural maps of the recombinant plasmid shuttle vector pEG342 of this invention, which is 14.1 kb in size and of its derivative plasmid from a site-specific recombination event, plasmid pEG342Δ which is 8.9 kb in size. The plasmid shuttle vector pEG342 is similar to plasmid shuttle vector pEG930.9 shown in Figure 10 except that a *cryIIA* gene (solid long arrow) replaces the *cryIIIB2* gene and accompanying transcription terminator of pEG930.9. Other symbols and abbreviations for both plasmid pEG342 and plasmid pEG342Δ are as described for Figures 4 and 10.

### Microorganism Deposits

To assure the availability of materials to those interested members of the public upon issuance of a patent on the present application, deposits of the following microorganisms were made prior to the filing of present application with the ARS Patent Collection, Agricultural Research Service Culture Collection, Northern Regional Research Laboratory (NRRL), 1815 North University Street, Peoria, Illinois 61604:

| Bacterial Strain | Recombinant Plasmid | NRRL Accession Number | Date of Deposit |
|---|---|---|---|
| *E. coli* EG7534 | pEG854 | NRRL B-18632 | March 27, 1990 |
| *E. coli* EG7669 | pEG922 | NRRL B-21068 | April 1, 1993 |
| *E. coli* EG7683 | pEG911-1 | NRRL B-21069 | April 1, 1993 |
| *B. thuringiensis* EG2158 | none | NRRL B-18213 | April 29, 1987 |
| *B. thuringiensis* EG7684 | pEG928.9 | NRRL B-21121 | July 7, 1993 |
| *B. thuringiensis* EG7673 | pEG930.9Δ | NRRL B-21070 | April 1, 1993 |
| *B. thuringiensis* EG7674 | pEG928.9Δ | NRRL B-21071 | April 1, 1993 |
| *B. thuringiensis* EG7681 | pEG931Δ | NRRL B-21072 | April 1, 1993 |
| *B. thuringiensis* EG7826 | pEG337Δ | NRRL B-21249 | May 10 1994 |
| *B. thuringiensis* EG7841 | pEG935Δ | NRRL B-21250 | May 10, 1994 |
| *B. thuringiensis* EG7856 | pEG342Δ | NRRL B-21251 | May 10, 1994 |

These microorganism deposits were made under the provisions of the "Budapest Treaty on the International Recognition of the Deposit of Microorganisms for the Purpose of Patent Procedure."

### Description of the Preferred Embodiments

The transposon, or transposable element, of this invention was isolated from *Bacillus thuringiensis* and has been designated as transposon Tn*5401*. Tn*5401* has the nucleotide sequence (SEQ ID NO:1) shown in Figure 1. Two open reading frames within transposon Tn*5401* are also shown in Figure 1, along with their respective deduced amino acid sequences, and these are discussed in more detail below.

A structural map of Tn*5401* is shown in Figure 2 and includes the location of open reading frames within this 4837 basepair (bp) transposon; these elements are indicated by segments with arrowheads. The genes of these open reading frames, *orf1, tnpI* and *tnpA,* are as follows:
*orf1* (open arrow in Figure 2) potentially encodes a cryptic protein, whose significance is not presently known, of 85 amino acids (10.1 kDa) in the 3'-5' direction. The deduced amino acid sequence of orfl is shown in the Sequence Listing accompanying this specification and designated as SEQ ID NO:4. Although not shown in Figure 1, it is derived from the complementary nucleotide sequence extending from nucleotide base positions 351 to 608.
*tnpI* (dark shaded arrow in Figure 2) encodes a protein, designated the resolvase protein of Tn*5401,* of 306 amino acids (35,613 Da) in the 5'-3' direction. In Figure 1, the resolvase gene encodes the resolvase protein having the amino acid sequence (SEQ ID NO:2) located between nucleotide base positions 763 to 1682. The nucleotide base sequence of the resolvase gene, as shown in Figure 1, extends from nucleotide base positions 764 to 1681 (excluding the terminal nonsense codon).
*tnpA* (light shaded arrow in Figure 2) encodes a protein, designated the transposase protein of Tn*5401,* of 1005 amino acids (116,250 Da) in the 5'-3' direction. In Figure 1, the transposase gene encodes the transposase protein having the amino acid sequence (SEQ ID NO:3) located between nucleotide base positions 1755 to 4771. The nucleotide base sequence of the transposase gene, as shown in Figure 1, extends from nucleotide base positions 1756 to 4770 (excluding the terminal nonsense codon).

Another important distinguishing characteristic of transposon Tn*5401* is an internal resolution site, IRS, located 5' to the resolvase open reading frame, within a ∼550 bp *Cla*I-*Nsi*I fragment. This location of the IRS is shown by brackets on the linear structural map of Figure 2 and has been designated in the Figure as "IRS region." In Figure 1, the internal resolution site is located within the DNA fragment extending from nucleotide positions 217 (the initial nucleotide of a *Cla*I restriction endonuclease cleavage site) to 764 (the initial nucleotide of a *Nsi*I restriction endonuclease cleavage site) . The IRS located on this *Cla*I-*Nsi*I fragment is believed to be situated on a ∼150 bp fragment immediately upstream of (5' to) the resolvase open reading frame, i.e., upstream of the *Nsi*I site that initiates the resolvase *tnp*I gene, in particular, within the DNA fragment extending from nucleotide base positions 608 to 763 shown in Figure 1.

Transposon Tn*5401* is also characterized by 53 bp inverted repeats at the termini, which are depicted by the solid black arrowheads in the structural map of Figure 2.

Several restriction endonuclease cleavage sites, i.e., *Nsi*I (two occurrences), *Nsp*I (two occurrences), *Cla*I (one occurrence), *Bss*HII (one occurrence), are also shown on the linear structural map of Tn*5401* in Figure 2 and in the nucleotide sequence of Figure 1, and these are useful for isolating the IRS, as well as the orf1, resolvase and transposase genes.

Transcriptional start sites within Tn*5401* have been mapped by primer extension analysis. Overlapping divergent promoters are located 5' to the resolvase gene: one directs the transcription of both *tnpI* and *tnpA.* Both promoters are derepressed on recombinant plasmids when the *tnpI* and *tnpA* genes are deleted, suggesting that transcription within the transposon is autoregulated, presumably by the resolvase protein.

Conserved sequence elements within the above-noted promoter region apparently serve as recognition sites for the resolvase protein. The conserved sequence elements are a 12 bp sequence ATGTCCRCTAAY (R=purine; Y=pyrimidine), which is believed to be the recognition/binding site for the recombinase protein. This sequence occurs four times in the intergenic region between orf1 and *tnpI.* The dyad sequence ATGTCCACTAATtaatATTAGTGGACAT (nucleotide positions 639-666 in Figure 1), involving two copies of the 12 bp sequence in opposite orientation, may be the site at which site-specific recombination actually occurs during the transposition process. All four copies of the 12 bp sequence are believed to be essential for site-specific recombination to occur. The 12 bp sequence is also located within the terminal inverted repeats of transposon Tn*5401,* thus accounting for the unusual length of these repeats.

Transposon Tn*5401* appears to belong to the class of transposons designated as Tn*3*-type transposons, described by Heffron in "Tn*3* and Its Relatives" in *Mobile Genetic Elements,* Shapiro, ed., Academic Press, Orlando (1983), pp. 223-260. Transposons in the Tn*3* family have the following characteristics:
(1) short inverted repeats at either end, which exhibit homology with other family members,
(2) a high molecular weight protein (transposase) encoded by the transposon and essential for transposition;
(3) a two stage transposition mechanism involving fusion of donor (with transposon) and recipient DNA molecules, including a duplication of the transposon to form a cointegrate molecule, followed by a resolution/recombination event at an internal resolution site within each transposon copy to yield donor and recipient DNA molecules each containing the transposon;
(4) a recombinase protein encoded by the transposon and required for resolution of the cointegrate molecule;
(5) an internal site-specific recombination site that enables the resolvase protein to effect resolution/recombination of the cointegrate molecule; and
(6) a 5-bp duplication of target DNA at the site of insertion, AT-rich target sites apparently being favored.

Members of the Tn*3* family or class of transposons are predominantly derived from gram-negative bacteria, but one exception is Tn*4430* originally isolated from a gram-positive organism and described by Mahillon et al., *EMBO J. 7*:1515-1526 (1988). Until the inventor's discovery of Tn*5401*, the prior art transposon Tn*4430* was the only transposon reported to be originally isolated from a *B.t.* or Bacillus species.

Transposon Tn*5401* is present in *B.t.* var. *morrisoni* strain EG2158 which produces a coleopteran-active protein encoded by a *cryIIIA* gene on an 88 megadalton (MDa) resident plasmid. Tn*5401* is located on two resident plasmids of *B.t.* strain EG2158, a 35 MDa plasmid and a 72 MDa plasmid.

Subsequent to the discovery of Tn*5401*, the inventor has screened numerous other *B.t.* species and determined that Tn*5401* is only rarely found among *B.t.* species. This finding contrasts with the prior art transposon Tn4430 which is widely distributed among *B.t.* species.

Transposon Tn*5401* has been identified by the inventor as also being present in *B.t. "tenebrionis"* (DSM2803), also called *B.t.* "san diego", a *morrisoni* variety that is coleopteran toxic like *B.t.* strain EG2158.

The transposition mechanism of Tn*5401* appears to be similar to that of other transposable elements in the Tn*3* class. The transpositioning functionality of transposon Tn*5401* is not limited to gram-positive bacteria such as *B.t.* but may likely also be demonstrated in gram-negative bacteria such as *E. coli.* For Tn*5401* and other Tn*3*-type transposons, the net outcome of transposition is the insertion of a duplicate copy of the transposon into another (target) plasmid or chromosomal site. The first step of transposition involves the joinder, or cointegration, of a transposon-containing donor plasmid with a target plasmid to form a cointegrate DNA molecule that contains a duplicated copy of the transposon. The transposase gene in the transposon and its encoded protein transposase are essential for this initial step of transposition and apparently effect formation of the duplicate copy of the transposon in the cointegrate plasmid.

The second step of transposition involves a site-specific recombination event, in which the original transposon-containing donor plasmid and target plasmid, the latter now containing a copy of the transposon, are formed from the cointegrate plasmid. The site-specific recombination event occurs at a specific site in the transposon, the internal resolution site, referred to herein as the IRS. The resolvase protein encoded by the resolvase gene in the transposon apparently catalyzes a recombination event between the two internal resolution sites of the duplicate transposons, resulting in the formation of the transposon-containing donor plasmid and a target plasmid that has been modified by incorporation of a copy of the transposon.

The site-specific recombination event occurs between the duplicate internal resolution sites in the cointegrate molecule and has the effect of removing, or excising, the DNA located between the duplicate sites. The resolvase/recombinase protein encoded by the resolvase gene in the transposon can apparently catalyze this site-specific recombination event on any plasmid molecule containing two copies of the internal resolution site. These aspects of the transposition mechanism and of transposon Tn*5401* are utilized in the site-specific recombination system of this invention, which is described in more detail below.

Transposon Tn*5401* is also useful in transposon tagging to isolate genes of interest, e.g., in mutational studies of protein toxin genes in *B.t.* Transposons are known to be useful as molecular probes and genetic markers. In the event a transposon inserts itself into a gene, the gene is inactivated and a mutant phenotype is produced. Tn*5401* is especially useful for such gene studies in *B.t.* since this transposon favors insertion into plasmid DNA rather than chromosomal DNA. The present invention, for this reason, includes use of recombinant plasmids containing Tn*5401*, preferably in conjunction with a selectable and/or screenable marker, e.g., an antibiotic resistance marker gene, functional in the host microorganism harboring the recombinant plasmid.

This invention also extends to mutants and derivatives (hereinafter referred to collectively as "mutants") of (i) transposon Tn*5401* which are capable of functioning as transposable elements, (ii) the resolvase gene of Tn*5401* whose resolvase/recombinase gene products have resolving functionality, and (iii) the transposase gene of Tn*5401* whose transposase gene products have transpositioning functionality. Methods for creating or obtaining such mutants are well known to those skilled in the art of molecular cloning.

In another aspect of this invention, transposon Tn*5401* has been isolated on a recombinant plasmid designated pEG922, whose circular restriction map is shown in Figure 3. Transposon Tn*5401* is located on a ∼7 kb SstI-SstI DNA fragment in plasmid pEG922 and the *tnpI* and *tnpA* genes of Tn*5401* are shown in Figure 3. As is evident from Figure 3, the transposon Tn*5401* has been tagged with a selectable marker gene, *tet,* for tetracycline antibiotic resistance. The derivation of recombinant plasmid pEG922 is described in more detail in Example 2. Plasmid pEG922 is useful for its ability to transform gram-positive bacteria such as *B. thuringiensis, B. cereus, B. megaterium,* and *B. subtilis,* as well as transform gram-negative bacteria such as *E. coli,* and such transformed bacteria are capable of expressing the *tpnI* and *tnpA* genes on Tn*5401.* Consequently, transformed bacteria containing pEG922 are able to produce the recombinase/resolvase protein gene product of *tnpI.* Likewise, expression of the transposase protein gene product of *tnpA* enables the transposon to exhibit transpositioning functionality. Besides plasmid pEG922, other recombinant plasmids containing the transposon Tn*5401* are also within the scope of this invention.

Other embodiments of this invention include a bacterium, e.g., *Bacillus thuringiensis* or *E. coli,* transformed with a recombinant plasmid carrying transposon Tn*5401. E. coli* strain EG7669 harbors recombinant plasmid pEG922 and is one such strain.

Additional embodiments of this invention also include recombinant plasmids containing either the *tnpA* gene or *tnp*I gene, or both, of Tn*5401*, as well as bacteria transformed with such recombinant plasmids and capable of expressing the gene or genes in such plasmids. Preferred bacteria include Bacillus species, particularly *B.t.,* and include *E. coli.*

The transposable element Tn*5401* of this invention provides the basis for a site-specific recombination system for construction of stable recombinant plasmids in insecticidal recombinant *B.t.* strains that contain only DNA that is native to *B.t.* and that are free of foreign (non-*B.t*. derived) DNA. This site-specific recombination system utilizes the internal resolution site of Tn*5401*, two copies of the internal resolution site being incorporated into a recombinant *B.t.* plasmid in the same orientation. The two copies of the internal resolution site are used to bracket all DNA elements foreign to *B.t.*, e.g., selectable marker genes, *E. coli* replicons and other DNA useful in the construction and characterization of such a recombinant plasmid. Elimination of the foreign DNA is accomplished by a site-specific recombination event involving the internal resolution sites. This event is effected enzymatically by using the site-specific resolvase/recombinase protein that is encoded by the *tnpI* gene of Tn*5401*. The resultant recombinant plasmids are completely free of foreign DNA. Such recombinant *B.t.* plasmids, harboring a desired *B.t.* toxin gene or combinations of toxin genes, may be used to construct *B.t.* strains for use as the active ingredient in commercial *B.t.*-based insecticides.

An essential aspect of the site-specific recombination system of this invention is a plasmid shuttle vector having the following elements: (i) an origin of replication functional in *B.t.;* (ii) DNA not native to *B.t.;* and (iii) two identical internal resolution sites oriented in the same direction and flanking the DNA not native to *B.t.* The two identical internal resolution sites thus segregate the DNA not native to *B.t.* from the DNA native to *B.t.* Use of this plasmid shuttle vector in a *B.t.* host strain facilitates removal or excision of the non-*B.t.* DNA via a site-specific recombination event involving, i.e., between, the two internal resolution sites. The site-specific recombination event is catalyzed by the introduction of resolvase/recombinase protein that recognizes the particular IRS site utilized in the plasmid shuttle vector.

The plasmid shuttle vector optionally and preferably contains at least one insecticidal protein toxin gene that is intended to be introduced into the recombinant *B.t.* strain construct. This gene (or genes) is situated on the plasmid shuttle vector in a location outside of the DNA not native to *B.t.* and outside of the internal resolution sites that flank the foreign DNA.

One preferred embodiment of the plasmid shuttle vector of this invention is plasmid pEG928.9, whose circular structural map is shown in Figure 4. Details of the derivation of plasmid shuttle vector pEG928.9 are described in Example 3.

The duplicate copies of the internal resolution sites (IRS) utilized in this plasmid shuttle vector are desirably derived from, or identical to, an IRS of a Tn*3*-type transposon.

Particularly suitable Tn*3*-type transposon sources for the IRS are transposons native to *B.t.* such as transposons Tn*4430* and Tn*5401*. These IRS-source transposons are well suited for construction of insecticidal recombinant *B.t.* strains having no DNA that is not native to *B.t.* A disadvantage of Tn*4430* as the IRS source is the widespread existence of this transposon in *B.t.* strains. The host *B.t.* strain selected for construction of the recombinant *B.t.* should be free of the transposon utilized as the IRS source in the plasmid shuttle vector, so as to avoid possible interference with the site-specific recombination event in the method of this invention.

For this reason, duplicate copies of the internal resolution site in the plasmid shuttle vector are most preferably derived from, or identical to, the internal resolution site of transposon Tn*5401*. As noted earlier in the discussion of Tn*5401*, this transposon is infrequently found in *B.t.* species, a fact that makes most *B.t.* strains suitable candidates as host strains for the site-specific recombination method of this invention.

It should be noted, however, that internal resolution sites or site-specific recombination sites from other sources are likewise usable in this plasmid shuttle vector and in the site-specific recombination system of this invention, if the fact of the IRS or the site-specific recombination site not being native to *B.t.* is not critical.

In the plasmid shuttle vector of this invention, the origin of replication functional in *B.t.* is preferably a replication origin that is native to *B.t.,* i.e., is identical to or derived from a *B.t.* plasmid origin of replication. *B.t.* replication origins from large *B.t.* plasmids, i.e., plasmids larger than about 20-25 mDa in size, are preferred since such replicons are more likely to produce stable recombinant plasmids than replicons derived from small *B.t.* plasmids, which typically replicate by a different mechanism, i.e., rolling circle replication.

Preferred *B.t.* plasmid origins of replications are *ori43 ori60* and *ori44,* described in PCT International Patent Publication No. WO 91/18102, published November 28, 1990 by applicant Ecogen Inc. The *ori43* replicon is present in plasmid shuttle vector pEG854, which is contained in *E. coli* strain EG7534 which is a deposited microorganism described in WO 91/18102. The preferred *B.t.* origin of replication also includes mutants of these three and other *B.t.* replicons, particularly those mutants exhibiting higher copy numbers than the progenitor replicon. One such replicon, *ori43.9*, is utilized in plasmid shuttle vector pEG928.9 of this invention and is preferred because its high copy number characteristic often promotes increased expression levels of insecticidal toxin protein genes located on the same plasmid.

The plasmid shuttle vector of this invention also contains DNA elements not native to *B.t.,* and this foreign DNA is flanked, or segregated, by the duplicate copies of the internal resolution sites. The foreign DNA is excised from the plasmid shuttle vector by the site-specific recombination event between the two internal resolution sites, but this non-native DNA can serve many useful purposes prior to the recombination event. Examples of such useful foreign DNA are selectable and/or screenable marker genes, such as antibiotic resistance genes functional in *B.t.* or *E. coli* or other cloning hosts; origins of replication functional in *E. coli;* and origins of replication functional in gram-positive microorganisms other than *B.t.,* e.g., in Bacillus species. Other DNA elements not native to *B.t.* may also be useful in the construction, development and characterization of insecticidal recombinant *B.t.* constructs, and these are also within the scope of the term "DNA not native to *B.t.",* as used herein. The term "DNA not native to *B.t.",* as used herein, is not intended to cover short polynucleotide stretches that are derived from multiple cloning sites or that are other synthesized, non-biological DNA.

The choice of the insecticidal protein toxin gene that is optionally and preferably present in the plasmid shuttle vector is not critical. The insecticidal protein toxin gene is normally selected to enhance the insecticidal characteristics of the *B.t.* host strain transformed with the plasmid shuttle vector. The insecticidal toxin gene is preferably selected from among wild-type or recombinant *B.t.* toxin genes. Exemplary *B.t.* toxin genes are those described by Höfte and Whiteley, 1989, as well as more recently reported *B.t.* genes such as *cryIF, cryIIIB2* and *cryIIIB3.*

Bacteria transformed with the plasmid shuttle vector and capable of expressing at least one of the genes in the plasmid shuttle vector are also within the scope of this invention and are desirably selected from the group consisting of *Bacillus thuringiensis* and *E. coli.* One such recombinant *Bacillus thuringiensis* strain is *B.t.* strain EG7684 which contains plasmid shuttle vector pEG928.9.

It should be evident that the site-specific recombination system of this invention is not strictly limited to *B.t.* but is equally applicable to the construction of other Bacillus species recombinant constructs, if suitable changes are made in the plasmid shuttle vector, e.g., selection of an origin of replication functional in the selected Bacillus host species, DNA not native to the selected host species and optional insecticidal protein toxin genes capable of being expressed by the selected replicon.

The site-specific recombination system of this invention is schematically exemplified in Figure 5, which illustrates plasmid shuttle vector pEG928.9 undergoing a site-specific recombination event catalyzed with recombinase/resolvase protein produced by the *tnpI* gene of the Tn*5401*-containing plasmid pEG922. The resultant plasmid pEG928.9Δ contains a single copy of the IRS, lacks DNA not native to *B.t.,* and contains a *B.t.*-derived replicon and a *B.t. cry*I-type protein toxin gene. The method of this invention as exemplified in Figure 5 is described in detail in Example 5.

A preferred method of this invention, for constructing a recombinant *B.t.* strain containing no DNA elements foreign to *B.t.*, involves (a) transforming a host *B.t.* strain with a plasmid shuttle vector containing (i) an origin of replication native to *B.t.;* (ii) DNA not native to *B.t.* and useful in the construction of recombinant *B.t.* strains, selected from the group consisting of selectable marker genes, origins of replication functional in *E. coli,* and origins of replication functional in Bacillus host species other than *B.t.;* (iii) one or more insecticidal *B.t.* protein toxin genes; and (iv) two identical internal resolution sites oriented in the same direction and flanking the DNA not native to *B.t.,* the sites being the same as an internal resolution site from a Tn*3*-type transposon native to *B.t.;* (b) introducing into the transformed *B.t.* strain a resolvase protein to effect a site-specific recombination event involving the internal resolution sites, thereby excising from the plasmid shuttle vector the DNA not native to *B.t.;* and (c) recovering a recombinant *B.t.* strain containing a recombinant plasmid capable of replicating in the *B.t.* strain and containing (i) an origin of replication native to *B.t.;* (ii) one or more insecticidal protein toxin genes; and (iii) a single internal resolution site, derived from the site-specific recombination event.

In this method, the resolvase/recombinase protein should correspond to that produced by the resolvase/recombinase gene in the Tn*3*-type transposon used as the IRS source. The only requirement is that the resolvase/recombinase protein recognize the particular IRS site utilized.

The elements of the recombinant plasmid present in the recovered recombinant *B.t.* strain correspond, of course, to the same elements in the plasmid shuttle vector originally introduced into the host *B.t.* strain. Selection of the elements of the plasmid shuttle vector used in this method is governed by the same considerations discussed earlier for the plasmid shuttle vector of this invention.

Preferred Tn*3*-type transposon sources for the duplicate IRS sites in the plasmid shuttle vector are Tn*4430* and Tn*5401*.

Introduction of the resolvase protein into the *B.t.* transformant containing the plasmid shuttle vector serves to effect a site-specific recombination event between the IRS sites in the vector. This introduction of the protein catalyzing agent may be accomplished by transforming the *B.t.* transformant with a second recombinant plasmid containing a resolvase gene and capable of expressing the resolvase protein. To facilitate efficient removal of the resolvase gene containing plasmid from the *B.t.* host strain following site-specific recombination, this plasmid desirably contains a temperature-sensitive replicon or other means for effecting its deletion and an antibiotic selectable marker gene different from the selectable marker gene carried on the plasmid shuttle vector. This approach is utilized in the site-specific recombination method described in Example 5.

Alternative means exist for introducing the recombinase protein into the transformed *B.t.* host strain containing the plasmid shuttle vector. One technique involves the direct introduction of the protein into the transformed *B.t.* cells, via the transient introduction of the recombinase protein via electroporation, lipofection or the like.

A second approach involves insertion of the recombinase gene into the plasmid shuttle vector within the non-*B.t.* DNA region flanked by the IRS sites. For IRS sites the same as that of transposon Tn*5401*, a mutant of the corresponding resolvase gene, *tnpI,* should produce a recombinase protein that is thermosensitive, being inactive at ∼37°C but active at ∼30°C. This *tnpI*^{*ts*} variant could be obtained by a variety of well-known *in vitro* mutagenesis procedures, including chemical mutagenesis of the *tnpI* gene, followed by selection for *tnpI* variants that catalyze recombination at 30°C but not at 37°C. Transformation of a suitable *B.t.* host strain with a *tnpI*^{*ts*}-containing plasmid shuttle vector at a temperature of 37°C will prevent expression of the *tnpI* gene, but this will allow for selection of transformants containing the plasmid shuttle vector. Subsequently, the *B.t.* transformants are grown at a temperature of 30°C, resulting in expression of a functional recombinase protein and excision of the foreign DNA elements, as well as excision of the *tnpI*^{*ts*} gene, since both are contained within the non-*B.t.* DNA region flanked by the IRS sites.

Both of these alternative procedures for introducing the recombinase protein to effect site-specific recombination avoid the need to introduce a second recombinant plasmid, i.e., one containing an expressible recombinase gene, into the transformed *B.t.* strain and avoid the need to thereafter delete the same second recombinant plasmid following the recombination event.

The site-specific recombination system of this invention yields recombinant toxin plasmids that possess a unique combination of elements. The recombinant plasmids, capable of replicating in *B.t.* bacteria, contain at least one insecticidal protein toxin gene, an origin of replication functional in *B.t.,* and a single internal resolution site (or other single site-specific recombination site).

In a preferred embodiment, the single internal resolution site of the recombinant plasmid is derived from a Tn*3*-type transposon or is identical to the IRS in such a transposon. The Tn*3*-type transposon IRS source is desirably one that is native to *B.t.* The internal resolution site is preferably identical to the IRS of transposon Tn*4430* or, more preferably, transposon Tn*5401*.

The origin of replication in these recombinant toxin plasmids is preferably native to *B.t.* The *B.t.*-functional origin of replication is preferably derived from, or identical to, a replicon of a large *B.t.* plasmid, for the same reasons discussed previously for the plasmid shuttle vector of this invention.

The bacteria containing these recombinant toxin plasmids are preferably *Bacillus thuringiensis* but other bacterial hosts can be used, provided that the replicon in the plasmid is capable of functioning in such a non-*B.t.* host.

Particularly preferred recombinant *B.t.* constructs containing the recombinant plasmids of this invention are described in Example 5. It should be evident from the discussion in Example 5 that this invention provides the means to construct a wide variety of insecticidal recombinant *B.t.* strains containing no DNA elements not native to *B.t.* The site-specific recombination system of this invention facilitates construction of insecticidal recombinant *B.t.* strains with good stability characteristics, exhibiting limited horizontal transfer of their recombinant plasmids. The invention also permits the rapid construction and evaluation of recombinant *B.t.* constructs with unique complements of *B.t.* toxin genes that previously could not be quickly and easily realized with the prior art techniques.

The basic methods employed in the construction and evaluation of the recombinant plasmids described in this specification are generally well-known to those proficient in the art of molecular cloning. Descriptions of these general laboratory procedures and definitions of nomenclature may be found in Maniatus et al., *Molecular Cloning, A Laboratory Manual,* Cold Spring Harbor Laboratory, Cold Spring Harbor, NY (1982) and in a subsequent edition by Sambrook et al. (1989).

The following Examples provide further explanation of the invention and methods of its use.

### Example 1

### Isolation and DNA Sequence Analysis of Tn5401

The transposon Tn*5401* of this invention was initially isolated from copies of a recombinant plasmid which had been introduced into *B.t.* var. *morrisoni* strain EG2158 by electroporation. Transposon Tn*5401* was subsequently shown to be located on two resident plasmids (35 and 72 MDa in size) of *B.t.* strain EG2158 from which it had apparently "jumped", or transposed itself, into the recombinant plasmids.

The recombinant plasmid pEG911, shown in Figure 6, was used as the donor plasmid in transformation studies with *B.t.* strain EG2158, employing the conventional electroporation protocol described by Mettus et al., *Applied and Environ. Microbiol. 56:* 1128-1134 (1990). Restriction enzyme analysis of DNA from recombinant plasmids isolated from several *B.t.* strain EG2158 transformants indicated that a subpopulation of the pEG911 plasmids contained a common ∼5 kilobase (kb) DNA insert.

Recombinant plasmid DNAs from two different, independent *B.t.* strain EG2158 transformants, each containing the 5 kb DNA insert, were recovered in *Escherichia coli* by selecting for *E. coli* transformants resistant to 50 lg/ml ampicillin. Linear restriction maps of two independent plasmid clones, designated pEG911-1 and pEG911-3, are shown in Figure 7. Each of these plasmids contains a ∼5 kb insertion (shown as a solid black box and designated Tn*5401)* upstream of the *cryIIIB2 B.t.* protein toxin gene (shown as a white arrow) but in opposite orientations as indicated by the placement of the unique *Bss*HII and *Cla*I restriction endonuclease cleavage sites.

Plasmid pEG911-1 was chosen as the template for DNA sequence analysis of the ∼5 kb DNA insert. Double-stranded DNA was sequenced according to the well-known dideoxy chain termination method using [alpha-³⁵S)dATP. Synthetic oligonucleotides were generated to serve as primers for DNA sequence analysis. Sequence analysis was initiated using primers complementary to DNA flanking the DNA insert. Subsequently, sequencing primers were synthesized as needed based on the derived DNA sequences.

The complete nucleotide sequence of the ∼5 kb insert of plasmid pEG911-1 is shown in Figure 1 and has been identified as a transposable element based on an analysis of its characteristics. This transposable element, having a size of 4837 basepairs, has been designated as transposon Tn*5401.*

For microorganism deposit purposes, plasmid pEG911-1, containing transposon Tn*5401* on the ∼5 kb insert, was introduced into an *E. coli* host strain, *E. coli* JM110, to yield *E. coli* strain EG7683.

A linear structural map of Tn*5401 is* shown in Figure 2, which includes the location of open reading frames denoted by arrows: *orf1* (open arrow) potentially encodes a cryptic protein of unknown significance, of 85 amino acids in the 3'-5' direction; *tnpI* (dark shaded arrow) encodes a protein in the 5'-3' direction which has been designated the resolvase protein of Tn*5401* and which contains 306 amino acids; and *tnpA* (light shaded arrow) encodes a protein in the 5'-3' direction which has been designated the transposase protein of Tn*5401* and which contains 1005 amino acids. Not shown on Figure 2 is a second open reading frame, *orf2,* which, like *orf1,* is oriented in the 3'-5' direction and potentially encodes a small cryptic protein which is of unknown significance and which contains 74 amino acids. Although not shown in Figure 1, this deduced second small cryptic protein is derived from the complementary nucleotide sequence extending from nucleotide base positions 122 to 343.

Another distinguishing characteristic of Tn*5401* shown in Figure 2 is the location of its internal resolution site, located within the bracketed DNA fragment designated as "IRS region" and is likely located near the promoter region for the *tnpI* gene. Yet another characteristic of Tn*5401* is the inverted repeats of 53 basepairs at either end of the transposon, shown as black arrowheads at both ends of the structural map in Figure 2.

Analysis of Tn*5401* and its elements was carried out using available computer databases to determine homologies with other DNA in the databases. These analyses indicate that Tn*5401* belongs in the Tn*3* family of transposons and, based on comparisons of its resolvase and transposase proteins, is related to Tn*4430*, the prior art transposon which is also present in *B.t.* species.

The 36 kDa resolvase protein of Tn*5401* shows 25% amino acid sequence identity to the resolvase/recombinase protein of the Tn*4430*, but no significant identity to the corresponding resolvase protein of Tn*3*.

The 116 kDa transposase protein of Tn*5401* shows 29% amino acid sequence identity to the transposase protein of Tn*4430* and 44% identity to the transposase protein of Tn*3*.

The respective sequences of the 10.1 kDa cryptic protein of *orf1* and of the 8.6 kDa cryptic protein of *orf2* in Tn*5401* showed no apparent homology to sequences in the databases.

### Example 2

### Construction of Transposon Vector pEG922 Containing Tn5401

The ability of the isolated transposon Tn*5401* to transpose in a *B.t.* strain is shown in this Example. As described in more detail below and as shown in Figure 8, the cloned transposon Tn*5401* was tagged with an antibiotic resistance marker gene, then inserted into a temperature-sensitive recombinant shuttle vector functional in *B.t.* The resultant Tn*5401*-containing shuttle vector, designated pEG922 and a circular structural map of which is shown in Figure 3, was used to transform a plasmid-free *B.t.* strain, and measurements of its transposition efficiency were obtained.

The recombinant DNA manipulations involved in the construction of pEG922 were carried out using conventional techniques, familiar to those skilled in the art of cloning DNA, and are shown in schematic form in Figure 8.

A ∼3 kb *Bss*HII-*Sst*I DNA fragment from plasmid pEG911-1, shown in Figure 7 and containing a portion of Tn*5401,* and a ∼2.3 kb *Bss*HII-*Sst*I DNA fragment from pEG911-3, also shown in Figure 7 and containing the remaining portion of Tn*5401*, were cloned together as shown in Figure 8A into the *Sst*I site of the well-known *E. coli* cloning vector pTZ19u. The resulting cloned DNA fragment contained a reconstructed, complete Tn*5401* transposon with flanking *SstI* sites as shown in Figure 8A.

The Tn*5401* transposon in this cloning vector was next tagged with a tetracycline antibiotic resistance marker gene (tet) from the well-known *Bacillus cereus* plasmid pBC16, contained on a ∼1.7 kb *Acc*I-*Acc*I DNA fragment, by insertion of this fragment into the unique *ClaI* site on transposon Tn*5401,* whose structural map is shown in Figure 2. It should be noted that an *Acc*I cleavage site is compatible with a *Cla*I site. The tagged transposon *Tn*5401 was subsequently recovered on a ∼7 kb *Sst*I fragment, as shown in Figure 8A.

As shown in Figure 8B, the tet-tagged transposon Tn*5401* on the *Sst*I fragment was inserted into the unique SstI site of a temperature-sensitive shuttle vector, pEG491, whose circular structural map is shown in Figure 8B. Plasmid pEG491 is a shuttle vector derived in part from the well-known cloning vector pUC18, which also contains a chloramphenicol acetyl transferase antibiotic resistance gene *(cat)* and a temperature-sensitive replicon *(rep*^{*ts*}*)* functional in gram-positive bacteria, the latter element being derived from plasmid pE194ts. The temperature-sensitive replicon *rep*^{*ts*} cannot operate at temperatures at or above 37°C, in contrast to most *B.t.*-derived replicons which operate at higher temperatures.

Plasmid pE194ts is described by Villafane et al. in *J. Bacteriol. 169*:4822-4829 (1987), and its use with a transposon (Tn*917*) is described by Youngman et al. at p.101-102 in *Plasmids: a practical approach,* Hardy, ed., IRL Press, Oxford, England (1987) pp. 79-103.

The resulting transposon vector, designated pEG922, contains the *tet*-tagged transposon Tn*5401* on a ∼7 kb fragment inserted into the *Sst*I site of plasmid pEG491, as shown in Figure 8B which contains a circular structural map of plasmid pEG922. Plasmid pEG491, as shown in Figure 8B, contains the *E. coli* replicon pUC18 (thin black segment), a chloramphenicol acetyl transferase gene, cat (thick black segment), and a temperature-sensitive replicon, *rep*^{*ts*}, from plasmid pE194ts which cannot operate at temperatures at or above 37°C. At the top of plasmid pEG491 is a multiple cloning site. Abbreviations for the restriction endonuclease cleavage sites in the multiple cloning site are explained in the discussion of Figure 3 (which also shows the structural map of pEG922) appearing in the Brief Description of the Drawings.

For microorganism deposit purposes, plasmid pEG922 was also used to transform a host strain, *E. coli* strain GM2163, to yield *E. coli* strain EG7669.

The recombinant shuttle plasmid pEG922 was introduced into a plasmid-free *B.t.* var. *kurstaki* strain EG7566 by electroporation following the procedures of Mettus et al. (1990). Transposition of Tn*5401* within the resulting transformed *B.t.* strain was measured using the procedure described by Youngman in *Plasmids: a practical approach,* Hardy, ed., IRL Press, Oxford, England (1987) pp 79-103. Transposition frequencies were measured as the quotient of the number of tetracycline resistant colonies observed at a temperature of 41°C divided by the number of chloramphenicol resistant colonies at a temperature of 30°C. Transposition frequencies of 10⁻⁴ were routinely obtained in transformed *B.t.* strain EG7566, indicating that transposon Tn*5401* contained on plasmid pEG922 was functional in *B.t.*

Subsequent studies by the inventor with plasmid pEG922 in plasmid-containing *B.t.* strains have shown that transposon Tn*5401* favors transposition into other plasmids, rather than into chromosomal DNA, and exhibits an apparent preference for AT-rich regions of DNA. Transposition frequencies observed for one plasmid-containing *B.t.* strain were generally two to three orders of magnitude higher than those obtained with the plasmid-free *B.t.* strain EG7566, typically being about 10⁻¹-10⁻².

### Example 3

### Construction of Plasmid Shuttle Vector pEG928.9

The plasmid shuttle vector pEG928.9 of this invention is useful for insecticidal recombinant *B.t.* strain development and is illustrated in Figure 4. Example 3 describes the construction of plasmid shuttle vector pEG928.9, and the recombinant DNA procedures involved in this construction are schematically illustrated in Figure 9.

The plasmid shuttle vector pEG854 was used as a starting point for the derivation of plasmid pEG928.9, as shown in Figure 9A. Plasmid shuttle vector pEG854 is contained in *E. coli* strain EG7534, which is a deposited microorganism described in PCT International Patent Publication WO 91/18102.

Plasmid shuttle vector pEG854 contains a *B.t.* plasmid origin of replication, *ori43* (light shaded segment), a multiple cloning site (shown at the top of the plasmid), the *E. coli* replicon pTZ19u, and a chloramphenicol acetyl transferase gene, *cat* (black arrow). The internal resolution site, IRS, of transposon Tn*5401* (see Figure 2) contained on a ∼650 bp *Nsi*I-*Nsi*I fragment from plasmid pEG911-1 (see Figure 7) was inserted into the unique *Pst*I site in the multiple cloning site of pEG854 to yield the cloned plasmid p76.

A second copy of the IRS from transposon Tn*5401*, contained on a 2.5 kb *Nsp*I-*Ns*pI fragment from plasmid pEG922 (see Figure 3), was inserted into the unique *Sph*I site in the multiple cloning site of p76 to yield the cloned plasmid p83, as shown in Figure 9B. Both IRS copies were in the same orientation (clockwise) in plasmid p83, as shown by the open arrows. The IRS copy in the *Nsp*I-*Nsp*I fragment also contained the tetracycline antibiotic resistance gene, *tet* (dark shaded arrow), that had been introduced into pEG922 (see Figures 3 and 8) as a selectable marker tag for transposon Tn*5401*.

As shown in Figure 9B, plasmid p83 was digested with *Sal*I, *Bln*I and *Sst*I, and fragments containing the IRS sites were isolated: a 3.5 kb *Sal*I-*Bln*I fragment containing the IRS and *B.t.* origin of replication *ori43,* and a 2.5 kb *Sst*I-*Sal*I fragment containing the IRS and the *tet* selectable marker gene. As shown in Figure 9C, these two IRS-containing fragments were inserted together into the *Sst*I and *Xba*I sites in the multiple cloning site of the well-known *E. coli* 2.86 kb phagemid vector pTZ19u to generate the cloned plasmid p84. The two copies of the IRS were in the same orientation, as shown in Figure 9C, with the IRS sites segregating the *B.t.* origin of replication *ori43* from the *tet* selectable marker gene and pTZ19u replicon.

The multiple cloning site in the 8.9 kb plasmid p84 was removed, as shown in Figure 9D, by digesting with *Asp*718 and *Hind*III, blunting and protruding ends with Klenow polymerase and religating to generate the cloned plasmid p85. Plasmid p85, containing the *B.t.* origin of replication *ori43,* was manipulated to replace *ori43* with a *cryI-type B.t.* protein toxin gene, specifically a *cryIC-cryIA(c)* fusion gene. The choice of the specific *B.t.* toxin gene for insertion into p85 is not critical; any insecticidal protein toxin gene could be utilized, e.g., a *B.t. cryI, cryII, cryIII* or *cryIV* toxin gene could be utilized.

Plasmid p85 was cleaved with *Sal*I and *Xba*I and the vector fragment lacking *ori43* was ligated to a *Sal*I-*Bln*I fragment containing a *cryI-type* gene as shown in Figures 9D and 9E; note that a *Bln*I cleavage site is compatible with that of *Xba*I. The resultant plasmid clone was designated plasmid p86, shown in Figure 9E.

Plasmid pEG928.9, the shuttle vector plasmid of this invention, was obtained from plasmid p86 by insertion of a *B.t.* plasmid origin of replication into p86, as shown in Figure 9E. A 2.8 kb *Sal*I fragment, containing *B.t.* plasmid origin of replication *ori*43.9, was inserted into the unique *Sal*I site of p86 to yield pEG928.9. The *ori43.9 B.t.* origin of replication gene and the *cry*I-type protein toxin gene are transcribed in opposite directions. As shown in Figure 9E, the duplicate copies of the Tn*5401* internal resolution site segregate the DNA not native to *B.t.,* i.e., the *E. coli* replicon pTZ19u and the tet selectable marker gene from the *B.t.* origin of replication and adjacent *cry*I-type protein toxin gene.

For microorganism deposit purposes, plasmid shuttle vector pEG928.9 was used to transform an acrystalliferous *B.t.* host strain, *B.t.* var. *kurstaki* strain EG10368 which is a derivative of *B.t.* var. *kurstaki* strain HD73-26 described in U.S. Patent No. 5,080,897 issued to González, Jr. et al. on January 14, 1992, to yield *B.t.* var. *kurstaki* strain EG7684.

In an analogous manner, other plasmid shuttle vectors were also constructed and two of these, plasmid shuttle vectors pEG930.9 and pEG931, are illustrated in Figures 10 and 11. These plasmid shuttle vectors differ from plasmid pEG928.9 primarily in the insecticidal protein toxin gene carried on the plasmids: plasmid pEG930.9 carries a coleopteran toxin *cryIIIB2* gene (described in U.S. Patent No. 5,187,091 issued to Donovan et al. on February 16, 1993) and plasmid pEG931 carries a lepidopteran toxin *cryIC* gene, whose gene product exhibits good activity against *Spodoptera* insect species. As is evident from the circular structural maps in Figures 10 and 11, plasmid shuttle vectors pEG930.9 and pEG931 contain a *cryI* transcription terminator located downstream of their respective *cryIIIB2* and *cryIC* genes.

Use of plasmid shuttle vectors pEG928.9, pEG930.9 and pEG931 in a site-specific recombination system for constructing insecticidal recombinant *B.t.* strains is described in Example 5.

### Example 4

### Site-Specific Recombination Catalyzed by Recombinase Protein from Tn5401

The ability of recombinase/resolvase protein from transposon Tn*5401* to catalyze, in *trans*, a site-specific recombination event in a transformed, recombinant *B.t.* strain was demonstrated in this Example 4. The recombinant plasmid used to transform the host *B.t.* strains was plasmid p83, described in Example 3 and a circular structural map of which is illustrated in Figure 9B. Plasmid p83 contains two identical copies of the Tn*5401*-derived internal resolution site, IRS, oriented in the same direction and flanking a tetracycline antibiotic resistance gene, *tet,* as shown in Figure 9B. Plasmid p83 also contains an origin of replication functional in *B.t.*, i.e., *B.t.*-derived *ori43*, and another selectable marker gene, a chloramphenicol acetyl transferase gene, *cat,* as shown in Figure 9B.

A site-specific recombination event involving plasmid p83 was demonstrated by showing that the *cat* gene encoding resistance to chloramphenicol would be maintained after a site-specific recombination event between the two IRS regions but that tetracycline resistance would be lost because of excision of the *tet* gene during such recombination. The source of recombinase protein for catalyzing the site-specific recombination was *B.t.* var. *morrisoni* strain EG2158, which harbors transposon Tn*5401* which contains the recombinase gene, *tnpI.*

Plasmid p83 was first introduced by a conventional electroporation technique into the transposon-free *B.t.* var. *kurstaki* strain EG7566, a plasmid-free derivative of *B.t.* var. *kurstaki* strain HD73-26 described in U.S. Patent No. 5,080,897 issued to González, Jr. et al. on January 14, 1992, and also into the Tn*5401*-containing *B.t.* strain EG2158. Transformed *B.t.* colonies were selected separately for tetracycline resistance (Tet^{R}) and for chloramphenicol resistance (Cm^{R}) and results are shown in the following table:

| **Host B.t. Strain** | **Cm**^{**R**} **Colonies** | **Tet**^{**R**} **Colonies** |
|---|---|---|
| EG7566 | > 1000 | > 1000 |
| EG2158 | > 1000 | 0 |

Both transformed *B.t.* strains exhibited chloramphenicol resistance, apparently due to the presence of the *cat* gene in the introduced plasmid p83. For the transposon-free *B.t.* strain EG7566 transformants, the existence of tetracycline resistance indicated that plasmid p83 was likely present as an intact plasmid, i.e,, no site-specific recombination event had occurred. Restriction enzyme analysis of recombinant plasmids isolated from representative *B.t.* strain EG7566 transformants indicated that the structural integrity of plasmid p83 had been maintained.

The Tn*5401*-containing *B.t.* strain EG2158 transformants, on the other hand, exhibited no tetracycline resistance, indicating the likely loss of the *tet* selectable marker gene from site-specific recombination between the two IRS regions in p83. Restriction enzyme analysis of recombinant plasmids recovered from representative chloramphenicol-resistant *B.t.* strain EG2158 transformants confirmed that recombination had occurred between the two IRS regions, resulting in excision of the *tet* gene from this location in plasmid p83.

### Example 5

### Construction of Recombinant B.t. Strains via Site-Specific Recombination Event Using Plasmid Shuttle Vector pEG928.9

Example 5 illustrates a method of constructing insecticidal recombinant *B.t.* strains containing no DNA foreign to *B.t.*, utilizing the plasmid shuttle vector pEG928.9 and the Tn*5401* transposon-containing recombinant plasmid pEG922 to effect a site-specific recombination event that produces the desired *B.t.* strain construct. The schematic steps of this method are shown in Figure 5, and detailed circular structural maps of plasmid pEG928.9 and plasmid 922 are shown in Figures 4 and 3, respectively, and explained in the Brief Description of the Drawings for these two Figures.

Plasmid shuttle vector pEG928.9, containing a *cryI*-type gene (a *cryIc-cryIA(c)* fusion gene), a *B.t.* origin of replication region *(ori43.9,* a high copy number mutant of *ori43*, derived from a 43-MDa *B.t.* toxin plasmid), and two identical internal resolution site (IRS) regions oriented in the same direction, was used to transform a *B.t.* host strain that served as the basis for the recombinant *B.t.* construct. As is discussed in Example 3, plasmid pEG928.9 also contains DNA not native to *B.t.* that is useful in the construction (particularly, development and characterization) of recombinant *B.t.* strains. This foreign DNA consists of an *E. coli* replicon pTZ19u and a tetracycline resistance gene, *tet,* useful as a selectable marker. The DNA not native to *B.t.* is desirably absent from the insecticidal recombinant *B.t.* construct produced by this method and for this reason is flanked by the duplicate IRS regions. The site-specific recombination event that occurs between the two IRS regions effects excision of the foreign DNA from the plasmid, and this was accomplished in this Example 5 as follows.

*B.t.* var. *kurstaki* strain EG10324 served as the host strain in this Example. B.t. strain EG10324 is a phage resistant mutant of B.t. var. *kurstaki* strain EG2348, described in U.S. Patent No. 5,080,897 issued to González, Jr. et al. on January 14, 1992. This transconjugant *B.t.* strain exhibits insecticidal activity against lepidopteran insects. The addition of a recombinant toxin plasmid via the method of this Example was intended to broaden the insecticidal spectrum of the host strain. The *cryIC*-type *B.t.* toxin gene carried by plasmid shuttle vector pEG928.9 produces a toxin protein with good activity against Spodoptera species.

*B.t.* strain EG10324 was transformed with plasmid shuttle vector pEG928.9 using conventional electroporation techniques, e.g., similar to those described in Example 6 of WO 91/18102. *B.t.* strain EG10324 transformants that were selected for tetracycline resistance were analyzed via restriction enzyme digests, and this analysis confirmed the structural integrity of plasmid pEG928.9 in these tet^{R} colonies.

These *B.t.* strain EG10324 transformants were next transformed with the Tn*5401* transposon-containing plasmid pEG922, selecting this time for chloramphenicol resistance. Plasmid pEG922, described in Example 2 and shown in Figure 3, contains the Tn*5401* transposon of this invention, tagged with a tetracycline antibiotic resistance gene, *tet.* As noted previously in description of the construction of this plasmid in Example 2, plasmid pEG922 contains a thermosensitive replicon, *rep*^{*ts*}*,* that is functional in gram-positive bacteria but that only operates at temperatures below 37°C, in contrast to most *B.t.* replicons which operate at higher temperatures. This transposon-containing plasmid also contains another selectable marker gene, *cat,* for chloramphenicol acetyl transferase resistance.

*B.t.* strain EG10324 double transformants, i.e., containing both plasmid shuttle vector pE928.9 and the Tn*5401*-containing plasmid pE922, were selected for colonies exhibiting chloramphenicol resistance. In the double recombinant derivative of *B.t.* strain EG10324, plasmid pEG928.9 underwent the site-specific recombination event between its IRS regions, and this event was catalyzed by the introduction of recombinase/resolvase protein produced by expression of the *tnpI* gene in the Tn*5401*-containing plasmid pEG922. Production of the recombinase protein was ensured by culturing the double recombinant *B.t.* strain colonies overnight at a temperature of about 30°C, at which the temperature-sensitive replicon in plasmid pEG922 operates.

The site-specific recombination event for plasmid pEG928.9 is schematically shown in Figure 5, and this resulted in the formation of plasmid pEG928.9Δ. Plasmid pEG928.9Δ is a 8.0 mDa recombinant plasmid that contains the *ori43.9* origin of replication functional in *B.t.*, the *cryIC-cryIA(c) B.t.* protein toxin fusion gene, and a single copy of the internal resolution site, derived from the site-specific recombination event.

After the site-specific recombination had been effected, removal of plasmid pEG922 from the double recombinant *B.t.* strain EG10324 transformants also containing plasmid pEG928.9Δ was accomplished by culturing these *B.t.* colonies overnight at a temperature of 37°C, a growth procedure effective to cure temperature-sensitive plasmid pEG922 from the resulting *B.t.* colonies.

The desired insecticidal recombinant *B.t.* construct, containing only a single recombinant plasmid, pEG928.9Δ, was recovered and was designated as *B.t.* strain EG7674.

*B.t.* strain EG7674 lacks the selectable marker genes utilized during its construction and is therefore chloramphenicol-and tetracycline-sensitive. *B. t.* strain EG7674 also lacks the *E. coli* replicon that was originally present in plasmid pEG928.9 but that was subsequently excised during the site-specific recombination event.

Plasmid assay studies of *B.t.* strain EG10324 and its recombinant derivatives described in this Example 10 confirmed the absence of plasmid pEG922 from *B.t.* strain EG7674. Hybridization with the *ori43.9* plasmid origin of replication in a Southern blot study of the plasmid assay gel established the presence of pEG928.9Δ as the only recombinant plasmid harbored by *B.t.* strain EG7674.

*B.t.* strain EG7674, containing no DNA not native to *B.t.*, is insecticidal to a wide spectrum of lepidopteran insects and, because of the additional *cryIC-cryIA(c)* fusion gene on its recombinant plasmid pEG928.9Δ, is designed to exhibit improved insecticidal activity against *Spodoptera exigua* (beet armyworm) and *Spodoptera littoralis* (Egyptian leaf roller), as compared with the host *B.t.* strain EG10324.

In a similar manner, five other insecticidal recombinant *B.t.* constructs were prepared via the site-specific recombination method described above. These *B.t.* constructs were similar to *B.t.* strain EG7674 in that their respective recombinant plasmids contained insecticidal *B.t.* protein toxin genes but no DNA not native to *B.t.*

The first construct was a coleopteran-toxic *B.t.* construct which used, as the host strain, transconjugant *B.t.* var. *kurstaki* strain EG2424 (described in U.S. Patent No. 5,024,837 issued to Donovan et al. on June 18, 1991) and plasmid shuttle vector pEG930.9 whose circular structural map is shown in Figure 10. Plasmid shuttle vector pEG930.9 is similar to plasmid pEG928.9 except that, in lieu of the *cryI*-type gene of pEG928.9, it contains the coleopteran toxin *cryIIIB2* gene (described in U.S. Patent No. 5,187,091 issued to Donovan et al. on February 16, 1993) and it contains a transcription terminator downstream of the *cryIIIB2* gene. The resulting recombinant *B.t.* construct contained plasmid pEG930.9Δ, whose circular structural map is also shown in Figure 10, and was designated *B.t.* strain EG7673. The presence of the *cryIIIB2* gene in this recombinant *B.t.* construct, complementing the *CryIIIA* coleopteran toxin gene present on an 88 mDa plasmid of host *B.t.* strain EG2424, is designed to provide a wider spectrum of insecticidal activity against coleopteran insects, as compared with host *B.t.* strain EG2424.

The second *B.t.* construct was a lepidopteran-toxic *B.t.* construct which used a novel *B.t.* strain, designated EG4923, as the host strain and plasmid shuttle vector pEG931 whose circular structural map is shown in Figure 11. Plasmid shuttle vector pEG931 is similar to plasmid pEG928.9 except that (i) a *cryIC* gene replaces the *cryIC-cryIA(c)* fusion gene of pEG928.9, (ii) it contains a transcription terminator downstream of the *cryIC* gene, and (iii) the *B.t.* origin of replication is *ori43* rather than the high copy number mutant *ori43.9* used in pEG928.9. The resulting recombinant *B.t.* construct contained plasmid pEG931Δ, whose circular structural map is also shown in Figure 11, and was designated *B.t.* strain EG7681. The presence of the *cryIC* gene in this recombinant *B.t.* construct, complementing the *cryIA(c)* genes of host *B.t.* strain EG4923, is designed to provide a wider spectrum of insecticidal activity against lepidopteran insects, as compared with host *B.t.* strain EG4923.

The third *B.t.* construct was a lepidopteran-toxic *B.t.* construct which used a novel *B.t.* strain, designated EG4923, as the host strain and plasmid shuttle vector pEG935 whose circular structural map is shown in Figure 12. Plasmid shuttle vector pEG935 is similar to plasmid pEG931 (shown in Figure 11) except that the *B.t.* plasmid origin of replication is *ori60* rather than *ori43* used in pEG931. The resulting recombinant *B.t.* construct contained plasmid pEG935Δ, whose circular structural map is also shown in Figure 12, and was designated *B.t.* strain EG7841. The presence of the *cryIC* gene in this recombinant *B.t.* construct, complementing the *cryIA(c)* genes of host *B.t.* strain EG4923, is designed to provide a wider spectrum of insecticidal activity against lepidopteran insects, as compared with host *B.t.* strain EG4923.

The fourth *B.t.* construct was a lepidopteran-toxic *B.t.* construct which used *B.t.* strain EG10324 as the host strain and plasmid shuttle vector pEG337 whose circular structural map is shown in Figure 13. Host *B.t.* strain EG10324 is a phage-resistant derivative of *B.t.* strain EG2348, described in U.S. Patent No. 5,080,897 issued to González, Jr. et al. on January 14, 1992. Plasmid shuttle vector pEG337 is similar to plasmid pEG931 (shown in Figure 11) except that a DNA fragment with a lepidopteran toxin *cryIF* gene (described in U.S. Patent No. 5,188,960 issued to Payne et al. on February 23, 1993 and in PCT International Patent Publication No. WO 91/16434 of Ecogen Inc. dated October 31, 1991) replaces the *cryIC* gene and accompanying transcription terminator of PEG931. The resulting recombinant *B.t.* construct contained plasmid pEG337Δ, whose circular structural map is also shown in Figure 13, and was designated *B.t.* strain EG7826. The presence of the *cryIF* gene in this recombinant *B.t.* construct, complementing the *cryIA-type* genes of host *B.t.* strain EG10324, is designed to provide a wider spectrum of insecticidal activity against lepidopteran insects, as compared with host *B.t.* strain EG10324.

The fifth *B.t.* construct was a lepidopteran-toxic *B.t.* construct which used *B.t.* strain EG7584 as the host strain and plasmid shuttle vector pEG342 whose circular structural map is shown in Figure 14. Host *B.t.* strain EG7584 is a plasmid-cured derivative of *B.t.* strain HD-263 that is crystal negative, i.e., it contains no toxin plasmids. Plasmid shuttle vector pEG342 is similar to plasmid pEG930.9 (shown in Figure 10) except that a DNA fragment with a *cryIIA* gene (described in U.S. Patent No. 5,196,342 issued to Donovan on March 23, 1993) replaces the *CryIIIB2* gene and accompanying transcription terminator of pEG930.9. The resulting recombinant *B.t.* construct contained plasmid pEG342Δ, whose circular structural map is also shown in Figure 14, and was designated *B.t.* strain EG7856. The presence of the *cryIIA* gene in this recombinant *B.t.* construct is designed to provide a high level of production of lepidopteran-toxic CryIIA protein during fermentation of this *B.t.* strain.

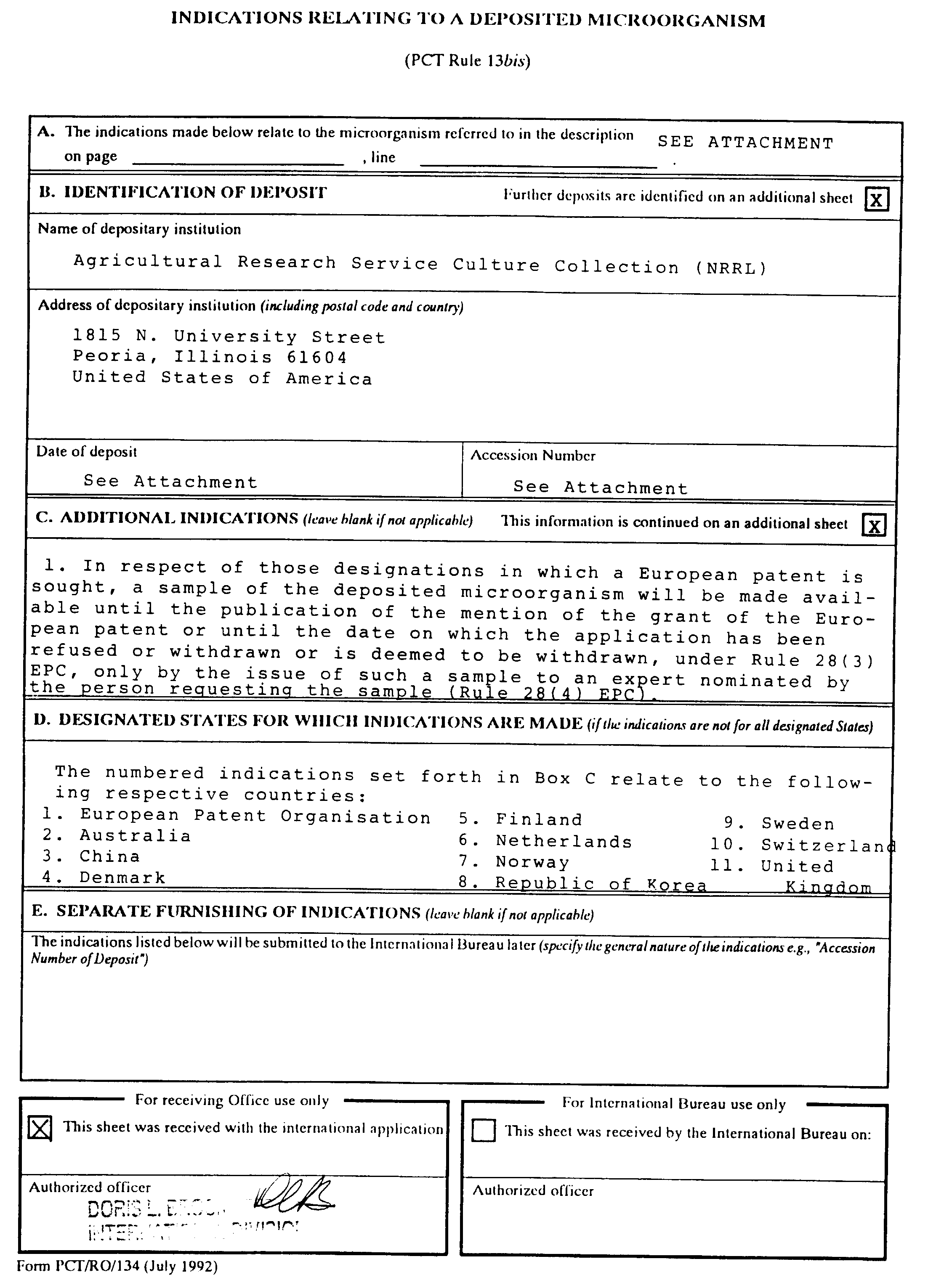
INDICATIONS RELATING TO A DEPOSITED MICROORGANISM

(PCT Rule 13bis)

A. The indications made below relate to the microorganism referred to in the description SEE ATTACHMENT
on page ______________ , line ______________ .

B. IDENTIFICATION OF DEPOSIT | Further deposits are identified on an additional sheet [X]

Name of depositary institution
Agricultural Research Service Culture Collection (NRRL)

Address of depositary institution (including postal code and country)
1815 N. University Street
Peoria, Illinois 61604
United States of America

Date of deposit | Accession Number
See Attachment | See Attachment

C. ADDITIONAL INDICATIONS (leave blank if not applicable) | This information is continued on an additional sheet [X]

1. In respect of those designations in which a European patent is
sought, a sample of the deposited microorganism will be made avail-
able until the publication of the mention of the grant of the Euro-
pean patent or until the date on which the application has been
refused or withdrawn or is deemed to be withdrawn, under Rule 28(3)
EPC, only by the issue of such a sample to an expert nominated by
the person requesting the sample (Rule 28(4) EPC).

D. DESIGNATED STATES FOR WHICH INDICATIONS ARE MADE (if the indications are not for all designated States)

The numbered indications set forth in Box C relate to the follow-
ing respective countries:
1. European Patent Organisation | 5. Finland | 9. Sweden
2. Australia | 6. Netherlands | 10. Switzerland
3. China | 7. Norway | 11. United
4. Denmark | 8. Republic of Korea | Kingdom

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[X] This sheet was received with the international application | [ ] This sheet was received by the International Bureau on:

Authorized officer | Authorized officer
DORIS L. [?][?][?][?][?]
INTER[?][?][?][?][?][?][?][?][?][?][?][?]

Form PCT/RO/134 (July 1992)

## Claims

1. A DNA sequence designated as transposon Tn5401 containing an internal resolution site and having the nucleotide base sequence shown in Figure 1, or a mutant thereof capable of functioning as a transposable element.

2. A recombinant plasmid containing the transposon Tn5401 of claim 1.

3. A bacterium transformed with the recombinant plasmid of claim 2.

4. The bacterium of claim 3, wherein the bacterium is E. coli strain EG7669 (Accession No. NRRL B-21068) or E. coli strain EG7683 (Accession No. NRRL B-21069).

5. A DNA sequence containing the internal resolution site of transposon Tn5401, extending from and including nucleotide base positions 608 to 763 shown in Figure 1.

6. A Tn5401 resolvase gene, comprising:
(a) the DNA sequence shown at nucleotide base positions 764 to 1681 in Figure 1;
(b) a DNA sequence encoding the amino acid sequence identified as SEQ ID NO:2; or
(c) a mutant of (a) or (b) whose resolvase gene product has resolving functionality.

7. A resolvase protein encoded by the Tn5401 resolvase gene of claim 6.

8. A Tn5401 transposase gene containing an internal resolution site comprising:
(a) the DNA sequence shown at nucleotide base positions 1756 to 4770 in Figure 1;
(b) a DNA sequence encoding the amino acid sequence identified as SEQ ID NO:3; or
(c) a mutant of (a) or (b) whose transposase gene product has transpositioning functionality.

9. A transposase protein encoded by the Tn5401 transposase gene of claim 8.

10. A recombinant plasmid containing the gene of claim 6 or claim 8.

11. A bacterium transformed with the recombinant plasmid of claim 10 and capable of expressing the gene on the recombinant plasmid.

12. A plasmid shuttle vector, comprising
(i) an origin of replication functional in Bacillus thuringiensis (B.t.);
(ii) DNA not native to B.t.; and
(iii) two identical internal resolution sites oriented in the same direction and flanking the DNA not native to B.t., enabling such non-B.t. DNA to be excised via a site-specific recombination event involving the two internal resolution sites.

13. The plasmid shuttle vector of claim 12 which further comprises one or more insecticidal protein toxin genes located outside of the DNA not native to B.t. and also located outside of the internal resolution sites flanking said DNA not native to B.t..

14. The plasmid shuttle vector of claim 12 or 13 wherein the internal resolution sites are derived from a Tn3-type transposon.

15. The plasmid shuttle vector of claim 14 wherein the internal resolution sites are derived from transposon Tn5401.

16. The plasmid shuttle vector of claim 15 wherein the internal resolution site of Tn5401 is contained in a DNA sequence extending from and including nucleotide base positions 608 to 763 shown in Figure 1.

17. The plasmid shuttle vector of any one of claims 12 to 16 wherein the origin of replication functional in B.t. is native to B.t.

18. The plasmid shuttle vector of any one of claims 12 to 17 wherein the origin of replication is derived from a large B.t. plasmid.

19. The plasmid shuttle vector of claim 17 or 18 wherein the origin of replication native to B.t. is ori43, ori43.9, ori44 or ori60.

20. A bacterium transformed with the plasmid shuttle vector of any one of claims 12 to 19 and capable of expressing at least one of the genes in the plasmid shuttle vector.

21. The bacterium of any one of claims 3, 11 or 20 wherein the bacterium is Bacillus thuringiensis or E. coli.

22. A method of constructing a recombinant Bacillus thuringiensis (B.t.) strain containing no DNA elements foreign to B.t. which comprises:
(a) transforming a host B.t. strain with a plasmid shuttle vector containing:
(i) an origin of replication native to B.t.;
(ii) DNA not native to B.t. and useful in the construction of recombinant B.t. strains, which is a selectable marker gene, an origin of replication functional in E. coli, or an origin of replication functional in a Bacillus host species other than B.t.
(iii) one or more insecticidal B.t. protein toxin genes; and
(iv) two identical internal resolution sites oriented in the same direction and flanking the DNA not native to B.t., enabling such non-B.t. DNA to be excised via a site-specific recombination event involving the two internal resolution sites;
(b) introducing into the transformed B.t. strain a resolvase protein to effect a site-specific recombination event involving the internal resolution sites, thereby excising from the plasmid shuttle vector the DNA not native to B.t.; and
(c) recovering a recombinant B.t. strain containing a recombinant plasmid capable of replicating in the B.t. strain and containing
(i) an origin of replication native to B.t.;
(ii) one or more insecticidal B.t. protein toxin genes; and
(iii) a single internal resolution site, derived from the site-specific recombination event.

23. The method of claim 22 wherein the internal resolution sites in the plasmid shuttle vector are derived from transposon Tn4430 or Tn5401.

24. The method of claim 22 or 23 wherein the resolvase protein is directly introduced into the host B.t. strain.

25. The method of claim 22 or 23 wherein the resolvase protein is produced by expression of a corresponding resolvase gene carried by a recombinant plasmid, the plasmid having been introduced into the host B.t. strain.

26. The method of claim 25 wherein the recombinant plasmid carrying the resolvase gene is a plasmid with a thermosensitive replicon, to facilitate recovery of a recombinant B.t. strain free of the resolvase-containing recombinant plasmid.

27. The method of claim 26, wherein the thermosensitive plasmid is removed from the host B.t. strain following the site-specific recombination event effected by the resolvase protein.

28. The method of any one of claims 22 to 27 wherein the resolvase protein is the resolvase protein of claim 7.

29. A recombinant plasmid capable of replicating in a Bacillus thuringiensis (B.t.) bacterium, the plasmid comprising:
(i) at least one insecticidal protein toxin gene;
(ii) an origin of replication functional in B.t.; and
(iii) a single internal resolution site.

30. The recombinant plasmid of claim 29, wherein the internal resolution site is derived from a Tn3-type transposon.

31. The recombinant plasmid of claim 30, wherein the transposon is native to B.t.

32. The recombinant plasmid of any one of claims 29 to 31 wherein the internal resolution site is derived from transposon Tn4430 or Tn5401.

33. The recombinant plasmid of claim 32 wherein the internal resolution site of transposon Tn5401 extends from and includes nucleotide base positions 608 to 763 shown in Figure 1.

34. The recombinant plasmid of any one of claims 29 to 33 wherein the origin of replication functional in B.t. is derived from a large B.t. plasmid.

35. The recombinant plasmid of any one of claims 29 to 34 wherein the origin of replication functional in B.t. is ori43, ori43.9, ori44 or ori60.

36. A bacterium containing the recombinant plasmid of any one of claims 29 to 35 and capable of expressing the insecticidal protein toxin gene or genes in the recombinant plasmid.

37. The bacterium of claim 36 wherein the bacterium is Bacillus thuringiensis.

38. A Bacillus thuringiensis bacterium designated as B.t. strain EG7673 (Accession No. NRRL B-21070), B.t. strain EG7674 (Accession No. NRRL B-21071), B.t. strain EG7681 (Accession No. NRRL B-21072), B.t. strain EG7841 (Accession No. NRRL B-21250), B.t. strain EG7826 (Accession No. NRRL B-21249), B.t. strain EG7856 (Accession No. NRRL B-21251), or B.t. strain EG7684 (Accession No. NRRL B-21121).

39. An insecticidal composition comprising the Bacillus thuringiensis bacterium of claim 37 or 38 and an agriculturally acceptable adjuvant.

40. A method of controlling insect pests comprising applying to a host plant for such insect pests the insecticidal composition of claim 39.

## Patentansprüche

1. DNA-Sequenz mit der Bezeichnung Transposon Tn5401, die eine interne Auflösungsstelle enthält und die in Figur 1 gezeigte Nucleotidbasensequenz besitzt, oder eine Mutante davon, die als transposables Element funktionieren kann.

2. Rekombinantes Plasmid, das das Transposon Tn5401 nach Anspruch 1 enthält.

3. Bakterium, das mit dem rekombinanten Plasmid nach Anspruch 2 transformiert ist.

4. Bakterium nach Anspruch 3, wobei das Bakterium E. coli-Stamm EG7669 (Hinterlegungsnummer NRRL B-21068) oder E. coli-Stamm EG7683 (Hinterlegungsnummer NRRL B-21069) ist.

5. DNA-Sequenz, die die interne Auflösungsstelle des Transposons Tn5401 enthält, die sich über die in Figur 1 gezeigten Nucleotidbasenpositionen 608 bis 763 erstreckt und diese einschließt.

6. Tn5401-Resolvasegen, umfassend:
(a) die in Figur 1 an Nucleotidbasenpositionen 764 bis 1681 gezeigte DNA-Sequenz;
(b) eine DNA-Sequenz, die die als SEQ ID No:2 bezeichnete Aminosäuresequenz codiert; oder
(c) eine Mutante von (a) oder (b), deren Resolvase-Genprodukt auflösende Aktivität besitzt.

7. Resolvase-Protein, das durch das Tn5401-Resolvasegen nach Anspruch 6 codiert ist.

8. Tn5401-Transposasegen, das eine interne Auflösungsstelle enthält, umfassend:
(a) die in Figur 1 an Nucleotidbasenpositionen 1756 bis 4770 gezeigte DNA-Sequenz;
(b) eine DNA-Sequenz, die die als SEQ ID No:3 bezeichnete Aminosäuresequenz codiert; oder
(c) eine Mutante von (a) oder (b), deren Transposase-Genprodukt verschiebende Aktivität besitzt.

9. Transposase-Protein, codiert durch das Tn5401-Transposasegen nach Anspruch 8.

10. Rekombinantes Plasmid, das das Gen nach Anspruch 6 oder 8 enthält.

11. Bakterium, das mit dem rekombinanten Plasmid nach Anspruch 10 transformiert ist und das das Gen des rekombinanten Plasmids exprimieren kann.

12. Plasmid-Shuttlevektor, umfassend:
(i) einen in Bacillus thuringiensis (B.t.) funktionsfähigen Replikationsursprung;
(ii) nicht aus B.t. stammende DNA; und
(iii) zwei identische interne Auflösungsstellen, die in der gleichen Richtung orientiert sind und die die nicht aus B.t. stammende DNA flankieren, so daß es möglich ist, daß die nicht-B.t.-DNA über ein ortsspezifisches, die beiden internen Auflösungsstellen einbeziehendes Rekombinationsereignis ausgeschnitten werden kann.

13. Plasmid-Shuttlevektor nach Anspruch 12, der außerdem ein oder mehrere insektizid wirkende Proteintoxingene umfaßt, die außerhalb der nicht aus B.t. stammenden DNA und auch außerhalb der internen Aufläsungssteilen, die die nicht aus B.t. stammende DNA flankieren, liegen.

14. Plasmid-Shuttlevektor nach Anspruch 12 oder 13, wobei die internen Auflösungsstellen von einem Tn3-Typ-Transposon stammen.

15. Plasmid-Shuttlevektor nach Anspruch 14, wobei die internen Auflösungsstellen von Transposon Tn5401 stammen.

16. Plasmid-Shuttlevektor nach Anspruch 15, wobei die interne Auflösungsstelle von Tn5401 in der DNA-Sequenz enthalten ist, die sich über die in Figur 1 gezeigten Nucleotidbasenpositionen 608 bis 763 erstreckt und diese einschließt.

17. Plasmid-Shuttlevektor nach einem der Ansprüche 12 bis 16, wobei der in B.t. funktionsfähige Replikationsursprung aus B.t. stammt.

18. Plasmid-Shuttlevektor nach einem der Ansprüche 12 bis 17, wobei der Replikationsursprung aus einem großen B.t.-Plasmid stammt.

19. Plasmid-Shuttlevektor nach Anspruch 17 oder 18, wobei der aus B.t. stammende Replikationsursprung ori43, ori43.9, ori44 oder ori60 ist.

20. Bakterium, das mit dem Plasmid-Shuttlevektor nach einem der Ansprüche 12 bis 19 transformiert ist und das mindestens eines der Gene des Plasmid-Shuttlevektors exprimieren kann.

21. Bakterium nach einem der Ansprüche 3, 11 oder 20, wobei das Bakterium Bacillus thuringiensis oder E. coli ist.

22. Verfahren zur Herstellung eines rekombinanten, keine für B.t. fremde DNA-Elemente enthaltenden Bacillus thuringiensis (B.t.)-Stamms, das umfaßt:
(a) Transformation eines Wirts-B.t.-Stamms mit einem Plasmid-Shuttlevektor enthaltend:
(i) einen aus B.t. stammenden Replikationsursprung;
(ii) DNA, die nicht aus B.t. stammt und verwendbar in der Herstellung von rekombinanten B.t.-Stämmen ist, welche ein selektionierbares Markergen, ein in E. coli funktionsfähiger Replikationsursprung oder ein in einer anderen als B.t. Bacillus-Wirtsspezies funktionsfähiger Replikationsursprung ist;
(iii) ein oder mehrere insektizid wirkende B.t.-Proteintoxingene; und
(iv) zwei identische interne Auflösungsstellen, die in die gleiche Richtung orientiert sind und die nicht aus B.t. stammende DNA flankieren, so daß es möglich ist, daß die nicht-B.t.-DNA über ein die beiden internen Auflösungsstellen einbeziehendes, ortsspezifisches Rekombinationsereignis ausgeschnitten werden kann;
(b) Einführung eines Resolvase-Proteins in den transformierten B.t.-Stamm, um ein die internen Auflösungsstellen einbeziehendes ortsspezifisches Rekombinationsereignis zu bewirken, wobei die nicht aus B.t. stammende DNA aus dem Plasmid-Shuttlevektor ausgeschnitten wird; und
(c) Gewinnung eines rekombinanten B.t.-Stamms, enthaltend ein rekombinantes Plasmid, das in dem B.t.-Stamm replizieren kann und enthält:
(i) einen aus B.t. stammenden Replikationsursprung;
(ii) ein oder mehrere insektizid wirkende B.t.-Proteintoxingene; und
(iii) eine einzelne interne Auflösungsstelle, die aus dem ortsspezifischen Rekombinationsereignis stammt.

23. Verfahren nach Anspruch 22, wobei die internen Auflösungsstellen des Plasmid-Shuttlevektors von Transposon Tn4430 oder Tn5401 stammen.

24. Verfahren nach Anspruch 22 oder 23, wobei das Resolvase-Protein direkt in den Wirts-B.t.-Stamm eingeführt wird.

25. Verfahren nach Anspruch 22 oder 23, wobei das Resolvase-Protein durch die Expression eines auf einem rekombinanten Plasmid liegenden entsprechenden Resolvasegens produziert wird, wobei das Plasmid in den Wirts-B.t.-Stamm eingeführt worden ist.

26. Verfahren nach Anspruch 25, wobei das das Resolvasegen tragende rekombinante Plasmid ein Plasmid mit einem temperatursensitiven Replicon ist, um die Gewinnung eines rekombinanten B.t.-Stamms zu erleichtern, der frei von dem die Resolvase enthaltenen rekombinanten Plasmid ist.

27. Verfahren nach Anspruch 26, wobei das temperatursensitive Plasmid nach dem durch das Resolvase-Protein bewirkten ortsspezifischen Rekombinationsereignis aus dem Wirts-B.t.-Stamm entfernt wird.

28. Verfahren nach einem der Ansprüche 22 bis 27, wobei das Resolvase-Protein das Resolvase-Protein nach Anspruch 7 ist.

29. Rekombinantes Plasmid, das im Bacillus thuringiensis (B.t.)-Bakterium replizieren kann, wobei das Plasmid umfaßt:
(i) mindestens ein insektizid wirkendes Proteintoxingen;
(ii) einen in B.t. funktionsfähigen Replikationsursprung; und
(iii) eine einzelne interne Auflösungsstelle.

30. Rekombinantes Plasmid nach Anspruch 29, wobei die interne Auflösungsstelle von einem Tn3-Typ-Transposon stammt.

31. Rekombinantes Plasmid nach Anspruch 30, wobei das Transposon aus B.t. stammt.

32. Rekombinantes Plasmid nach einem der Ansprüche 29 bis 31, wobei die interne Auflösungsstelle aus Transposon Tn4430 oder Tn 5401 stammt.

33. Rekombinantes Plasmid nach Anspruch 32, wobei die interne Auflösungsstelle von Transposon Tn 5401 sich über die in Figur 1 gezeigten Nucleotidbasenpositionen 608 bis 763 erstreckt und diese einschließt.

34. Rekombinantes Plasmid nach einem der Ansprüche 29 bis 33, wobei der in B.t. funktionsfähige Replikationsursprung von einem großen B.t.-Plasmid stammt.

35. Rekombinantes Plasmid nach einem der Ansprüche 29 bis 34, wobei der in B.t. funktionsfähige Replikationsursprung ori43, ori43.9, ori44 oder ori60 ist.

36. Bakterium, das das rekombinante Plasmid nach einem der Ansprüche 29 bis 35 enthält und das (die) insektizid wirkende(n) Proteintoxingen oder -gene des rekombinanten Plasmids exprimieren kann.

37. Bakterium nach Anspruch 36, wobei das Bakterium Bacillus thuringiensis ist.

38. Bacillus thuringiensis-Bakterium, mit der Bezeichnung B.t.-Stamm EG7673 (Hinterlegungsnummer NRRL B-21070), B.t.-Stamm EG7674 (Hinterlegungsnummer NRRL B-21071), B.t.-Stamm EG7681 (Hinterlegungsnummer NRRL B-21072), B.t.-Stamm EG7841 (Hinterlegungsnummer NRRL B-21250), B.t.-Stamm EG7826 (Hinterlegungsnummer NRRL B-21249), B.t.-Stamm EG7856 (Hinterlegungsnummer NRRL B-21251) oder B.t.-Stamm EG7684 (Hinterlegungsnummer NRRL B-21121).

39. Insektizid wirkendes Mittel, umfassend das Bacillus thuringiensis-Bakterium nach Anspruch 37 oder 38 und ein landwirtschaftlich verträgliches Adjuvans.

40. Verfahren zur Kontrolle von Insektenschädlingen, umfassend das Aufbringen des insektizid wirkenden Mittels nach Anspruch 39 auf eine Wirtspflanze für solche Insektenschädlinge.

## Revendications

1. Séquence d'ADN identifiée en tant que « transposon Tn5401 » contenant un site de résolution interne et ayant la séquence de bases nucléotidiques représentée sur la figure 1, ou un mutant de celui-ci capable de fonctionner comme élément transposable.

2. Plasmide de recombinaison contenant le transposon Tn5401 selon la revendication 1.

3. Bactérie transformée par le plasmide de recombinaison selon la revendication 2.

4. Bactérie selon la revendication 3, cette bactérie appartenant à la souche d'E. coli EG7669 (No d'accès NRRL B-21068) ou la souche d'E. coli EG7683 (No d'accès NRRL B-21069).

5. Séquence d'ADN contenant un site de résolution interne du transposon Tn5401, s'étendant de la base nucléotidique en position 608 à celle en 763, comme représenté sur la figure 1, en incluant ces bases.

6. Gene Tn5401 d'une résolvase comprenant :
(a) la séquence d'ADN s'étendant de la base nucléotidique en position 764 à celle en position 1681 sur la figure 1;
(b) une séquence d'ADN codant pour une séquence d'acides aminés identifiée en tant que SEQ ID NO: 2; ou
(c) un mutant de (a) ou (b) dans lequel le produit du gène de la résolvase a une activité de résolution.

7. Protéine résolvase codée par le gène Tn5401 de la résolvase selon la revendication 6.

8. Gène Tn5401 d'une transposase contenant un site de résolution interne comprenant :
(a) la séquence d'ADN allant de la base nucléotidique en position 1756 à celle en position 4770 sur la figure 1;
(b) une séquence d'ADN codant pour la séquence d'acides aminés identifiée en tant que SEQ ID NO:3; ou
(c) un mutant de (a) ou de (b) dont le produit du gène de la transposase a une activité de transposition.

9. Protéine transposase codée par le gène Tn5401 de la transposase selon la revendication 8.

10. Plasmide de recombinaison contenant un gène selon la revendication 6 ou la revendication 8.

11. Bactérie transformée par le plasmide de recombinaison selon la revendication 10, capable d'exprimer le gène dans le plasmide de recombinaison

12. Vecteur navette plasmidique comprenant :
(i) une origine d'une réplication fonctionnelle dans Bacillus thuringiensis (B.t.);
(ii) un ADN n'appartenant pas à (B. t.); et
(iii) deux sites de résolution interne identiques orientés dans la même direction et bordant l'ADN n'appartenant pas à B. t., ce qui permet une excision de l'ADN n'appartenant par à B. t. par une opération de recombinaison dirigée sur un site impliquant les deux sites internes de résolution.

13. Vecteur navette plasmidique selon la revendication 12, qui comprend, en outre, un ou plusieurs gènes d'une toxine protéique insecticide situés à l'extérieur de l'ADN n'appartenant pas à B. t. et situés également à l'extérieur des sites de résolution internes bordant ledit ADN n'appartenant pas à B. t.

14. Vecteur navette plasmidique selon la revendication 12 ou la revendication 13, dans lequel les sites de résolution internes sont dérivés du transposon de type Tn3.

15. Vecteur navette plasmidique selon la revendication 14, dans lequel les sites internes de résolution sont dérivés du transposon Tn5401.

16. Vecteur navette plasmidique selon la revendication 15, dans lequel le site interne de résolution de Tn5401 est contenu dans la séquence d'ADN s'étendant de la base nucléotidique en position 608 à celle en position 763, comme représenté sur la figure 1, et incluant ces bases.

17. Vecteur navette plasmidique selon l'une quelconque des revendications 12 à 16, dans lequel l'origine d'une réplication fonctionnelle dans B. t. appartient à B. t.

18. Vecteur navette plasmidique selon l'une quelconque des revendication 12 à 17, dans lequel l'origine d'une réplication est dérivée d'un plasmide de B. t. de grande taille.

19. Vecteur navette plasmidique selon la revendication 17 ou 18, dans lequel l'origine d'une réplication appartenant à B. t. est ori43, ori43,9, ori44 ou ori60.

20. Bactérie transformée par le vecteur navette plasmidique selon l'une quelconque des revendications 12 à 19, qui est capable d'exprimer au moins un des gènes dans le vecteur navette plasmidique.

21. Bactérie selon l'une quelconque des revendications 3, 11 ou 20, cette bactérie étant Bacillus thuringiensis ou E. coli.

22. Procédé pour obtenir une souche de Bacillus thuringiensis (B. t.) de recombinaison ne contenant aucun élément d'ADN étranger à B. t., qui comprend :
(a) de transformer une souche de B. t. hôte avec un vecteur navette plasmidique contenant :
(i) une origine d'une réplication appartenant à B. t.;
(ii) un ADN n'appartenant pas à B. t., utile pour l'obtention de souches de B. t. de recombinaison, qui peut être un gène marquer sélectionné, une origine d'une réplication fonctionnelle dans E. coli, ou une origine d'une réplication fonctionnelle dans un Bacillus hôte d'une espèce autre que B. t.
(iii) un ou plusieurs gènes d'une toxine protéique insecticide de B. t.; et
(iv) deux sites de résolution internes identiques orientés dans la même direction et bordant l'ADN n'appartenant pas à B. t., ce qui permet à un tel ADN m'appartenant pas à B. t. d'être excisé par l'intermédiaire d'un opération de recombinaison dirigée sur un site impliquant les deux sites de résolution internes;
(b) d'introduire dans la souche transformée de B. t. une protéine résolvase pour effectuer une opération de recombinaison dirigée sur un site impliquant les sites de résolution internes, ce qui permet d'exciser du vecteur navette plasmidique l'ADN n'appartenant pas à B. t.; et
(c) récupérer une souche B. t. de recombinaison contenant un plasmide de recombinaison capable de se répliquer dans la souche de B. t. et contenant :
(i) une origine d'une réplication appartenant à B. t.;
(ii) un ou plusieurs gènes de toxine protéique insecticide de B. t.; et
(iii) un seul site de résolution interne, provenant de l'opération de recombinaison dirigée sur un site.

23. Procédé selon la revendication 22, dans lequel les sites de résolution internes dans le vecteur navette plasmidique sont dérivés du transposon Tn4430 ou du transposon Tn5401.

24. Procédé selon la revendication 22 ou la revendication 23, dans lequel la protéine résolvase est introduite directement dans la souche hôte de B. t.

25. Procédé selon la revendication 22 ou la revendication 23, dans lequel la protéine résolvase est produite par l'expression du gène correspondant de la résolvase porté par un plasmide de recombinaison, le plasmide ayant été introduit aans la souche hôte de B. t.

26. Procédé selon la revendication 25, dans lequel le plasmide de recombinaison portant le gène de la résolvase est un plasmide avec un réplicon thermosensible, pour faciliter la récupération d'une souche de B. t. de recombinaison exempte de plasmide de recombinaison contenant la résolvase.

27. Procédé selon la revendication 26, dans lequel le plasmide thermosensible est éliminé de la souche hôte de B. t., après l'opération de recombinaison dirigée sur un site assurée par la protéine résolvase.

28. Procédé selon l'une quelconque des revendications 22 à 27, dans lequel la protéine résolvase est la protéine résolvase de la revendication 7.

29. Plasmide de recombinaison capable d'une réplication dans la bactérie Bacillus thuringiensis (B. t.), le plasmide comprenant :
(i) au moins un gène pour une toxine protéique insecticide;
(ii) une origine d'une réplication fonctionnelle dans B. t.; et
(iii) un seul site de résolution interne.

30. Plasmide de recombinaison selon la revendication 29, dans lequel le site de résolution interne est dérivé d'un transposon de type Tn3.

31. Plasmide de recombinaison selon la revendication 30, dans lequel le transposon appartient à B. t.

32. Plasmide de recombinaison selon l'une quelconque des revendications 29 à 31, dans lequel le site de résolution interne est dérivé du transposon Tn4430 ou du transposon Tn5401.

33. Plasmide de recombinaison selon la revendication 32, dans lequel le site de résolution interne du transposon Tn5401 s'étend de la base nucléotidique en position 608 à celle en position 763, comme représenté sur la figure 1, en incluant ces bases.

34. Plasmide de recombinaison selon l'une quelconque des revendications 29 à 33, dans lequel l'origine d'une réplication fonctionnelle dans B. t. est dérivée d'un plasmide de taille importante de B. t.

35. Plasmide de recombinaison selon l'une quelconque des revendications 29 à 34, dans lequel l'origine d'une réplication fonctionnelle dans B. t. est ori43, ori43,9, ori44 ou ori60.

36. Bactérie contenant le plasmide de recombinaison selon l'une quelconque des revendications 29 à 35, capable d'exprimer le ou les gènes de la toxine protéique insecticide dans le plasmide de recombinaison.

37. Bactérie selon la revendication 36, dans laquelle la bactérie est Bacillus thuringiensis.

38. Bactérie Bacillus thuringiensis, appelées souche B. t. EG7673 (No d'accès NRRL B-21070), souche B. t. EG7674 (No d'accès NRRL B-21071), souche B. t. EG7681 (No d'accès NRRL B-21072), souche B. t. EG7841 (No d'accès NRRL B-21250), souche B. t. EG7826, (No d'accès NRRL B-21249), souche B. t. EG7856 (No d'accès NRRL B-21251) ou souche B. t. EG7684 (No d'accès NRRL B-21121).

39. Composition insecticide comprenant la bactérie Bacillus thuringiensis selon la revendication 37 ou la revendication 38 et un adjuvant acceptable pour l'agriculture.

40. Procédé pour combattre les insectes parasites comprenant une opération consistant à appliquer sur une plante parasitée par de tels insectes la composition insecticide de la revendication 39.


SEQUENCE LISTING

(1) GENERAL INFORMATION:

(i) APPLICANT: Baum, James A.

(ii) TITLE OF INVENTION: Bacillus thuringiensis Transposon Tn5401
and Its Use in a Site-Specific Recombination System For
Recombinant Bacillus thuringiensis Strain Development

(iii) NUMBER OF SEQUENCES: 4

(iv) CORRESPONDENCE ADDRESS:
(A) ADDRESSEE: Panitch Schwarze Jacobs & Nadel c/o A.S. Nadel
(B) STREET: 1601 Market Street, 36th Floor
(C) CITY: Philadelphia
(D) STATE: Pennsylvania
(E) COUNTRY: U.S.A.
(F) ZIP: 19103

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: US 08/089,986
(B) FILING DATE: 08-JUL-1993

(2) INFORMATION FOR SEQ ID NO:1:

(i) SEQUENCE CHARACTERISTICS:
(A) LENGTH: 4837 base pairs
(B) TYPE: nucleic acid
(C) STRANDEDNESS: double
(D) TOPOLOGY: circular

(ii) MOLECULE TYPE: DNA (genomic)

(ix) FEATURE:
(A) NAME/KEY: transposon
(B) LOCATION: 1..4837

(ix) FEATURE:
(A) NAME/KEY: CDS
(B) LOCATION: 764..1684

(ix) FEATURE:
(A) NAME/KEY: CDS
(B) LOCATION: 1756..4773

(ix) FEATURE:
(A) NAME/KEY: CDS
(B) LOCATION: complement (351..608)

(xi) SEQUENCE DESCRIPTION: SEQ ID NO:1:

GGGGTATGTG TAGCAATGGA ACAGAATCAC GCAACAAGCA TTAGCGGACA TTATTCGCAC 60

ACAAAAAAGG AAGGTTCTTC GATTCAGAAG ACCTTTCTTT TAAAAATGCA TGTTTGCCTT 120

ATTTATAGAT GTCACCACGA TTTCCAATTG CTTGTATGTA TATGACTTTC TCATCATGAT 180

TTATTTCAAA TAAAATTCGA AAGGTTCCAA TCCGTAATCG ATATAGTTCT GTGTAACCTT 240

TCATACTTTT AATATCTCCT TCAGGAGGAA TCTTAAGAAG TCCCTTCAAT CCTTCTGCAA 300

TTCTTTTTTG AATCCCTTTT TCTTGCTTTG CAATAAATTT CACCGCGGAC TTATGGTAAA 360

TCAATTTGTA GTCCGAATTC ACGTTTTGCG TCCTCCCCTG ATACATATCC TTCTTCACTG 420

TTTAACTGTT CTAACTCTTG TGTAGACAGC GGTTCATGAT CAGGATCTGC CATATCAATT 480

TTTTCCCATT CTTTAGGTTT TCTTCTTGAC CGTTGAACAA GAAATTCTAA AAAGTCAAAT 540

GCTGCTTTTT CATCTTGTTG ATCCAGGTGA TCAATTAACC GATACAATTC ATCTTTACGA 600

ATAGCCATGT GTTACACCTA CTTTCGAGAT AGTTTTAAAT GTCCACTAAT TAATATTAGT 660

GGACATGAAG TGTGGGAAAA TAAATGTTTG ATGTCCGCTA ACATAATTGA TAAGATTAAA 720

ATATCATGTC CGCTAATGTA AGTCAATAAA AGAGGAGGTA TTT ATG CAT TCC ACT 775
                                            Met His Ser Thr
                                              1

AAA ACA ATT TCT ATA CAA GCA ACA TCT TTG ATT TCC GAT TTT ATT TCT 823
Lys Thr Ile Ser Ile Gln Ala Thr Ser Leu Ile Ser Asp Phe Ile Ser
  5                  10                  15                  20

AGC TTA TCT CAA GAA GGA GAT TTG CAT ACA AAA ACA CTA AAA GAA TAT 871
Ser Leu Ser Gln Glu Gly Asp Leu His Thr Lys Thr Leu Lys Glu Tyr
                 25                  30                  35

ACG AGT GAT TTA AAA GAT TTT GTA TTT TGG TTT GAA AAT GTG TGG GGA 919
Thr Ser Asp Leu Lys Asp Phe Val Phe Trp Phe Glu Asn Val Trp Gly
             40                  45                  50

AAA CAT GCT GAG GAT ACT CTT TTT CAT CCA ATA GAA GTT ACC GCT CGC 967
Lys His Ala Glu Asp Thr Leu Phe His Pro Ile Glu Val Thr Ala Arg
         55                  60                  65

ACT ATT GCT CGA TAT CGA GGG CAT ATG CAA GTT ACA AGA TTA CTA AAA 1015
Thr Ile Ala Arg Tyr Arg Gly His Met Gln Val Thr Arg Leu Leu Lys
     70                  75                  80

CCT TCT ACG ATT AAC CGG CGC ATT AAT TCA ATC AAA CGT TAT TTT GAC 1063
Pro Ser Thr Ile Asn Arg Arg Ile Asn Ser Ile Lys Arg Tyr Phe Asp
 85                  90                  95                 100

TGG GCT AAG CAA AAA GGA CTG GTA CAA ACA AAT TAT TCA AAA TCA ATT 1111
Trp Ala Lys Gln Lys Gly Leu Val Gln Thr Asn Tyr Ser Lys Ser Ile
                105                     110                 115

AAG TTT GTA CCA ACA GAA AAA ACG AGT CCC AAA CGC ATG TCA GAT AAA 1159
Lys Phe Val Pro Thr Glu Lys Thr Ser Pro Lys Arg Met Ser Asp Lys
            120                     125                 130

GAA GAA GCC GCT TTA ATG CAT GCC GTT GAA AAA TAC GGC ACA CTA CGT 1207
Glu Glu Ala Ala Leu Met His Ala Val Glu Lys Tyr Gly Thr Leu Arg
        135                     140                 145

GAC AGG GCA ATG ATT ATT TTT ATG CTT CAT ACT GGC CTT CGT TCA ATG 1255
Asp Arg Ala Met Ile Ile Phe Met Leu His Thr Gly Leu Arg Ser Met
    150                     155                 160

GAA GTG TGT GAT GTT CAA ATA GAG GAT GTT ATC ATG AGA AAA AGA GGC 1303
Glu Val Cys Asp Val Gln Ile Glu Asp Val Ile Met Arg Lys Arg Gly
165                 170                     175                 180

GGC TAT GTT GTT GTT CGA TCT GGA AAA CGA AAT AAA CAG AGG GAA GTG 1351
Gly Tyr Val Val Val Arg Ser Gly Lys Arg Asn Lys Gln Arg Glu Val
                185                     190                 195

CCT TTG AAT AGT ACA GCT CGT TGT GCA CTA GAA GAA CAT ATC AGA TTA 1399
Pro Leu Asn Ser Thr Ala Arg Cys Ala Leu Glu Glu His Ile Arg Leu
            200                     205                 210

AGT GAG ATT TCA CAG AGT TAT TTG TTT CCT TCT TCT AAA ACA GGA AAA 1447
Ser Glu Ile Ser Gln Ser Tyr Leu Phe Pro Ser Ser Lys Thr Gly Lys
        215                     220                 225

CGC CTA CAA GAA AGA GCG ATC CGC CAT ATT CTT CAG AAG TAT ATT AGA 1495
Arg Leu Gln Glu Arg Ala Ile Arg His Ile Leu Gln Lys Tyr Ile Arg
    230                     235                 240

CTT GCA AAG TTA GAA GGA TTT AGT GCC CAT GAT TTA AGG CAT CGC TTT 1543
Leu Ala Lys Leu Glu Gly Phe Ser Ala His Asp Leu Arg His Arg Phe
245                 250                     255                 260

GGT TAT GTG ATG GCT GAA CGC ACA CCA TTA CAT CGT CTT GCA CAA ATT 1591
Gly Tyr Val Met Ala Glu Arg Thr Pro Leu His Arg Leu Ala Gln Ile
                265                 270                 275

ATG GGA CAC GAT AAC TTG AAT ACC ACG ATG ATT TAT GTA AGA GCT ACA 1639
Met Gly His Asp Asn Leu Asn Thr Thr Met Ile Tyr Val Arg Ala Thr
            280                 285                 290

CAA GAA GAT TTA CAG GGA GAA GTA GAA AAG ATT GCC TGG AAC TAAAGAATGC 1691
Gln Glu Asp Leu Gln Gly Glu Val Glu Lys Ile Ala Trp Asn
        295                 300                 305

ACATTATCCT ACTCATTTGG TCATGTGATA CAAAATAAGA ATTGTAACAG GAGGAACAAG 1751

GGTT ATG CCT GTA GAT TTT TTA ACA CCT GAA CAA GAA GAA AAA TAT GGT 1800
     Met Pro Val Asp Phe Leu Thr Pro Glu Gln Glu Glu Lys Tyr Gly
       1               5                  10                  15

TGT TTT TGT GAC ACT CCA ACA TCA GAG CAG TTA GCA AAA TAT TTT TGG 1848
Cys Phe Cys Asp Thr Pro Thr Ser Glu Gln Leu Ala Lys Tyr Phe Trp
                 20                  25                  30

TTA GAT GAT ACA GAC AAA GAA CTG ATA TGG AAT CGT CGT GGA GAG CAT 1896
Leu Asp Asp Thr Asp Lys Glu Leu Ile Trp Asn Arg Arg Gly Glu His
             35                  40                  45

AAT CAA CTT GGT TTC GCT GTT CAA TTA GGA ACC GTT AGG TTC TTA GGA 1944
Asn Gln Leu Gly Phe Ala Val Gln Leu Gly Thr Val Arg Phe Leu Gly
         50                  55                  60

ACA TTT TTA TCT GAT CCT ACA AAT GTA CCA CAA TCG GTT ATT ACA TAT 1992
Thr Phe Leu Ser Asp Pro Thr Asn Val Pro Gln Ser Val Ile Thr Tyr
     65                  70                  75

ATG GCA AAT CAA CTT CAT CTA GAT GCT CAA AGC TTT TCT CGT TAT CGA 2040
Met Ala Asn Gln Leu His Leu Asp Ala Gln Ser Phe Ser Arg Tyr Arg
 80                  85                  90                  95

AAT AAA CGA AGT CAG TGG GAT CAA ATG CAA GAG ATA CGT TCT GTT TAT 2088
Asn Lys Arg Ser Gln Trp Asp Gln Met Gln Glu Ile Arg Ser Val Tyr
                100                 105                 110

GGA TAT AAA AAC TTT ACA GAT AAA TCA ACA CAT TGG CGA TTC ATC AGA 2136
Gly Tyr Lys Asn Phe Thr Asp Lys Ser Thr His Trp Arg Phe Ile Arg
            115                 120                 125

TGG CTA TAT GCA CGT GCT TGG CTA TAT AAT GAA CGG CCA AGT GTC TTA 2184
Trp Leu Tyr Ala Arg Ala Trp Leu Tyr Asn Glu Arg Pro Ser Val Leu
        130                 135                 140

TTT GAT TTA GCA ACA GCA CGA TGT ATC GAA CAA AAA ATT TTA CTA CCT 2232
Phe Asp Leu Ala Thr Ala Arg Cys Ile Glu Gln Lys Ile Leu Leu Pro
    145                 150                 155

GGT GTA TCT GTA TTA ACA AGG CTA GTA TCA ACG GTT CGT GAT CGT TCA 2280
Gly Val Ser Val Leu Thr Arg Leu Val Ser Thr Val Arg Asp Arg Ser
160                 165                 170                 175

GCA GAA AAT ATA TGG AAA AAG CTC TCT AGT CTT CCG GAT AAT GTT CAG 2328
Ala Glu Asn Ile Trp Lys Lys Leu Ser Ser Leu Pro Asp Asn Val Gln
                180                 185                 190

AAA AAA CAA TTA GAA AAC CTT CTT CAG ATA GAT CAA AAA ACA AAG AAA 2376
Lys Lys Gln Leu Glu Asn Leu Leu Gln Ile Asp Gln Lys Thr Lys Lys
            195                 200                 205

ACG TAT TTA GAG CGT CTA AGT AAT CCC CCT GTT CCG ATT AGT GTT ACG 2424
Thr Tyr Leu Glu Arg Leu Ser Asn Pro Pro Val Pro Ile Ser Val Thr
        210                 215                 220

GGC ATT AAG AAT ACG CTG ATT CGT TTA CAA GAG CTT CGT CAA TTG AAC 2472
Gly Ile Lys Asn Thr Leu Ile Arg Leu Gln Glu Leu Arg Gln Leu Asn
    225                 230                 235

ACT GAA AAT TGG GAT ATG TCT AGA ATT CCT TCG AAA AGA TTA CAA CAA 2520
Thr Glu Asn Trp Asp Met Ser Arg Ile Pro Ser Lys Arg Leu Gln Gln
240                 245                 250                 255

TTC GCG CGT CAC ACA GTC GCT GTT AGA TCA CAA GCA ATT GCT AGA ATG 2568
Phe Ala Arg His Thr Val Ala Val Arg Ser Gln Ala Ile Ala Arg Met
                260                     265                 270

CCC GAT CAA CGA CGT ATG GCT ATG TTA GTT GCA TTT GCT AAA ATG TAT 2616
Pro Asp Gln Arg Arg Met Ala Met Leu Val Ala Phe Ala Lys Met Tyr
            275                     280                 285

ACA CAA AGT GCT CAG GAT GAT GTC ATT GAT ATT TTT GAT AGA TAT TTA 2664
Thr Gln Ser Ala Gln Asp Asp Val Ile Asp Ile Phe Asp Arg Tyr Leu
        290                     295                 300

ACA GAT TTA TTT GCT AAG ACA TAT CGA AAG GAA CAA AAA GAA CGT CTT 2712
Thr Asp Leu Phe Ala Lys Thr Tyr Arg Lys Glu Gln Lys Glu Arg Leu
    305                     310                 315

CGT ACA ATT AAG GAT TTA GAT AAG GCA GCG CGC CAA TTA CGG GAA GCT 2760
Arg Thr Ile Lys Asp Leu Asp Lys Ala Ala Arg Gln Leu Arg Glu Ala
320                     325                 330                 335

TGT GTA ATA TTA TTA GAA CAT ACG GAT CCT TCT GTC CAT CCA AAA ACG 2808
Cys Val Ile Leu Leu Glu His Thr Asp Pro Ser Val His Pro Lys Thr
                340                     345                 350

GCA GTG TTT GAA AAA ATT TCA GAA AAG GAT TTA ATA CAA GCT GTC CAA 2856
Ala Val Phe Glu Lys Ile Ser Glu Lys Asp Leu Ile Gln Ala Val Gln
            355                     360                 365

ATT GTT GAT TCA CTC ACC TAT TCA CCA AAT CAA ACA CTA GCC TAT TCA 2904
Ile Val Asp Ser Leu Thr Tyr Ser Pro Asn Gln Thr Leu Ala Tyr Ser
        370                     375                 380

GGA TTG TTA CAA CAT TAT GGC ATA ATC CGA AAA TTT CTT CCT TTA CTC 2952
Gly Leu Leu Gln His Tyr Gly Ile Ile Arg Lys Phe Leu Pro Leu Leu
    385                     390                 395

ATG GAA GAA ATT GAA TTA CAA GCA ACG CCT GCT GGA TTA CCC ATC TTG 3000
Met Glu Glu Ile Glu Leu Gln Ala Thr Pro Ala Gly Leu Pro Ile Leu
400                     405                 410                 415

CAA GCA TGG AAT TTT GTA AAA GAG CAT GGG AAA TCC AAT AAG AAA AGA 3048
Gln Ala Trp Asn Phe Val Lys Glu His Gly Lys Ser Asn Lys Lys Arg
                420                 425                 430

TGG AAA AAT GCT CCT CTT GCC GGT TTG AAT GCA AAT TGG TCT AAG GTT 3096
Trp Lys Asn Ala Pro Leu Ala Gly Leu Asn Ala Asn Trp Ser Lys Val
            435                 440                 445

GTA ATT GAT AAA GAT TCC GGA ACT GTA AAT CAT CGA GCA TAT ACG TTT 3144
Val Ile Asp Lys Asp Ser Gly Thr Val Asn His Arg Ala Tyr Thr Phe
        450                 455                 460

TGG ATG CTC GAA CAA GTA TTA GAA GCT TTG CAC CGA CAT GAT CTA TAT 3192
Trp Met Leu Glu Gln Val Leu Glu Ala Leu His Arg His Asp Leu Tyr
    465                 470                 475

ATA GTA GGA AGT GAA AAA TAT GGG GAC CTT CGC GCA CAA TTA TTA CAA 3240
Ile Val Gly Ser Glu Lys Tyr Gly Asp Leu Arg Ala Gln Leu Leu Gln
480                 485                 490                 495

GAC GAA GAA TGG AAA AGT ATT CGT CCT AGT ATT CTT CGC TCA TTA GAC 3288
Asp Glu Glu Trp Lys Ser Ile Arg Pro Ser Ile Leu Arg Ser Leu Asp
                500                 505                 510

TGG TCA ATA GAT TCT TAT GAA TCA TTG ACA CCG TTA AAA GAA GAG TTA 3336
Trp Ser Ile Asp Ser Tyr Glu Ser Leu Thr Pro Leu Lys Glu Glu Leu
            515                 520                 525

GAC AAA ACT TAT CAT CAA GTC ATT GAG AAT TGG GAG AAT AAT CCT GCG 3384
Asp Lys Thr Tyr His Gln Val Ile Glu Asn Trp Glu Asn Asn Pro Ala
        530                 535                 540

GTG CAA ATA GAC ACA TTT GCA GGT AAA GAG AGA ATT GTT TTG ACA CCT 3432
Val Gln Ile Asp Thr Phe Ala Gly Lys Glu Arg Ile Val Leu Thr Pro
    545                 550                 555

TTA GAC AAA CAA CCA GAA CCT GAA TCA CTA CAA AAA CTA AAA CAA CAA 3480
Leu Asp Lys Gln Pro Glu Pro Glu Ser Leu Gln Lys Leu Lys Gln Gln
560                 565                 570                 575

ATA CAT ACG ATG TTG CCA AAT ATA GAT ATT CCT CAA TTA TTA CTC GAA 3528
Ile His Thr Met Leu Pro Asn Ile Asp Ile Pro Gln Leu Leu Leu Glu
580 585 590

GTA AAT CGT TGG ACG GGA TTT ATG GAT GGT TTT CGA CAT ATT AGT GAG 3576
Val Asn Arg Trp Thr Gly Phe Met Asp Gly Phe Arg His Ile Ser Glu
595 600 605

GCT AAA TCT AGA ATT AAC GAG TTA CCT ATA AGT ATC TGT GCA TTG CTT 3624
Ala Lys Ser Arg Ile Asn Glu Leu Pro Ile Ser Ile Cys Ala Leu Leu
610 615 620

ATA TCT CAA GCA TGC AAT ATT GGG TTA AGA CCT TTA GTT CAA GAT GGG 3672
Ile Ser Gln Ala Cys Asn Ile Gly Leu Arg Pro Leu Val Gln Asp Gly
625 630 635

GTT CCT TCA TTA GAA CGT GAT CGT CTT ACA TGG ATT GAA CAA AAT TAT 3720
Val Pro Ser Leu Glu Arg Asp Arg Leu Thr Trp Ile Glu Gln Asn Tyr
640 645 650 655

TTT CGT GCA GAA ACA CTT TCA GAA TCA AAC GCG AAA CTT GTA GAT TTT 3768
Phe Arg Ala Glu Thr Leu Ser Glu Ser Asn Ala Lys Leu Val Asp Phe
660 665 670

CAT AGC CAA TTA CAG CTG GCT AAA ATG TGG GGT GGT GGA GAA ATT GCT 3816
His Ser Gln Leu Gln Leu Ala Lys Met Trp Gly Gly Gly Glu Ile Ala
675 680 685

TCA GCT GAT GGA TTA CGT TTC ATC ACA CCA GTA AAA TCC GTA CAC ACT 3864
Ser Ala Asp Gly Leu Arg Phe Ile Thr Pro Val Lys Ser Val His Thr
690 695 700

GGT CCA AAT CCT AAA TAT TTC GGT TCT GGT CGT GGT GTT ACG TAT TAC 3912
Gly Pro Asn Pro Lys Tyr Phe Gly Ser Gly Arg Gly Val Thr Tyr Tyr
705 710 715

AAC TAT ACG AGC GAT CAA TTT ACC GGA CTC CAC GGT TTG GTG ATT CCA 3960
Asn Tyr Thr Ser Asp Gln Phe Thr Gly Leu His Gly Leu Val Ile Pro
720 725 730 735

GGC ACA ATT CGT GAT TCA TTA TTA TAC TTA CTT CAA TGT GTG TTA GAA CAA 4008
Gly Thr Ile Arg Asp Ser Leu Leu Tyr Leu Leu Gln Cys Val Leu Glu Gln
                740                     745                     750

AAT ACG AAC TTA CAG CCA AAA GAA ATT ATG ACA GAT ACA GCT GGG TAT 4056
Asn Thr Asn Leu Gln Pro Lys Glu Ile Met Thr Asp Thr Ala Gly Tyr
            755                     760                     765

AGT GAT ATT ATT TTT GGG CTC TTT GGA TTA TTA GGA TAT CAA TTT AGT 4104
Ser Asp Ile Ile Phe Gly Leu Phe Gly Leu Leu Gly Tyr Gln Phe Ser
        770                     775                     780

CCT CGT TTA GCT GAT ATC AGT GAA TCA CGT CTT TGG CGT TTT GAT GCG 4152
Pro Arg Leu Ala Asp Ile Ser Glu Ser Arg Leu Trp Arg Phe Asp Ala
    785                     790                     795

AAC TCA GAT TAT AGC ATG TTA AAT AAT TTG TCT AAA AGT CGC ATT CGT 4200
Asn Ser Asp Tyr Ser Met Leu Asn Asn Leu Ser Lys Ser Arg Ile Arg
800                     805                     810                     815

GAA GAA CTC ATA CAT CGT CAT TGG GAA GAC ATG CTT CGT GTT GCG GGA 4248
Glu Glu Leu Ile His Arg His Trp Glu Asp Met Leu Arg Val Ala Gly
                820                     825                     830

TCT TTG AAA CTA AAT AAA ATA AAT GCA ACA CAT CTT ATC CAA GCA CTT 4296
Ser Leu Lys Leu Asn Lys Ile Asn Ala Thr His Leu Ile Gln Ala Leu
            835                     840                     845

CAG TAT AAT GGG AAA CCA ACT ATG TTA GGG CGA GCA ATT GGA GAA TTG 4344
Gln Tyr Asn Gly Lys Pro Thr Met Leu Gly Arg Ala Ile Gly Glu Leu
        850                     855                     860

GGG AGA CTC TTT AAA ACA CGT TAT TTA CTC TTA TAT TTA CAT GAT GAA 4392
Gly Arg Leu Phe Lys Thr Arg Tyr Leu Leu Leu Tyr Leu His Asp Glu
    865                     870                     875

AAT TAT CGT CGT AAA ATT TTA AAT CAA CTC AAT AGA GGG GAA GCA AGG 4440
Asn Tyr Arg Arg Lys Ile Leu Asn Gln Leu Asn Arg Gly Glu Ala Arg
880                     885                     890                     895

CAT AGT TTA GCG AGG GCT GTA TTT TAC GGC AAA CGT GGA GAA CTT CAT 4488
His Ser Leu Ala Arg Ala Val Phe Tyr Gly Lys Arg Gly Glu Leu His
900 905 910

CAA TCC TAT CGA GAA GGA CAA GAA GAG CAA TTA GGT GCA TTA GGT TTA 4536
Gln Ser Tyr Arg Glu Gly Gln Glu Glu Gln Leu Gly Ala Leu Gly Leu
915 920 925

GTA GTA AAT GCA ATT ATT GTA TGG AAT ACA CGA TAT ATA GAA TCT GCG 4584
Val Val Asn Ala Ile Ile Val Trp Asn Thr Arg Tyr Ile Glu Ser Ala
930 935 940

TTA CAA GTA CTC CGA AAT CGC GGT CAT ACA ATT GAT AAT GAT GAT ATA 4632
Leu Gln Val Leu Arg Asn Arg Gly His Thr Ile Asp Asn Asp Asp Ile
945 950 955

TCT AGA CTT TCA CCA TTA GGC CAT AAA CAC ATT AAC ATA GTA GGT CGG 4680
Ser Arg Leu Ser Pro Leu Gly His Lys His Ile Asn Ile Val Gly Arg
960 965 970 975

TAT TCA TTT GTT CTC CCA GAA GAA GTA AAA GAT GGG CAA TTA CGT ACA 4728
Tyr Ser Phe Val Leu Pro Glu Glu Val Lys Asp Gly Gln Leu Arg Thr
980 985 990

CTA ACA TAT GAA GAA ACA AAC AAA AAG GAA CCT GAT TCT TTA TAAGAATAGG 4780
Leu Thr Tyr Glu Glu Thr Asn Lys Lys Glu Pro Asp Ser Leu
995 1000 1005

TTCCTAATGT CCGCTAATGC TTGTTGCGTG ATTTGTTCC ATTGCTACAC ATACCCC 4837

(2) INFORMATION FOR SEQ ID NO:2:

(i) SEQUENCE CHARACTERISTICS:
(A) LENGTH: 306 amino acids
(B) TYPE: amino acid
(D) TOPOLOGY: linear

(ii) MOLECULE TYPE: protein

(xi) SEQUENCE DESCRIPTION: SEQ ID NO:2:
   Met His Ser Thr Lys Thr Ile Ser Ile Gln Ala Thr Ser Leu Ile Ser
   1               5                   10                  15
   Asp Phe Ile Ser Ser Leu Ser Gln Glu Gly Asp Leu His Thr Lys Thr
               20                  25                  30
   Leu Lys Glu Tyr Thr Ser Asp Leu Lys Asp Phe Val Phe Trp Phe Glu
           35                  40                  45
   Asn Val Trp Gly Lys His Ala Glu Asp Thr Leu Phe His Pro Ile Glu
       50                  55                  60
   Val Thr Ala Arg Thr Ile Ala Arg Tyr Arg Gly His Met Gln Val Thr
   65                  70                  75                  80
   Arg Leu Leu Lys Pro Ser Thr Ile Asn Arg Arg Ile Asn Ser Ile Lys
                   85                  90                  95
   Arg Tyr Phe Asp Trp Ala Lys Gln Lys Gly Leu Val Gln Thr Asn Tyr
               100                 105                 110
   Ser Lys Ser Ile Lys Phe Val Pro Thr Glu Lys Thr Ser Pro Lys Arg
           115                 120                 125
   Met Ser Asp Lys Glu Glu Ala Ala Leu Met His Ala Val Glu Lys Tyr
       130                 135                 140
   Gly Thr Leu Arg Asp Arg Ala Met Ile Ile Phe Met Leu His Thr Gly
   145                 150                 155                 160
   Leu Arg Ser Met Glu Val Cys Asp Val Gln Ile Glu Asp Val Ile Met
                   165                 170                 175
   Arg Lys Arg Gly Gly Tyr Val Val Val Arg Ser Gly Lys Arg Asn Lys
               180                 185                 190
   Gln Arg Glu Val Pro Leu Asn Ser Thr Ala Arg Cys Ala Leu Glu Glu
           195                 200                 205
   His Ile Arg Leu Ser Glu Ile Ser Gln Ser Tyr Leu Phe Pro Ser Ser
       210                 215                 220
   Lys Thr Gly Lys Arg Leu Gln Glu Arg Ala Ile Arg His Ile Leu Gln
   225                 230                 235                 240
   Lys Tyr Ile Arg Leu Ala Lys Leu Glu Gly Phe Ser Ala His Asp Leu
                   245                 250                 255
   Arg His Arg Phe Gly Tyr Val Met Ala Glu Arg Thr Pro Leu His Arg
               260                 265                 270
   Leu Ala Gln Ile Met Gly His Asp Asn Leu Asn Thr Thr Met Ile Tyr
           275                 280                 285
   Val Arg Ala Thr Gln Glu Asp Leu Gln Gly Glu Val Glu Lys Ile Ala
       290                 295                 300
   Trp Asn
   305

(2) INFORMATION FOR SEQ ID NO:3:

    (i) SEQUENCE CHARACTERISTICS:
        (A) LENGTH: 1005 amino acids
        (B) TYPE: amino acid
        (D) TOPOLOGY: linear

    (ii) MOLECULE TYPE: protein

    (xi) SEQUENCE DESCRIPTION: SEQ ID NO:3:

Met Pro Val Asp Phe Leu Thr Pro Glu Gln Glu Glu Lys Tyr Gly Cys
1               5                   10                  15

Phe Cys Asp Thr Pro Thr Ser Glu Gln Leu Ala Lys Tyr Phe Trp Leu
                20                  25                  30
Asp Asp Thr Asp Lys Glu Leu Ile Trp Asn Arg Arg Gly Glu His Asn
            35                  40                  45
Gln Leu Gly Phe Ala Val Gln Leu Gly Thr Val Arg Phe Leu Gly Thr
        50                  55                  60
Phe Leu Ser Asp Pro Thr Asn Val Pro Gln Ser Val Ile Thr Tyr Met
65                  70                  75                  80
Ala Asn Gln Leu His Leu Asp Ala Gln Ser Phe Ser Arg Tyr Arg Asn
                85                  90                  95
Lys Arg Ser Gln Trp Asp Gln Met Gln Glu Ile Arg Ser Val Tyr Gly
            100                 105                 110
Tyr Lys Asn Phe Thr Asp Lys Ser Thr His Trp Arg Phe Ile Arg Trp
        115                 120                 125
Leu Tyr Ala Arg Ala Trp Leu Tyr Asn Glu Arg Pro Ser Val Leu Phe
    130                 135                 140
Asp Leu Ala Thr Ala Arg Cys Ile Glu Gln Lys Ile Leu Leu Pro Gly
145                 150                 155                 160
Val Ser Val Leu Thr Arg Leu Val Ser Thr Val Arg Asp Arg Ser Ala
                165                 170                 175
Glu Asn Ile Trp Lys Lys Leu Ser Ser Leu Pro Asp Asn Val Gln Lys
            180                 185                 190
Lys Gln Leu Glu Asn Leu Leu Gln Ile Asp Gln Lys Thr Lys Thr
        195                 200                 205
Tyr Leu Glu Arg Leu Ser Asn Pro Pro Val Pro Ile Ser Val Thr Gly
    210                 215                 220

Ile Lys Asn Thr Leu Ile Arg Leu Gln Glu Leu Arg Gln Leu Asn Thr
225                 230                 235                 240

Glu Asn Trp Asp Met Ser Arg Ile Pro Ser Lys Arg Leu Gln Gln Phe
                245                 250                 255

Ala Arg His Thr Val Ala Val Arg Ser Gln Ala Ile Ala Arg Met Pro
            260                 265                 270

Asp Gln Arg Arg Met Ala Met Leu Val Ala Phe Ala Lys Met Tyr Thr
        275                 280                 285

Gln Ser Ala Gln Asp Asp Val Ile Asp Ile Phe Asp Arg Tyr Leu Thr
    290                 295                 300

Asp Leu Phe Ala Lys Thr Tyr Arg Lys Glu Gln Lys Glu Arg Leu Arg
305                 310                 315                 320

Thr Ile Lys Asp Leu Asp Lys Ala Ala Arg Gln Leu Arg Glu Ala Cys
                325                 330                 335

Val Ile Leu Leu Glu His Thr Asp Pro Ser Val His Pro Lys Thr Ala
            340                 345                 350

Val Phe Glu Lys Ile Ser Glu Lys Asp Leu Ile Gln Ala Val Gln Ile
        355                 360                 365

Val Asp Ser Leu Thr Tyr Ser Pro Asn Gln Thr Leu Ala Tyr Ser Gly
    370                 375                 380

Leu Leu Gln His Tyr Gly Ile Ile Arg Lys Phe Leu Pro Leu Leu Met
385                 390                 395                 400

Glu Glu Ile Glu Leu Gln Ala Thr Pro Ala Gly Leu Pro Ile Leu Gln
                405                 410                 415

Ala Trp Asn Phe Val Lys Glu His Gly Lys Ser Asn Lys Lys Arg Trp
            420                 425                 430

Lys Asn Ala Pro Leu Ala Gly Leu Asn Ala Asn Trp Ser Lys Val Val
            435                 440                 445

Ile Asp Lys Asp Ser Gly Thr Val Asn His Arg Ala Tyr Thr Phe Trp
    450                 455                 460

Met Leu Glu Gln Val Leu Glu Ala Leu His Arg His Asp Leu Tyr Ile
465                 470                 475                 480

Val Gly Ser Glu Lys Tyr Gly Asp Leu Arg Ala Gln Leu Leu Gln Asp
                485                 490                 495

Glu Glu Trp Lys Ser Ile Arg Pro Ser Ile Leu Arg Ser Leu Asp Trp
            500                 505                 510

Ser Ile Asp Ser Tyr Glu Ser Leu Thr Pro Leu Lys Glu Glu Leu Asp
        515                 520                 525

Lys Thr Tyr His Gln Val Ile Glu Asn Trp Glu Asn Asn Pro Ala Val
    530                 535                 540

Gln Ile Asp Thr Phe Ala Gly Lys Glu Arg Ile Val Leu Thr Pro Leu
545                 550                 555                 560

Asp Lys Gln Pro Glu Pro Glu Ser Leu Gln Lys Leu Lys Gln Gln Ile
                565                 570                 575

His Thr Met Leu Pro Asn Ile Asp Ile Pro Gln Leu Leu Leu Glu Val
            580                 585                 590

Asn Arg Trp Thr Gly Phe Met Asp Gly Phe Arg His Ile Ser Glu Ala
        595                 600                 605

Lys Ser Arg Ile Asn Glu Leu Pro Ile Ser Ile Cys Ala Leu Leu Ile
    610                 615                 620

Ser Gln Ala Cys Asn Ile Gly Leu Arg Pro Leu Val Gln Asp Gly Val
625                 630                 635                 640

Pro Ser Leu Glu Arg Asp Arg Leu Thr Trp Ile Glu Gln Asn Tyr Phe
                645                 650                 655

Arg Ala Glu Thr Leu Ser Ser Glu Ser Asn Ala Lys Leu Val Asp Phe His
            660                 665                 670

Ser Gln Leu Gln Leu Ala Lys Met Trp Gly Gly Gly Glu Ile Ala Ser
        675                 680                 685

Ala Asp Gly Leu Arg Phe Ile Thr Pro Val Lys Ser Val His Thr Gly
    690                 695                 700

Pro Asn Pro Lys Tyr Phe Gly Ser Gly Arg Gly Val Thr Tyr Tyr Asn
705                 710                 715                 720

Tyr Thr Ser Asp Gln Phe Thr Gly Leu His Gly Leu Val Ile Pro Gly
                725                 730                 735

Thr Ile Arg Asp Ser Leu Tyr Leu Leu Gln Cys Val Leu Glu Gln Asn
            740                 745                 750

Thr Asn Leu Gln Pro Lys Glu Ile Met Thr Asp Thr Ala Gly Tyr Ser
        755                 760                 765

Asp Ile Ile Phe Gly Leu Phe Gly Leu Leu Gly Tyr Gln Phe Ser Pro
    770                 775                 780

Arg Leu Ala Asp Ile Ser Glu Ser Arg Leu Trp Arg Phe Asp Ala Asn
785                 790                 795                 800

Ser Asp Tyr Ser Met Leu Asn Asn Leu Ser Lys Ser Arg Ile Arg Glu
                805                 810                 815

Glu Leu Ile His Arg His Trp Glu Asp Met Leu Arg Val Ala Gly Ser
            820                 825                 830

Leu Lys Leu Asn Lys Ile Asn Ala Thr His Leu Ile Gln Ala Leu Gln
        835                 840                 845

Tyr Asn Gly Lys Pro Thr Met Leu Gly Arg Ala Ile Gly Glu Leu Gly
    850                 855                 860

Arg Leu Phe Lys Thr Arg Tyr Leu Leu Leu Tyr Leu His Asp Glu Asn
865                 870                 875                 880

Tyr Arg Arg Lys Ile Leu Asn Gln Leu Asn Arg Gly Glu Ala Arg His
                885                 890                 895

Ser Leu Ala Arg Ala Val Phe Tyr Gly Lys Arg Gly Glu Leu His Gln
            900                 905                 910

Ser Tyr Arg Glu Gly Gln Glu Glu Gln Leu Gly Ala Leu Gly Leu Val
        915                 920                 925

Val Asn Ala Ile Ile Val Trp Asn Thr Arg Tyr Ile Glu Ser Ala Leu
    930                 935                 940

Gln Val Leu Arg Asn Arg Gly His Thr Ile Asp Asn Asp Asp Ile Ser
945                 950                 955                 960

Arg Leu Ser Pro Leu Gly His Lys His Ile Asn Ile Val Gly Arg Tyr
                965                 970                 975

Ser Phe Val Leu Pro Glu Glu Val Lys Asp Gly Gln Leu Arg Thr Leu
            980                 985                 990

Thr Tyr Glu Glu Thr Asn Lys Lys Glu Pro Asp Ser Leu
        995                 1000                1005

(2) INFORMATION FOR SEQ ID NO:4:

     (i) SEQUENCE CHARACTERISTICS:
          (A) LENGTH: 85 amino acids
          (B) TYPE: amino acid
          (D) TOPOLOGY: linear

    (ii) MOLECULE TYPE: protein

(xi) SEQUENCE DESCRIPTION: SEQ ID NO:4:

Met Ala Ile Arg Lys Asp Glu Leu Tyr Arg Leu Ile Asp His Leu Asp
1               5                   10                  15

Gln Gln Asp Glu Lys Ala Ala Phe Asp Phe Leu Glu Phe Leu Val Gln
            20                  25                  30

Arg Ser Arg Arg Lys Pro Lys Glu Trp Glu Lys Ile Asp Met Ala Asp
        35                  40                  45

Pro Asp His Glu Pro Leu Ser Thr Gln Glu Leu Glu Gln Leu Asn Ser
ts  50                  55                  60

Glu Glu Gly Tyr Val Ser Gly Glu Asp Ala Lys Arg Glu Phe Gly Leu
65                  70                  75                  80

Gln Ile Asp Leu Pro
                85